(19) Europäisches Patentamt
European Patent Office
Office européen des brevets

(11) EP 4 025 907 B1

# (12) EUROPEAN PATENT SPECIFICATION

(45) Date of publication and mention of the grant of the patent:
**20.11.2024 Bulletin 2024/47**

(21) Application number: **20767927.5**

(22) Date of filing: **26.08.2020**

(51) International Patent Classification (IPC):
*G01N 33/00* (2006.01) *G01N 33/02* (2006.01)

(52) Cooperative Patent Classification (CPC):
**G01N 33/0047; G01N 33/02**

(86) International application number:
**PCT/US2020/047911**

(87) International publication number:
**WO 2021/045943 (11.03.2021 Gazette 2021/10)**

(54) **DEVICE AND METHOD FOR DETECTING INSECT LARVAE AND ADULT INSECTS IN STORED PRODUCTS BY SENSING THEIR VOLATILE PHEROMONES AND SEMIOCHEMICALS**

VORRICHTUNG UND VERFAHREN ZUR DETEKTION VON INSEKTENLARVEN UND ADULTEN INSEKTEN IN GELAGERTEN PRODUKTEN DURCH MESSUNG IHRER FLÜCHTIGEN PHEROMONE UND SEMIOCHEMIKALIEN

DISPOSITIF ET PROCÉDÉ DE DÉTECTION DE LARVES D'INSECTES ET D'INSECTES ADULTES DANS DES PRODUITS STOCKÉS PAR DÉTECTION DE LEURS PHÉROMONES VOLATILES ET MÉDIATEURS CHIMIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.09.2019 US 201916558490**

(43) Date of publication of application:
**13.07.2022 Bulletin 2022/28**

(73) Proprietor: **Sensor Development Corporation**
**Elyria OH 44035 (US)**

(72) Inventors:
- **SMILANICH, Nicholas Joseph**
  **Rocky River, Ohio 44035 (US)**
- **REICHERT, Samuel Firestone**
  **Brunswick, Ohio 44212 (US)**
- **TUDRON, Frank Bernarn**
  **Mentor, Ohio 44060 (US)**

(74) Representative: **Kramer Barske Schmidtchen Patentanwälte PartG mbB**
**European Patent Attorneys**
**Landsberger Strasse 300**
**80687 München (DE)**

(56) References cited:
**US-A1- 2019 234 895**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 025 907 B1

## Description

## BACKGROUND

The following disclosure relates generally to the insect and insect infestation detection arts, chemical sensing arts, gas detection arts, volatile organic compound analysis arts, gas-sensing microchip arrays, and methods and devices related thereto. It finds particular application in association with arts related to the high sensitivity and selectivity detection of insects in stored food and other products or materials.

Stored product insects ("SPIs") are most often found feeding on finished food products, the ingredients for food or are infesting equipment where food is prepared, processed, packaged or stored. The economic losses from these pests in the processing, transportation, and storage systems can be in the millions of dollars per incident of contamination, product recall, consumer complaint/litigation, and pest control applications (Arthur et. al., 2009). Additionally, certain SPIs have health implications if accidently consumed, causing gastric stress in infants and elderly people (Okamura, 1967).

Current insect detection relies on flashlight inspection and the use of traps with multiple synthetic pheromone lures and traps to capture adult SPI. The pheromones are volatile organic compounds ("VOCs") that are emitted from male and or females of the individual species. Pheromone lures and traps rely on insect activity and this can be significantly affected by temperature. Pheromone volatility, quantity/quality, as well as human activity and insect dynamics interplay with these elements resulting in trap data that is quite variable. Interpretation of trap catch is based on a small sampling of the population (2-10% or less). This makes detection and remediation of pest infestations difficult.

The Indianmeal moth ("IMM") is the most common stored product insect found throughout the U.S. (Mueller, 1998; Resener 1996). It is the one insect found more often than any other on stored food and grain in the U.S. The adult IMM can be found almost anywhere in the temperate regions of the world. Further, in the U.S. and Europe it is the one insect pest that causes the most damage. There are two reasons that this insect has survived so well in our environment: 1) the large number of eggs the female lays in her short lifetime; and 2) its ability to genetically change or adapt to survive pesticides which man uses to protect his food (resistance). The IMM was found to be the most resistant insect known to man. Over a fifty-year period, the genetic makeup of this insect has been changed to resist the commonly used pesticide Malathion. In the 1970's, the IMM started showing signs of resistance to this commonly used insecticide. The IMM developed a 60,000-fold resistance to this pesticide.

The IMM are most often found feeding on finished food products, the ingredients for food such as stored wheat products, milled/processed wheat, and other stored products such as milled cereal products, flour, bran, pasta products, spices, or infesting equipment where food is prepared, processed, packaged or stored. IMM larvae are the destructive life stage of the insect, eating voraciously. The larvae are highly mobile and can continuously seek out new sources of food. The value of the food is damaged by the food they consume, the frass they deposit, and the webbing that the larvae leave behind as they move.

Further, the IMM is often a precursor of other stored product insects. An un-treated IMM infestation can be an indicator of other SPI infestations yet to come (Mueller, 2016). The five most commonly encountered stored product insects (SPI) include the Indianmeal moth (Plodia interpunctella), warehouse beetle (Trogoderma variabile), flour beetles (Tribolium spp.), grain beetles (Oryzaephilus spp.) and the cigarette beetle (Lasioderma serricorne) (Mueller, 1998; Hagstrum and Subramanyam, 2006). The economic losses from these pests in processing, transporting and storing can be in the millions of dollars per incident of contamination, product recall, consumer complaint/litigation, and pest control applications (Arthur, 2009). Yet there is no efficient, low cost method to monitor and detect them.

Several SPI pheromones have been identified but are not commercially available due to short shelf life and cost of production (Phillips et. al., 2000). The compounds are unique but can attract interspecies competitors such as in the stored food moth group and the Trogoderma complex. The single pheromone (Z,E)-9,12-Tetradecadienyl acetate is the predominant pheromone for Plodia, but will attract three other food moths of the Ephestia species. The pheromone compound R,Z 14-Methyl -8-Hexadecenal is the main component for attracting the warehouse beetle (Trogoderma variabile), but will also attract three other common Trogoderma species including a quarantine pest (Khapra beetle, Trogoderma granarium). Several species of flour beetles (Tribolium species) are attracted to a single compound 4,8-Dimethyldecanal, two species of grain beetles (Oryzaephilus species) are attracted to (Z,Z)-3,6-Dodecadien-11-olide, but (4S,6S,7S)-4,6-Dimethyl-7-hydroxynona-3-one, the pheromone for cigarette beetles (Lasioderma serricorne) is unique to the species.

Furthermore, with respect to possible target semiochemicals and/or kairomones, these semiochemicals and kairomones are high molecular weight VOCs. Thus, their vapor pressures and concentrations in the headspace over infested stored products will be low, and thus are much more difficult to detect.

Thus, it would be desirable to eliminate the variability and uncertainty of detecting pest presence/absence, abundance, and location by using methods, devices, and systems that can detect and measure multiple pheromone concentrations. Additionally, it would be desirable provide such methods, devices, and systems that can detect not only

insect larvae but also insect eggs by sensing their semiochemicals/kairomones in an analogous fashion. By allowing the detection of earlier life stages (e.g. eggs), the amount of loss to the stored products can be limited because most of the damage is done by the insects during the larval stage, not during adulthood. Threshold concentrations can be established to determine immediate absence or presence of the most common SPI within a trailer, land/ sea container, bulk tote, pallet of bagged ingredients or a storage room. It would also be desirable to provide the ability to detect a gradient of VOC concentrations, which could assist in locating and pinpointing SPI infestations within structures, wall voids, crack and crevices or equipment. Further, it is desirable to provide a handheld device, which would remove much of the dependency of insect mobility and environmental as factors that affect activity from the SPI monitoring model.

## INCORPORATION BY REFERENCE

The following references, are mentioned:

Arthur F. H. Johnson J. A. Neven L. G. Hallman G. J. Follett P. A. (2009). Insect Pest Management in Postharvest Ecosystems in the United States of America. Outlooks on Pest Management, 20: 279-284.

Hagstrum D.W. and Subramanyam B. (2006). Fundamentals of Stored-Product Entomology. St. Paul: AACC Int.

Mueller, David K (1998). Stored Product Protection: A Period of Transition. Insects Limited, Indianapolis, Ind.

Okumura, G.T. (1967). A Report of Canthariasis and Allergy Caused by Trogoderma (Coleoptera: Dermestidae). California Vector Views, Vol. 14 No. 3, pp. 19-22.

Phillips, T.W., Cogan, P.M. and Fadamiro, H.Y. (2000). Pheromones in B. Subramanyam and D. W. Hagstrum (Eds.). Alternatives to Pesticides in Stored-Product IPM, pp. 273-302 Kluwer Academic Publishers, Boston, MA.

Resener, A.M. (1997). National Survey of Stored Product Insects. Fumigants and Pheromones, Issue 46, pp3-4.

US2019/234895 discloses a known system to detect the presence of insect larvae and adult insects in stored products by sensing gas phase markers such as volatile pheromones, semiochemicals, and kairomones.

## BRIEF DESCRIPTION

Disclosed in accordance with the independent claims are low-cost and high-accuracy method, device and system for identifying insect infestations of a stored product (e.g. food) based on the detection of one or more target volatile organic compounds ("VOCs") within a target fluid flow (e.g. air sample) sampled from a region proximate to the stored product. The disclosed method, system and device are designed to provide early detection capability, which enables quick response to the threat of infestations (e.g. sanitation, freezing, fumigation, etc.). Further, they have minimal cost and high accuracy, which enables widespread application of real-time, non-invasive, detection of insect eggs, insect larva, and/or adult insects in settings where products are stored.

In accordance with an embodiment of the present disclosure, there is provided a method according to independent claim 10 wherein at least one of a plurality of VOC sensors is configured to detect the presence of 11,13-hexadecadienal as an egg-specific VOC for identifying an insect infestation of a stored product by detecting one or more target VOCs within a target fluid flow, the method comprising the steps of: heating, via a device comprising a plurality of VOC sensors, at least one of the plurality of VOC sensors to at least a first operating temperature; contacting the one or more VOC sensors with the target fluid flow; determining a set of conductance change values corresponding to each of the one or more VOC sensors contacted with the target fluid flow; and determining a gas component concentration for one or more of the target VOCs within the target fluid flow based on the set of conductance change values. Further, each of the VOC sensors include: a substrate having a first and second side; a resistive heater circuit formed on the first side of the substrate; a sensing circuit formed on the second side of the substrate; and a chemically sensitive film formed over the sensing circuit on the second side of the substrate. In particular embodiments, the method can include correcting the baseline resistance of the VOC sensors to an earlier baseline value after sampling VOC markers in a fluid flow, which may be accomplished by adjusting the operating temperature of one or more VOC sensors after each sampling of the target VOCs.

In accordance with another embodiment of the present disclosure, there is provided a device according to independent claim 1 for detecting one or more target VOCs within a target fluid flow the device comprising a sensor array having a plurality of VOC sensors, wherein each VOC sensor includes: a substrate; a resistive heater circuit formed on a first side of the substrate; a sensing circuit formed on a second side of the substrate; and a chemically sensitive film formed over the sensing circuit on the second side of the substrate, wherein at least one of the plurality of VOC sensors is configured to detect the presence of 11,13-hexadecadienal as an egg-specific VOC.

In accordance with still another embodiment of the present disclosure, there is provided a system according to independent claim 9 for identifying an insect infestation of a stored product the system comprising: a testing chamber enclosing a sensor array; an air transfer unit configured to retrieve a fluid flow and deliver the fluid flow to the testing chamber; and a controller operatively connected to the air transfer unit and the sensor array. The sensor array includes a plurality of VOC sensors, and the controller is configured to: operate the air transfer unit to retrieve the fluid flow from

a target area and deliver the fluid flow to the testing chamber; operate the sensor array to measure a conductance for one or more of the plurality of VOC sensors; determine a set of conductance change values corresponding to each of the one or more VOC sensors; and determine a gas component concentration for one or more target VOCs within the fluid flow based on the set of conductance change values. Further, at least one of the VOC sensors may be configured to detect the presence of 11,13-hexadecadienal as an egg-specific VOC.

## BRIEF DESCRIPTION OF THE DRAWINGS

The subject disclosure may take form in various components and arrangements of components, and in various steps and arrangement of steps. The drawings are only for purposes of illustrating the preferred embodiments and are not to be construed as limiting the subject disclosure.

**FIG. 1** is a flow chart illustrating a method of identifying an insect infestation in accordance with one embodiment of the subject application.
**FIGS. 2A-2B** are flow charts illustrating a further method of identifying an insect infestation in accordance with a further embodiment of the subject application.
**FIG.** 3 is a block diagram illustrating a system configured to perform the methods disclosed herein in accordance with one embodiment of the subject application.
**FIGS. 4A-4B** are an illustration of a first side (**FIG**. 4A) and a second side (**FIG**. 4B) of an individual VOC sensor in accordance with certain embodiments of the subject application.
**FIG. 5** is an illustration of an individual VOC sensor suspended in a holder in accordance with one embodiment of the subject application.
**FIG. 6** is a representative side-view cross-section of a sensor array comprising a plurality of VOC sensors in accordance with one embodiment of the subject application.
**FIG. 7** is a perspective view of a device is shown in accordance with certain aspects of this disclosure.
**FIG. 8** is a block diagram of an infestation detection system in accordance with one embodiment of the subject application.
**FIGS. 9A-9D** are graphs illustrating the sensitivity of a VOC sensor array to various target volatile organic compounds in accordance with one embodiment of the subject application.
**FIGS. 10A-10C** are graphs illustrating the response of a first VOC sensor to the presence of three target stored product insects ("SPIs") in accordance with one embodiment of the subject application.
**FIGS. 11A-11C** are graphs illustrating the response of a second VOC sensor to the presence of three target stored product insects ("SPIs") in accordance with another embodiment of the subject application.
**FIGS. 12A-12C** are graphs illustrating the response of a third VOC sensor to the presence of three target stored product insects ("SPIs") in accordance with one embodiment of the subject application.
**FIGS. 13A-13C** are graphs illustrating the response of a fourth VOC sensor to the presence of three target stored product insects ("SPIs") in accordance with one embodiment of the subject application.
**FIGS. 14A-14D** are graphs illustrating the response of four VOC sensors to the presence varying quantities of three target stored product insects ("SPIs") in accordance with one embodiment of the subject application.
**FIG. 15** is a graph illustrating a sensor response to the number of cocoons in a stored food product test example.
**FIGS. 16A-16C** are graphs illustrating baseline resistance curves over time for a particular sensor chip.
**FIGS. 17A-17C** are graphs illustrating a sensor chip's net resistance versus the count of insects, larvae, and larvae in cocoons.
**FIG. 18** is a graph illustrating a Pd-catalyzed sensor chip's responses to NOW insects at various life stages.
**FIGS. 19A-19G** are graphs illustrating the response of a VOC sensor to the presence of certain insects at various life stages.

## DETAILED DESCRIPTION

In the following specification and the claims which follow, reference will be made to a number of terms which shall be defined to have the following meanings.

### Definitions

In the following specification and the claims that follow, reference will be made to a number of terms which shall be defined to have the following meanings. Although specific terms are used in the following description for the sake of clarity, these terms are intended to refer only to the particular structure of the embodiments selected for illustration in the drawings, and are not intended to define or limit the scope of the disclosure. In the drawings and the following

description below, it is to be understood that like numeric designations refer to components of like function. Furthermore, it should be understood that the drawings are not to scale.

The singular forms "a," "an," and "the" include plural referents unless the context clearly dictates otherwise.

The term "comprising" is used herein as requiring the presence of the named components/steps and allowing the presence of other components/steps. The term "comprising" should be construed to include the term "consisting of", which allows the presence of only the named components/steps.

Numerical values should be understood to include numerical values which are the same when reduced to the same number of significant figures and numerical values which differ from the stated value by less than the experimental error of conventional measurement technique of the type described in the present application to determine the value.

All ranges disclosed herein are inclusive of the recited endpoint and independently combinable (for example, the range of "from 2 mm to 10 mm" is inclusive of the endpoints, 2 mm and 10 mm, and all the intermediate values).

The term "about" can be used to include any numerical value that can vary without changing the basic function of that value. When used with a range, "about" also discloses the range defined by the absolute values of the two endpoints, e.g. "about 2 to about 4" also discloses the range "from 2 to 4." More specifically, the term "about" may refer to plus or minus 10% of the indicated number.

The terms "ppm" and "ppb" should be understood to refer to "parts per million" and "parts per billion" respectively. As used herein, "ppm", "ppb", and the like refer to a volume fraction, rather than a mass fraction or mole fraction. For example, the value 1 ppm may be expressed as 1 μV/V, and the value 1 ppb may be expressed as 1 nV/V.

As used herein, the term "stored food product" should be understood to mean food products stored in some sort of container (e.g. made from paper, cardboard, plastic, foil, cellophane, etc.), and should be understood to include, for example and without limitation, flour, cereal, cake mix, cornmeal, rice, spaghetti, crackers, cookies, seeds, dried beans, popcorn, nuts, chocolate, raisins and other dried fruits, spices, powdered milk, tea, cured meats, birdseed, dry pet food, and almonds (e.g. shelled almonds).

The present disclosure may be understood more readily by reference to the following detailed description and the various drawings discussed therein.

## Methods

Disclosed herein are methods of determining whether an insect infestation is present in a stored product by detecting the presence of one or more target volatile organic compounds ("VOCs"), such as certain semiochemicals, kairomones, and/or pheromones of various stored product insects ("SPIs"), with high sensitivity and high selectively.

With reference to **FIG. 1,** a method **100** of identifying an insect infestation of a stored product by detecting one or more target volatile organic compounds within a target fluid flow is provided. The method includes: providing a device comprising a sensor array having a plurality of VOC sensors **(S110);** heating one or more of the plurality of VOC sensors to at least a first operating temperature **(S115);** contacting the one or more VOC sensors with the target fluid flow **(S120);** determining a set of conductance change values corresponding to each of the one or more VOC sensors contacted with the target fluid flow **(S125);** and determining a gas component concentration for one or more of the target VOCs within the target fluid flow based on the set of conductance change values **(S130).** In accordance with a first embodiment of the method **100,** each of the VOC sensors of the sensor array comprises: a substrate; a resistive heater circuit; a sensing circuit; and a chemically sensitive film formed over the sensing circuit. In some embodiments, the resistive heater circuit may be formed on a first side of the substrate, the sensing circuit may be formed on a second side of the substrate, and the chemically sensitive film may be formed over the sensing circuit on the second side of the substrate.

In particular embodiments, the method **100** includes measuring a signal conductance for the one or more VOC sensors after contacting the one or more VOC sensors with the target fluid flow. That is, the set of conductance change values may be determined based on the difference between the signal conductance for each of the one or more VOC sensors contacted with the target fluid flow and a baseline conductance of each of the corresponding VOC sensors. In some embodiments, the baseline conductance for one or more VOC sensors is measured while the one or more VOC sensors are in an atmosphere absent of any target VOCs.

In preferred embodiments, the target fluid flow is an air sample taken from within a proximity to the stored product being evaluated for possible insect infestation. That is, the target fluid flow may be a gas sample from the headspace over the stored product of interest.

The method **100** begins at **S105** and ends at **S135,** however, in particular embodiments, the method **100** may be repeated (e.g. repeating steps **S110** to **S130)** by sampling a plurality of target fluid flows (e.g. air samples) from within a plurality of proximities to the stored product(s) being evaluated. That is, the method **100** may identify a gradient of potential insect infestation by sampling one or more target fluid flows at a plurality of distances from the stored product(s) (e.g. at a distance less than about 30.48 cm (1 foot) from the stored product; at a distance between about 30.48 cm and 60.96 cm (1 foot and 2 feet) from the stored product; at distance between about 60.96 cm and 91.44 cm (2 feet and 3 feet) from the stored product; etc.).

In further embodiments, the one or more target VOCs are a semiochemical, a kairomone, and/or a pheromone associated with one or more insects such as SPIs. In particular, the one or more target VOCs may be a semiochemical, a kairomone, and/or a pheromone associated with the red flour beetle, sawtoothed grain beetle, warehouse beetle, Indianmeal moth, navel orangeworm, Mediterranean flour moth, almond moth (as known as tropical warehouse moth), Angoumois grain moth, and/or cigarette beetle, for example. In specific embodiments, the at least one of the one or more target VOCs within a fluid flow may be selected from a group consisting of: 4,8-dimethyldecanal; (Z,Z)-3,6-(11R)-Dodecadien-11-olide; (Z,Z)-3,6-Dodecadien-olide; (Z,Z)-5,8-(11R)-Tetradecadien-13-olide; (Z)-5-Tetradecen-13-olide; (R)-(Z)-14-Methyl-8-hexadecenal; (R)-(E)-14-Methyl-8-hexadecen-al; γ-ethyl-γ-butyrol-actone; (Z,E)-9,12-Tetradecadienyl acetate; (Z,E)-9,12-Tetra-decadien-1-ol; (Z,E)-9,12-Tetradecadienal; (Z)-9-Tetradecenyl acetate; (Z)-11-Hexa-decenyl acetate; (2S,3R,1'S)-2,3-Dihydro-3,5-dimethyl-2-ethyl-6(1-methyl-2-oxobutyl)-4H-pyran-4-one; (2S,3R,1'R)-2,3-Dihydro-3,5-dimethyl-2-ethyl-6(1-methyl-2-oxobutyl)-4H-pyran -4-one; (4S,6S,7S)-7-Hydroxy-4,6-dimethylnonan-3-one; (2S,3S)-2,6-Diethyl-3,5-dimethyl-3,4-dihydro-2H-pyran; 2-Palmitoyl-cyclohexane-1,3-dione; and 2-Oleoyl-cyclohexane-1,3-dione.

With reference to **FIGS. 2A** and **2B,** a method **200** of identifying an insect infestation of a stored product by detecting one or more target volatile organic compounds within a target fluid flow is provided in accordance with a further embodiment of the present disclosure. The method **200** begins at **S202.**

In a step **S204,** a device comprising a sensor array having a plurality of VOC sensors is provided. Each of the VOC sensors of the sensor array comprises: a substrate; a resistive heater circuit; a sensing circuit; and a chemically sensitive film formed over the sensing circuit. In some embodiments, the resistive heater circuit may be formed on a first side of the substrate, the sensing circuit may be formed on a second side of the substrate, and the chemically sensitive film may be formed over the sensing circuit on the second side of the substrate.

In particular embodiments, the sensor array includes a plurality of differentiated VOC sensors. That is, the surface composition for one or more of the plurality of VOC sensors may be varied through the inclusion of catalytic materials in the chemically sensitive film (i.e. active layer). In other words, chemically sensitive film of one or more VOC sensors can comprise a doping agent. In some embodiments, the doping agent may be, for example, a transition metal. For example, the doping agent may be selected from a group consisting of: platinum; palladium; molybdenum; tungsten; nickel; ruthenium; and osmium.

In a step **S206,** one or more of the plurality of VOC sensors are heated to at least a first operating temperature. In particular embodiments, the operating temperature is between about 180°C and about 400°C. In further embodiments, the operating temperature of the one or more VOC sensors is maintained during subsequent steps of the method. In particular, the heating circuit of each VOC sensor may be utilized to measure and control the temperature of the VOC sensor throughout its operation.

In particular embodiments of the method **200,** the method may include one or more calibration steps **208,** comprising: contacting one or more of the plurality of VOC sensors with a sample fluid flow, the sample fluid flow being absent of any target VOCs **(S210);** measuring a baseline conductance for one or more VOC sensors **(S212);** optionally removing the fluid flow from contact with the one or more VOC sensors **(S216);** contacting the one or more VOC sensors with a control fluid flow having a known concentration of the target VOC **(S218);** measuring a control conductance for each of the one or more VOC sensors **(S220);** calculating a specific net conductance value based on the measured control conductance of the VOC sensor and the known concentration of the target VOC within the control fluid flow **(S222);** and repeats at least steps **S218** to **S222** for a plurality of control fluid flows **(S226).** The calibration steps **208** may further comprise: removing the fluid flow from contact with the one or more VOC sensors **(S228);** and adjusting the baseline conductance of one or more VOC sensors **(S230)** after contact with at least one target VOC.

In a step **S210,** one or more of the plurality of VOC sensors are contacted with a sample fluid flow. In preferred embodiments, the sample fluid flow is a volume of air without any target VOCs for which the method **200** may be testing.

In a step **S212,** a baseline conductance for the one or more VOC sensors contacted with the sample fluid flow is measured using the sensing circuits of the VOC sensors. Because the film formed over the sensing circuit of the VOC sensors is chemically sensitive (e.g. semiconductive), the current flowing in the material is due to electrons in the film's conduction band, which may be affected by the presence of undesirable and/or targeted compounds (e.g. target VOCs). Thus, after attaining operating temperature in a step **S206,** and in contact with a gas sample (i.e. sample fluid flow) that does not contain the marker gas (i.e. fluid flows having at least one target VOC), the VOC sensor's resistance is measured and recorded as a baseline resistance or a baseline conductance. In some embodiments, a set of baseline conductances ($\{K_i^0\}$) **214** is determined and includes a baseline conductance (e.g. $K_1^0$, $K_2^0$, ... $K_n^0$) for each of the plurality of VOC sensors.

In a step **S216,** the sample fluid flow is removed from contact with the VOC sensors of the sensor array. In particular embodiments, this may include purging a chamber or reactor housing the sensor array and/or one or more of the VOC sensors.

In a step **S218,** one or more VOC sensors are contacted with a control fluid flow (e.g. marker gas) having a known concentration of at least one target VOC.

In a step **S220,** a control conductance for each of the one or more VOC sensors contacted with the control fluid flow is measured. Because contact with the control fluid flow may make greater or fewer electrons available to the conduction based of the chemically sensitive film, the VOC sensor's resistance / conductance changes.

Then, in a step **S222,** a specific net conductance value for each of the one or more VOC sensors is determined based on the measured test conductance of the VOC sensor and the known concentration of the target VOC within the control fluid flow. As investigated and disclosed herein, the amount of the conductance change may be proportional to the concentration of the gas, with the specific net conductance ("SNC") as used herein refers to the proportionality coefficient. In particular embodiments, the control fluid flow has a first target VOC concentration of about 10 ppb to about 400 ppb. In preferred embodiments, the control fluid flow has a target VOC concentration of about 200 ppb.

The resulting change between the baseline conductance and the control conductance measured for one or more of the plurality of VOC sensors is determined and divided by the specified (i.e. known) concentration to give a SNC value (i.e. a measure of sensitivity of that chip for that gas) with units generally expressed as "nano-mhos/part per billion" or "nmho/ppb". Each chip will have a different SNC for each of the target gases of interest in the application.

For further calibration, in a step **S226,** at least steps **S218** to **S222** may be repeated for additional control fluid flows to obtain a plurality of specific net conductance ("SNC") values for one or more of the VOC sensors, wherein each of the specific net conductance values for each of the VOC sensors corresponds to a different target VOC. In some embodiments, the plurality of SNC values is a set of SNC values ($\{SNC_{i,X}\}$) **224** and includes SNC values corresponding to one or more target VOCs for each of the plurality of VOC sensors (e.g. for a first VOC sensor, $SNC_{1,X_1}$, $SNC_{1,X_2}$, ... $SNC_{1,X_n}$; for a second VOC sensor, $SNC_{2,X_1}$, $SNC_{2,X_2}$, ... $SNC_{2,X_n}$; etc.), wherein $X_n$ represents a particular target VOC.

The method **200** may also include a step that comprises adjusting the baseline conductance / resistance of one or more of the VOC sensors **(S230/S232).** For example, after being contacted with a target VOC(s), a VOC sensor may have a subsequent (i.e. post-contact) baseline conductance different from its baseline conductance prior to contact with the target VOC(s). In particular embodiments, such baseline conductance variations may be accounted for by adjusting the baseline conductance after contact with the target VOC(s) in a step **S230/S232.** During calibration **208,** the control fluid flow may be removed **S228** (e.g. from the sensor array chamber), and the conductance of the VOC sensors may be adjusted in a step **S230** by measuring the conductance of each of the VOC sensors to determine a post-contact conductance for the VOC sensors, comparing the post-contact conductances with the baseline conductances **214,** and heating one or more of the VOC sensors to at least a second operating temperature such that the conductance of each of the VOC sensors at a second operating temperature matches the corresponding baseline conductance **214** prior to contact. The second operating temperature for each of the VOC sensors may be higher or lower than the first operating temperature of the corresponding VOC sensor, based on the measured post-contact conductance of that VOC sensor.

Turning to **FIG. 2B,** after calibration steps **208** the baseline conductance of the VOC sensors may be adjusted in a step **S232** by clearing the sensor array chamber of target VOCs, measuring the conductance of one or more VOC sensors, comparing the measured conductances with the corresponding baseline conductances, and heating one or more of the VOC sensors to at least a second operating temperature such that the conductance of each of the VOC sensors at the second operating temperature matches the corresponding baseline conductance 214.

Following the adjustment step **S232** or the heating step **S206,** one or more VOC sensors are contacted with a target fluid flow at a step **S234.** In particular embodiments, the target fluid flow is an air sample taken from within a proximity to the stored product being evaluated for possible insect infestation. As such, the target fluid flow may contain one or more target VOCs, such as a semiochemical, a kairomone, and/or a pheromone associated with one or more insects (e.g. SPIs). For example, several pheromones and semiochemicals are listed below in **Table 1** and **Table 2** for certain SPIs:

**TABLE 1. SPIs and their Pheromones**

| PEST | PHEROMONE | CHEMICAL NAME |
|---|---|---|
| **Red flour beetle** *Triboleum castaneum* | tribolure | 4,8-Dimethyldecanal |
| **Sawtoothed grain beetle** *Oryzaephilus surinamensis* | cucujolide IV<br>cucujolide IX<br>cucujolide V | (Z,Z)-3,6-(11R)-Dodecadien-11-olide<br>(Z,Z)-3,6-Dodecadienolide<br>(Z,Z)-5,8-(11R)-Tetradecadien-13-olide |

(continued)

| PEST | PHEROMONE | CHEMICAL NAME |
|---|---|---|
| | cucujolide III | (Z)-5-Tetradecen-13-olide |
| **Warehouse beetle** *Trogoderma variabile Ballion* | R,Z-trogodermal<br>R,E-trogodermal<br>γ-caprolactone | (R)-(Z)-14-Methyl-8-hexadecenal<br>(R)-(E)-14-Methyl-8-hexadecenal<br>γ-ethyl-γ-butyrolactone |
| **Indian meal moth** *Plodia interpunctella* | Z9E 12-14Ac<br>Z9E12-14OH<br>Z9E12-14Ald<br>Z9-14Ac<br>Z11-16Ac | (Z,E)-9,12-Tetradecadienyl acetate<br>(Z,E)-9,12-Tetradecadien-1-ol<br>(Z, E)-9,12-Tetradecadienal<br>(Z)-9-Tetradecenyl acetate<br>(Z)-11-Hexadecenyl acetate |
| **Cigarette beetle** *Lasioderma serricorne (F.)* | α-serricorone<br>β-serricorone<br>4S6S7S-serricornin<br>anhydroserricornin<br>2S3R-serricorone | (2S,3R,1'S)-2,3-Dihydro-3,5-dimethyl-2-ethyl-6(1-methyl-2-oxobutyl)-4H-pyran-4-one<br>(2S,3R,1'R)-2,3-Dihydro-3,5-dimethyl-2-ethyl-6(1-methyl-2-oxobutyl)-4H-pyran-4-one<br>(4S,6S,7S)-7-Hydroxy-4,6-dimethylnonan-3-one<br>(2S,3S)-2,6-Diethyl-3,5-dimethyl-3,4-dihydro-2H-pyran<br>(2S,3R)-2,3-Dihydro-3,5-dimethyl-2-ethyl-6-(1-methyl-2-oxobutyl)-4H-pyran-4-one |
| **Navel Orangeworm** *Amyelois transitella* | | 11,13-hexadecadienal |

TABLE 2. IMM Pheromone and Semiochemical Components

| | **Adult Indian meal moth** *Plodia interpunctella* | **Indian meal moth larvae** *Plodia interpunctella* |
|---|---|---|
| **PHEROMONE COMPONENT** | 9,12-Tetradecadienyl acetate<br>9,12-Tetradecadien-1-ol<br>9,12-Tetradecadienal<br>(Z)-9-Tetradecenyl acetate<br>(Z)-11-Hexadecenyl acetate | |
| **SEMIOCHEMICAL COMPONENT** | | 2-Palmitoyl-cyclohexane-1,3-dione<br>2-Oleoyl-cyclohexane-1,3-dione |

At a step **S236,** a signal conductance is measured for the one or more VOC sensors after contacting the one or more VOC sensors with the target fluid flow.

Then, at a step **S238,** a set of conductance change values ({$\Delta K_i$}) is determined for one or more of the VOC sensors of the sensor array. In particular embodiments, for each of the VOC sensors, the conductance change value may be determined as shown below:

$$\Delta K_i = K_i - K_i^0$$

wherein i is an integer, $\Delta K_i$ is the conductance change value for VOC sensor i, $K_i$ is the signal conductance of the VOC sensor i measured in the present of the target fluid flow, and $K_i^0$ is the baseline conductance for the VOC sensor i.

In a step **S240,** a gas component concentration ($[X]_n$) for one or more of the target VOCs within the target fluid flow is determined based on the set of conductance change values. In particular embodiments, more than one target VOC may be present in the target fluid flow, in additional to other background and/or interferent gases, making analysis difficult. In particular embodiments, the gas component concentrations ($[X]_n$) for the one or more target VOCs within the target fluid flow is determined based on the set of conductance change values and the one or more SNCs for each of the VOC sensors. In further embodiments, the gas component concentrations ($[X]_n$) for the one or more target VOCs within the target fluid flow is determined by solving a system of equations, for example, as illustrated below:

$$\Delta K_1 = SNC_{1A}[A] + SNC_{1B}[B] + SNC_{1C}[C] + SNC_{1D}[D] + SNC_{1E}[E] + SNC_{1F}[F]$$

$$\Delta K_2 = SNC_{2A}[A] + SNC_{2B}[B] + SNC_{2C}[C] + SNC_{2D}[D] + SNC_{2E}[E] + SNC_{2F}[F]$$

$$\Delta K_3 = SNC_{3A}[A] + SNC_{3B}[B] + SNC_{3C}[C] + SNC_{3D}[D] + SNC_{3E}[E] + SNC_{3F}[F]$$

$$\Delta K_4 = SNC_{4A}[A] + SNC_{4B}[B] + SNC_{4C}[C] + SNC_{4D}[D] + SNC_{4E}[E] + SNC_{4F}[F]$$

$$\Delta K_5 = SNC_{5A}[A] + SNC_{5B}[B] + SNC_{5C}[C] + SNC_{5D}[D] + SNC_{5E}[E] + SNC_{5F}[F]$$

$$\Delta K_6 = SNC_{6A}[A] + SNC_{6B}[B] + SNC_{6C}[C] + SNC_{6D}[D] + SNC_{6E}[E] + SNC_{6F}[F]$$

wherein $\Delta K_i$ is the measured conductance change for sensor "i", "i" ranging from 1 to 6, $SNC_{ij}$ is the "Specific Net Conductance" of sensor "i" when contacted by gas (e.g. target VOC) "j", "j" being gas or gas category A, B, C or D, E, F, and [X] is the concentration of gas A, B, C, or D expressed in gas volume-to-volume terms, that is, liters of gas per liter of total atmosphere.

Although six target VOCs (i.e. A, B, C, D, E, and F) and six sensors (i.e. 1, 2, 3, 4, 5, and 6) are illustrated above, the number of target VOCs and the number of VOC sensors present in the analysis may vary from application to application, or from use to use, and is not only limited to six. As a result, the problem of determining concentrations for several target VOCs and/or background and interferent gases present within a certain fluid flow becomes possible.

In some embodiments, the method **200** may further comprise operating a user interface to communicate the results of the analysis **(S242).** That is, the device provided in step **S204** may further comprise a user interface configured to display the results of the analysis of the target fluid flow to an associated user. For example, the user interface may be configured to display or otherwise indicate the presence of an insect infestation, including the presence of one or more insects (e.g. SPIs). The presence of an infestation by be indicated based on a pre-determined threshold concentrations, which may be associated with the type of storage facility (e.g. within a trailer, land/ sea container, bulk tote, pallet of bagged ingredients or a storage room) or the type of stored product being tested. The user interface may further be configured to display or otherwise indicate the level of the presence of insects based on the detected target VOCs (e.g. the degree of infestation).

In particular embodiments, the user interface may be a dedicated screen, such as a TFT LCD screen, an IPS LCD screen, a capacitive touchscreen LCD, an LED screen, an OLED screen, an AMOLED screen, or the like. In further embodiments, the user interface may comprise a wired or wireless communications protocol, such as Bluetooth, BLE, Wi-Fi, 3G, 4G, 5G, LTE, or the like, and the user interface may be configured to communicate the results of the analysis to a secondary device (e.g. a mobile phone, tablet, computer, etc.) of the associated user via the communication protocol.

In preferred embodiments, the target fluid flow is an air sample (or volume) taken from within a proximity to the stored product being evaluated for possible insect infestation. In a step **S244,** the steps **S232** to **S242** may be repeated by sampling a plurality of target fluid flows (e.g. air samples) from within a plurality of proximities to the stored product(s) being evaluated. That is, the method **200** may also include identify a source of insect infestation, for example, by detecting a gradient of target VOCs over two or more target fluid flows (e.g. a first target fluid flow, a second target fluid flow, a third target fluid flow, etc.) at varying distances from the stored product(s).

In further embodiments of the method **200,** the device provided in step **S204** may also comprise a controller operatively connected to the sensor array and the user interface, wherein the controller includes a processor that is configured to perform one or more steps of the method **200** described above, and a memory configured to store one or more of the data types discussed above. Furthermore, the memory may be configured to store instructions for performing

one or more of the steps of the method **200.**

At a step **S250,** the method **200** may end.

These and other aspects of devices used to implement the methods 100, 200 described herein may be understood more readily by reference to discussion below and the various drawings discussed therein.

## Devices and Systems

Disclosed herein are devices and systems performing the methods **100,** 200 described above. In particular, discussed herein are highly sensitive and highly selective devices for detecting one or more target volatile organic compounds ("VOCs"), such as certain semiochemicals, kairomones, and/or pheromones of various stored product insects ("SPIs"), within a target fluid flow. Further, the devices and systems may be compact and light enough to be easily portable and handheld.

With reference to **FIG. 3,** a block diagram illustrating a device **300** and a system **302** configured to perform the methods disclosed herein in accordance with one embodiment of the subject application. In particular, the device **300** comprises a sensor array **304** having a plurality of VOC sensors **306.** The plurality of VOC sensors **306** of the sensor array **304** may comprise from about two to about ten VOC sensors, including three, four, five, and six VOC sensors. In particular embodiments, the sensor array **304** may be enclosed in a chamber (or reactor) **308,** wherein the sensors **306** are exposed to (i.e. come into contact with) a desired atmosphere within the chamber **308.** The chamber may have an inlet **310** configured to receive a fluid flow **314** from outside the chamber, and an outlet **312** configured to relieve the chamber **308** of a fluid flow **316.**

As shown in **FIG. 4A** and **FIG. 4B,** which illustrates a first side (**FIG. 4A**) and a second side (**FIG. 4B**) of an individual VOC sensor **306** of a sensor array **304,** the VOC sensor **306** can comprise a substrate **318** having a first side **320** and a second side **322.** The substrate **318** can be, for example, a ceramic material, or may be an alumina ($Al_2O_3$) wafer or a silicon wafer. In particular embodiments, the substrate **318** may have an overall width of about 5 mm to about 20 mm, an overall height of about 4.3 mm to about 20 mm, and an overall thickness of about 0.32 mm to about 0.65 mm. The VOC sensor **306** may include a resistive heater circuit formed on the first side **320** of the substrate **318,** a sensing circuit **326** formed on the second side **322** of the substrate **318,** and a chemically sensitive film **328** formed over the sensing circuit **326** on the second side **322** of the substrate **318.**

The resistive heater circuit **324** may be formed on the substrate **318** from a heater circuit material using, for example, photolithography. In some embodiments, the heater circuit material may comprise platinum. In particular embodiments, the heater circuit material may be platinum ink comprising from about 70 wt% to about 95 wt% platinum.

The heater circuit material can be, for example, photolithographed on the substrate **318** into a desirable pattern. In particular embodiments, the resistive heater circuit **324** of at least one of the plurality of VOC sensors **306** of the sensor array **304** may have a serpentine (i.e. winding) pattern across a portion of the substrate **318.** For example, in some embodiments, the resistive heater circuit **324** can have a longitudinal trace width **330** of from about 0.288 mm to about 0.352 mm. In further embodiments, the resistive heater circuit **324** can have a longitudinal trace spacing **332** of from about 0.333 mm to about 0.407 mm, for example. In still further embodiments, at least a portion of the resistive heater circuit **324** may have a trace height **334** of about 3.80 mm to about 3.96 mm, an outer trace width **336** of about 4.40 mm to about 4.58 mm, and a trace thickness (i.e. depth) of about 0.19 mm to about 0.24 mm, including about 0.21 mm.

The first side **320** of the VOC sensor **306** substrate **318** may also include one or more terminals **338, 340.** For example, as shown in **FIG. 4A,** the first side **320** of substrate **318** includes at least two terminals **338, 340,** which are each operatively connected to a portion (e.g. opposite ends) **342, 344** of the resistive heater circuit **324.**

Turning now to **FIG. 4B,** the sensing circuit **326** may be formed on the substrate **318** from a sensing circuit material using, for example, photolithography. In some embodiments, the sensing circuit material may comprise platinum. In particular embodiments, the sensing circuit material may comprise a platinum ink having from about 70 wt% to about 95 wt% platinum.

The sensing circuit material can be, for example, photolithographed on the substrate **318** into a desirable pattern. In particular embodiments, the sensing circuit **326** includes a first sensing element **346** and a second sensing element **348** that form a pair of extended inter-digitated contacts (i.e. alternating, un-connected contacts in close proximity). The first sensing element **346** may comprise a plurality of extended contacts **350,** wherein each contact **350** has a latitudinal trace width **354** of from about 0.162 mm to about 0.198 mm, a latitudinal trace spacing **356** of from about 0.738 mm to about 0.902 mm, and a trace thickness (i.e. depth) of about 0.19 mm to about 0.24 mm. For example, the contacts **350** may have a latitudinal trace width **354** of about 0.18 mm, a latitudinal trace spacing **356** of about 0.82 mm, and a trace thickness of about 0.21 mm.

Similarly, the second sensing element **348** may comprise a plurality of extended contacts **352,** wherein each contact **352** has a latitudinal trace width **358** of from about 0.162 mm to about 0.198 mm, a latitudinal trace spacing **360** of from about 0.738 mm to about 0.902 mm, and a trace thickness (i.e. depth) of about 0.19 mm to about 0.24 mm. For example, the contacts **354** may have a latitudinal trace width **358** of about 0.18 mm, a latitudinal trace spacing **360** of

about 0.82 mm, and a trace thickness of about 0.21 mm.

In some embodiments, each of the first and second sensing elements **346, 348** may include at least three contacts **350, 352,** and have a latitudinal trace spacing **362** between each contact **350, 352** of the first and second sensing elements **346, 348** of from about 0.288 mm to about 0.352 mm, including about 0.32 mm. Further, each of the contacts **350, 352** may have a longitudinal trace length **364** of about 3.0 mm to about 4.0 mm, including about 3.8 mm.

The second side **322** of the substrate **318** may also comprise one or more terminals **366, 368** which may be operatively connected to a portion **370, 372** of the sensing circuit 326.

Additionally, the contacts **350, 352** of the sensing circuit **326** may be over-coated with a coating composition to form the chemically sensitive film **328.** In some embodiments, the coating composition may comprise a gel, and the film **328** may be formed by applying the coating composition to a portion of the substrate **318** (e.g. a portion covering the contacts **350, 352),** and then drying and calcining the coating composition at a high temperature such as, for example, from about 400°C to about 900°C, including from about 500°C to about 700°C.

In particular embodiments, the film **328** may be a metal oxide film, such as a tin oxide ($SnO_2$) semiconductor film. In such embodiments, the coating composition can comprise tin oxide produced using a water-based gel. In certain embodiments, the gel is made by a sol-gel process involving SnCl4 to form an acidic tin solution, which is neutralized to produce a $SnO_2$ gel. A nano-crystalline $SnO_2$ film **328** is then formed on the substrate **318,** for example, by spin coating the aqueous $SnO_2$ gel onto the sensor side **322** of the substrate **318,** drying the sensor **306** at a first temperature, and then calcining at a second temperature. In particular embodiments, the first temperature at which drying occurs is from about 100°C to about 150°C, and may preferably be about 130°C. In further embodiments, the second temperature at which calcining occurs is from about 400°C to about 900°C, and may preferably be from about 700°C to about 800°C. Importantly, these temperature ranges create a pore size distribution and particle size distribution that provides excellent sensitivity in the chemically sensitive films **328.**

Due to the chemical structures of the target VOCs and the operating conditions of each of the VOC sensors **306,** when the target VOCs (e.g. marker gases) come into contact with the chemically-sensitive film **328,** the number of electrons available in the conduction band of the film **328** may be affected (i.e. increased or decreased). In particular embodiments, the one or more of the target VOCs may be a "reducing gas", which donate additional electrons to the film's **328** conduction band, thereby reducing the resistance of film **328,** which may then be measured as a change in conductance of the film **328.** This effect can be seen in **FIGS. 19A-19G.**

Certain target pheromones, semiochemicals, and kairomones may comprise a six-membered carbon ring and one or more carbonyl groups (-C=O). This is the region of the molecule in which excess electron density is located, which allows for interaction with the semiconductor film **328,** contributing charge carriers to the conduction band of the film **328** (i.e. decreasing the resistance of the film **328**). The chemical structures for two semiochemicals are shown below in **Table 3:**

**TABLE 3. Semiochemical/Kairomone Chemical Structures**

| SPI | Chemical Formula | Chemical Structure |
|---|---|---|
| **Indian meal moth larvae** *Plodia interpunctella* | 2-palmitoyl-1,3-cyclohexanedione | 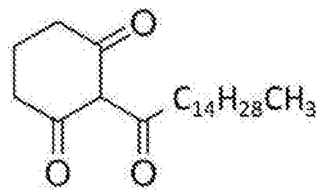 |
| **Indian meal moth larvae** *Plodia interpunctella* | 2-oleoyl-1,3-cyclohexanedione | 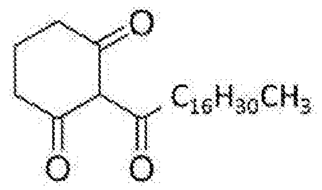 |

In preferred embodiments, the sensor array **304** includes a plurality of differentiated VOC sensors **306.** That is, the composition of one or more of the plurality of VOC sensors **306** are varied and optimized for specific detection needs. For example, the coating composition used to form the film **328** may include one or more catalysts or dopants (e.g. doping agents), which may be added while the gel coating composition is being made. In some embodiments, the coating composition including a doping agent. In some embodiments, the doping agent may be, for example, a transition metal. For example, the doping agent may be selected from a group consisting of: platinum; palladium; molybdenum;

tungsten; nickel; ruthenium; and osmium. As a result of the addition of a doping agent to a film **328** of a VOC sensor **306,** each VOC sensor **306** may be optimized for a given gas or target VOC.

In particular embodiments, the device **300** may include a plurality of VOC sensors **306** wherein at least one of the VOC sensors **306** is optimized for a particular gas or target VOC by the addition of a catalyst or dopant (i.e. doping agent). In further embodiments, each of the VOC sensors **306** present in the device **300** is optimized for a particular gas or target VOC by the addition of a catalyst or dopant (i.e. doping agent). For example, in specific embodiments, a sensor array **304** may include a first VOC sensor **306** configured to detect IMM larvae semiochemicals, a second VOC sensor **306** configured to detect an adult IMM pheromone, a third VOC sensor **306** configured to detect one or more egg-specific VOCs, and one or more VOC sensors **306** configured to detect potential interferent and/or background gases; however, other combinations and quantities of VOC sensors **306** are contemplated. In one such embodiment, the sensor array **304** may include a first and second VOC sensor **306** configured to detect IMM larva semiochemicals, a third VOC sensor **306** configured to detect an egg-specific VOC, a fourth VOC sensor **306** configured to detect an adult IMM pheromone, and as many as three VOC sensors **306** configured for potential interferent and/or background gases. Potential interferent and/or background gases may comprise, for example, hydrocarbons, alcohols, esters, and/or aldehydes.

Each of the VOC sensors **306** of the device **300** may be positioned within the chamber **308** such that the chemically sensitive film **328** is able to be exposed to a fluid flow that enters the chamber **308.** With reference to **FIG. 5,** in particular embodiments, each of the VOC sensors **306** may be suspended, for example, in a holder **500** using wire bonding **502, 504, 506, 50, 510, 512** to hold up the sensor **306** and to connect various sensor **306** terminals **340, 342, 366, 368** to contacts **514, 516, 518, 520, 522, 524** of the sensor holder **500.**

With further reference to **FIG.** 6, a side view of the device **300** is shown in accordance with certain aspects of this disclosure. In particular, the device **300** illustrates a sensor array **304** comprising six VOC sensors **306** (not visible) being suspended within a chamber **308** by sensor holders **500.** Further, in accordance with some embodiments, a portion **526** of each of the sensor holders **500** may operatively engage an adapter **528** operatively connecting holders **500** and VOC sensors **306** to a circuit board **530** of the device **300,** which allows for power to be supplied to the VOC sensors **306** and for measurements to be taken, for instance.

In other words, the sensor array **304** may be operatively connected to a controller **374** configured to perform one or more steps of the methods described above. In particular, the controller **374** may be configured to: heat one or more of the plurality of VOC sensors **306** to at least a first operating temperature; measure the conductance of one or more of the plurality of VOC sensors **306;** determine a set of conductance change values corresponding to each of the one or more VOC sensors **306** contacted with a fluid flow; and determine a gas component concentration for one or more of the target VOCs within the fluid flow based on the set of conductance change values.

With reference to **FIG. 7,** a perspective view of the device **300** is shown in accordance with certain aspects of this disclosure. As illustrated, the external housing **708** of the device **300** may have a height **709,** width **711,** and depth **713** may each be less than 12.7 cm (5 inches). In some embodiments, the external housing **708** of the device **300** can have a height **709** of from about 7.62 cm to about 10.16 cm (3 inches to about 4 inches), including about 8.636 cm (3.4 inches), a width **709** of from about 10.16 cm to about 12.7 cm (4 inches to about 5 inches), including about 12.39 cm (4.88 inches), and a depth **713** of from about 10.16 cm to about 12.7 cm (4 inches to about 5 inches), including about 10.59 cm (4.17 inches). However, other dimensions are contemplated.

Returning to **FIG. 3,** additional components of the infestation detection system **302** are described in accordance with various aspects of the subject application. A system **302** is provided for identifying an insect infestation of a stored product, the system **302** comprising the sensor array **304** as previously described. Further, in particular embodiments, the system **302** includes a testing chamber **308** enclosing the sensor array **304,** an air transfer unit **376,** and a controller **374** operatively connected to the air transfer unit **376** and the sensor array **304.**

The air transfer unit **376** can comprise, in various embodiments, a valve **378** for controlling the fluid flow through the system **302,** a pump **380** for retrieving (or drawing in) a fluid flow from outside the system **302** and for delivering (or pushing) the fluid flow through the system **302,** and a fluid flow sensor **382** for measuring the amount (e.g. a volume) of fluid that is retrieved by the air transfer unit **376.** In particular embodiments, the fluid flow sensor **382** may be a mass flow control valve or a differential pressure transducer. In further embodiments, the valve **378** and pump **380** may be user actuated. That is, an associated operator of the system **302** may direct (e.g. physically trigger) the retrieval of an external fluid flow using the air transfer unit **376.**

The air transfer unit **302** may also define a fluid flow path of a fluid flow 384 from outside the system **302,** to a flow **314** into the inlet **310** of the device **300,** and to a flow **316** exiting the outlet **312** of the device **300.** Portions of the fluid flows **314, 316, 384** may be transmitted along a fluid flow carrier, such as polymer tubing.

Additionally, the air transfer unit **376** can be operatively connected to the controller **374,** such that the controller **374** may operate the air transfer unit **376** to retrieve a fluid flow from and deliver the fluid flow to the chamber **308,** where the fluid flow can be in fluid contact with the VOC sensors **306.** In particular embodiments, the controller **374** may, for example, measure the amount (e.g. volume) of the fluid flow entering the system **302** and instruct the air transfer unit **376** (e.g. the pump **380** and/or valve **378)** to stop drawing in fluid (e.g. air) once the measured amount reaches a pre-determined threshold. In some embodiments, the pre-determined threshold is a volume sufficient for the device **300** to detect and measure the presence of one or more target VOCs in the fluid flow.

The controller **374** of the system **302** can be operatively connected to the air transfer unit **376** and the sensor array **304,** and may comprise a processor and a memory. The controller **374** may be further configured to: operate the air transfer unit **376** to retrieve a fluid flow (e.g. fluid flow **378**) from outside the system **302** and deliver the fluid flow (e.g. fluid flow **314**) to the testing chamber **308,** wherein the plurality of VOC sensors **306** are in fluid contact with the fluid flow **314;** operate the sensor array **304** to heat one or more VOC sensors **306** to at least a first operating temperature and measure the conductance for one or more of the plurality of VOC sensors **306;** determine a set of conductance change values corresponding to each of the one or more VOC sensors **306;** and determine a gas component concentration for one or more target VOCs within the fluid flow **314** based on the set of conductance change values.

In some embodiments, the system **302** further includes a user interface component(s) **380.** The user interface **380** may be operatively connected to the controller **374,** and the controller **374** can be configured to operate the user interface **380** to display and/or communicate the results of the testing performed via the system **302** to an associated user. The user interface **380** may be a dedicated display **382** visible to a user or operator of the system **302,** such as a display comprising a TFT LCD screen, an IPS LCD screen, a capacitive touchscreen LCD, an LED screen, an OLED screen, an AMOLED screen, or the like. In further embodiments, the user interface **380** may comprise a wired or wireless communications protocol **384,** such as Bluetooth, BLE, Wi-Fi, 3G, 4G, 5G, LTE, or the like, and the user interface **380** may be configured to communicate the results of the analysis to a secondary device **386** (e.g. a mobile phone, tablet, computer, etc.) of an associated user via the communication protocol.

The system **302** may also comprise a power supply **388** that is operatively connected to at least one of the air transfer unit **376,** the device **300,** the controller **374,** and the user interface **380.** The power supply **388** may be configured to deliver power to one or more of the components of the system **302,** while the controller **374** can be configured to operate the power supply **388.** In particular embodiments, the power supply **388** may be integrated into the system **302.** In further embodiments, the power supply **388** may be a removable, external accessory. In some embodiments, the power supply **388** may be a rechargeable power supply **388.**

The various components of the systems described are now discussed in more detail with reference to **FIG. 8.** As shown, **FIG.** 8 illustrates a block diagram of a system **700** for identifying an insect infestation of a stored product by, for example, detecting presence and measuring the level of one or more target VOCs. The system **700** includes a sensory array **306** comprising a controller **374** having a processor **702,** a memory **704,** and one or more input/output (I/O) interfaces **706, 708.** A bus **710** may operatively connect the processor **702,** memory **704,** and the I/O interfaces **706, 708** together. The memory **704** includes instructions **712** for performing one or more steps of the methods disclosed herein, and the processor **702,** in communication with the memory **704,** is configured to execute the instructions for performing the one or more steps.

As illustrated, the system **700** may also include a sensor array **304** comprising a plurality of VOC sensors **306,** as well as an air transfer unit **376** and a user interface **380.** The processor **702** may also control the overall operation of the system **700,** including the operation of the sensor array **304,** the air transfer unit **376,** and the user interface **380.**

The memory **704** may represent any type of non-transitory computer readable medium such as random-access memory (RAM), read only memory (ROM), magnetic disk or tape, optical disk, flash memory, or holographic memory. In one embodiment, the memory **704** comprises a combination of random-access memory and read only memory. In some embodiments, the processor **702** and memory **704** may be combined in a single chip. The input/output (I/O) interfaces **706, 708** allow the controller **374** to communicate with other components of the system **700,** such as the sensor array **304,** the fluid flow sensor **382,** the air transfer unit **376,** and the user interface **380,** via wired or wireless connections. The digital processor **702** can be variously embodied, such as by a single-core processor, a dual-core processor (or more generally by a multiple-core processor), a digital processor, and cooperating method coprocessor, a digital controller, or the like.

The term "software," as used herein, is intended to encompass any collection or set of instructions executable by a computer or other digital system so as to configure the computer or other digital system to perform the task that is the intent of the software. The term "software" is intended to encompass such instructions stored in storage mediums such as RAM, a hard disk, optical disk, or so forth, and is also intended to encompass so-called "firmware" that is software stored on a ROM or so forth. Such software may be organized in various ways, and may include software components organized as libraries, Internet-based programs stored on a remote server or so forth, source code, interpretive code, object code, directly executable code, and so forth. It is contemplated that the software may invoke system-level code or calls to other software residing on a server or other location to perform certain functions.

The instructions **712** of the controller **374** can include in various embodiments a conductance change module **714,** a specific net conductance ("SNC") data module **716,** a gas flow management module **718,** an operating temperature module **720,** a VOC concentration module **722,** and a report output module **724,** for example.

The conductance change module **714** can be configured to measure the conductance of one or more VOC sensors **306** of the sensor array **304** and record the conductance data **728** in memory **704.** That is, in particular embodiments, the conductance change module **714** can be configured to instruct the processor **702** to measure the bulk resistance change of the chemically sensitive film **328** of the one or more VOC sensors **306** using the respective sensing circuits **326.** Thus, the conductance change module **714** may be configured to measure and receive, via I/O interface **706,** conductance signals from the VOC sensors **306** of the sensor array **304,** and store the conductances in the memory **306** as conductance data **728.** The conductance change module **714** may also be configured to, for example, minimize electronic noise and drift of the conductance signals measured from the VOC sensors **306** to ensure accurate and precise measurements. In some embodiments, the conductance change module **714** may be configured to apply, for example, a signal model and/or algorithm to manage or eliminate the problems of conductance drift and electronic noise in the measurement of sensor conductance. In further embodiments, the conductance change module **714** may be configured to adjust the conductance values of the one or more VOC sensors by measuring the conductance of the VOC sensors and raising and/or lowering the operating temperature of one or more of the VOC sensors (via the operating temperature module **720**) until the conductance value for a VOC sensor matches a previously determined baseline conductance value.

The SNC data module **716** can be configured to determine the specific net conductance ("SNC") of one or more of the VOC sensors **306** of the sensor array **304,** as described previously. In particular, the SNC data module **716** and the conductance change module **714** may operate to measure and receive, via I/O interface **706,** certain conductance signals (e.g. conductance values of the VOC sensors contacted with a control fluid flow and/or a sample fluid flow absent target VOCs). Then, the SNC data module may determine a set of SNC values for the VOC sensors **306,** and store the set of SNC values as SNC data **726** in the memory **704.**

The gas flow management module **718** can be configured to operate the air transfer unit **326** to retrieve a fluid flow (e.g. fluid flow **384**), deliver the fluid flow to the device **300,** and purge the fluid flow (e.g. fluid flow **316**) from the system **302.** In particular, the gas flow management module **718** may be configured to receive, via I/O interface **706,** gas flow data **730** from the fluid flow sensor **382** of the air transfer unit **376.** For example, the gas flow data **730** may include a fluid intake threshold (e.g. volume) and measurements from the flow sensor **382,** which may be stored in memory **704.** Additionally, the gas flow management module **718** may be configured to operate the air transfer unit **376,** including the valve **378** and pump **380,** as well as the inlet **310** and outlet **312** controlling the fluid flow path through the system **302.**

The operating temperature module **720** can be configured to operate the heater circuits **324** of the VOC sensors **306** of the sensor array **304** via I/O interface **706.** In particular, the operating temperature module **720** may be configured to heat one or more of the VOC sensors **306** to at least a first operating temperature and a second operating temperature by instructing that power be applied to the heating circuits **324** of the VOC sensors **306.** The operating temperature module **720** may further be configured to monitor the temperature of each of the **VOC** sensors **306** of the sensor array **304,** and to adjust the power supplied to regulate the operating temperature(s) of the VOC sensors **306.** The temperature module **720** may store the set-point operating temperature(s) of the VOC sensors **306,** as well as the measured temperatures as temperature **732** in the memory **704.**

The VOC concentration module **722** can be configured to determine a gas component concentration for one or more target VOCs in a fluid flow, as described above. One or more of the target VOCs may be in a gaseous form within the fluid flow (e.g. an air flow). In particular embodiments, one or more of the target VOCs is at least one of: a pheromone; a semiochemical; and a kairomone. In further embodiments, at least one of the one or more target VOCs within the fluid flow may be selected from a group consisting of: 4,8-dimethyldecanal (Z,Z)-3,6-(11R)-Dodecadien-11-olide; (Z,Z)-3,6-Dodecadienolide; (Z,Z)-5,8-(11R)-Tetradecadien-13-olide; (Z)-5-Tetradecen-13-olide; (R)-(Z)-14-Methyl-8-hexadecenal; (R)-(E)-14-Methyl-8-hexadecen-al; γ-ethyl-γ-butyrolactone; (Z,E)-9,12-Tetradecadienyl acetate; (Z,E)-9,12-Tetra-decadien-1-ol; (Z,E)-9,12-Tetradecadienal; (Z)-9-Tetradecenyl acetate; (Z)-11-Hexa-decenyl acetate; (2S,3R,1'S)-2,3-Dihydro-3,5-dimethyl-2-ethyl-6(1-methyl-2-oxobutyl)-4H-pyran-4-one; (2S,3R,1'R)-2,3-Dihydro-3,5-dimethyl-2-ethyl-6(1-methyl-2-oxobutyl)-4H-pyran -4-one; (4S,6S,7S)-7-Hydroxy-4,6-dimethylnonan-3-one; (2S,3S)-2,6-Diethyl-3,5-dimethyl-3,4-dihydro-2H-pyran; 2-Palmitoyl-cyclohexane-1,3-dione; and 2-Oleoyl-cyclo-hexane-1,3-dione. However, other pheromones, semiochemicals, and kairomones are contemplated. The determined concentration for one or more of these target VOCs may be stored in the memory as VOC data **734.**

The report output module **724** can be configured to develop the desired system output **738** and operate a user interface **380,** via I/O interface **380,** to communicate the output **738** to an associated user of the system **302.** In particular embodiments, the user interface **380** may a dedicated display or may be a secondary user device (e.g. a PC, such as a desktop, a laptop, palmtop computer, portable digital assistant (PDA), server computer, cellular telephone, tablet computer, mobile devices, and the like, or a combination thereof). In some embodiments, the user interface **380** may include a speaker or speaker system. Thus, in some embodiments, the I/O interface **708** may be a wired communication interface. In other embodiments, the I/O interface **708** may comprise a wireless communication component, and communication with the user interface **380** may occur via a wireless communications protocol, such as Bluetooth, BLE, Wi-Fi, 3G, 4G, 5G, LTE, or the like.

In either case, the system output **738** may be communicated via the user interface **380** in various embodiments,

such as a graph, chart, table, or data set, for example, illustrating the determined VOC data. In some embodiments, the output **738** may include an audible component, such as an audio tone, set of tones, or audible words, which may be communicated via a speaker or speaker system of the user interface **380.** The audible output component may be a tone sounding at a frequency that varies based on the gas component concentration(s) of one or more of the target VOCs detected (e.g. increase frequency with higher detection levels). In particular embodiments, the output **738** comprises a determination of whether an insect infestation is likely present within a stored product. In further embodiments, the output **738** may include an estimate for probable cause of infestation (e.g. identifying one or more particular SPI based on the VOC data). In still further embodiments, the output **738** may include a recommendation for taking remedial action to protect the value of the stored product, such as fumigation.

## EXAMPLES

The following specific examples describe novel aspects of the present disclosure and procedures used therein. They are intended for illustrative purposes only and should not be construed as a limitation upon the broadest aspects of the invention.

## EXAMPLE 1

With reference to **FIGS. 9A-9D,** provided are graphs of laboratory bench tests of various embodiments of VOC sensor chips and their sensitivity to pheromones. Adult insect pheromones were made into test gases at a concentration of 2 ppm in dry nitrogen in an A31 compressed gas cylinder. This test gas was diluted with additional dry nitrogen to achieve a gas stream with pheromone concentrations between 100 ppb and 300 ppb. This gas stream was injected into the pre-prototype device and the net conductance was determined. The following charts show the response of five different sensors, one with no catalyst added, four with the catalysts Pd, Pt, Os and W added. The W catalyst provides excellent sensitivity for the IMM pheromone (**FIG. 9A**), for the cigarette beetle pheromone (**FIG. 9C**), and for the warehouse beetle pheromone (**FIG**. **9D**). The Pd catalyst shows excellent sensitivity for the red flour beetle pheromone (**FIG**. **9B**). The other catalysts are less effective in sensitive response to the pheromones.

## EXAMPLE 2

With reference to **FIGS. 10A-10C, FIGS. 11A-11C,** and **FIGS. 12A-12C,** provided are experimental results of field testing of sensor chip response to headspace over products with insects. In a field trial, the headspace gas over a 10 lb. sample of clean white wheat flour was injected into the pre-prototype device to establish a baseline resistance value. Once the baseline resistance value was established, the headspace gas over a companion 4.54 Kg (10 lb) sample of clean white wheat flour into which vials containing different numbers of the four live insects, IMM, red flour beetle, warehouse beetle and cigarette beetle were injected. The resistance data for the headspace gas over product with live insects embedded is shown for an uncatalyzed chip **(FIGS. 10A-10B),** a Pt-catalyzed chip **(FIGS. 11A-11C),** an Os-catalyzed chip **(FIGS. 12A-12C),** and a W-catalyzed chip **(FIGS. 13A-13C).**

As seen in each case, a decrease in resistance is clear with an increase in insect population. Additional insects produce additional pheromone in the headspace. A higher pheromone concentration causes a reduction in sensor chip resistance. Thus, the sensor chips are able to produce a signal dependent on the insect population. This signal can be analyzed and a correlation between insect population and signal can be established.

With respect to **FIGS. 14A-14D,** graphs are provided showing the analytical results of the data discussed above. The raw data was analyzed by converting the chip resistance values, R, into chip conductance values, mathematically represented as K. The net conductance was determined by subtracting the chip conductance when no insects are present, $K_b$ from the chip conductance when insects are present, $K_g$. The net conductance is represented as $\Delta K$ mathematically. Plots of $\Delta K$ vs insect number are shown in **FIGS. 14A-14D.** As a result, these plots allow for selection of the best catalyst for each pheromone: an uncatalyzed chip for IMM; an Os catalyzed chip for warehouse beetle; and an uncatalyzed chip for cigarette beetle, for example.

## EXAMPLE 3

In a third test, an embodiment of the present disclosure was used to detect pheromones and semiochemicals emitted by live adult female IMM, larvae, and larvae in cocoons in a stored food product. Two 37.85 litres (10 gallons) galvanized pails were filled halfway with white wheat flour (approximately 11.34 Kg (25 lbs)). One of the pails was used as a control and did not have any insects, while adult female IMM, IMM larvae, and larvae in cocoons were placed in the other pail. A device in accordance with one aspect of the present disclosure was connected to these pails via stainless steel tubing and a valve system preventing contamination between the "reference" pail and the insect-containing pail.

Jars containing adult insects, larvae, and larvae in cocoons were introduced into the experimental pail.

First, the insect-detecting device obtained baseline resistance readings by sampling the headspace gas from the "reference" pail (i.e. determining a baseline conductance for the VOC sensors by measuring conductance while the VOC sensors are in an atmosphere absent of any target VOCs. Baseline conductance/resistance readings were recorded for approximately 30 minutes or longer.

Then, the insect-detecting device sampled the headspace gas from the insect-containing pail and recorded resistance/conductance measurements for the VOC sensors for approximately 30 minutes or longer. With reference to **FIG. 15,** an example of VOC sensor response is illustrated.

These steps were repeated for several trials with live larvae, larvae in cocoons, and adult female moths. The following table summarizes the tests conducted:

| | Live Larvae | | Cocoons | | Adult Female Moths | |
|---|---|---|---|---|---|---|
| **Chip** | **Known** | **Predicted** | **Known** | **Predicted** | **Known** | **Predicted** |
| **Uncatalyzed** | 5 | 10 | 5 | 4 | 5 | 5 |
| | 100 | 52 | 25 | 26 | 25 | 25 |
| | 225 | 308 | 100 | 99 | 100 | 75 |
| | 325 | 431 | 225 | 224 | | |
| **Blind Test** | 150 | 304 | 150 | 123 | 75 | 51 |
| **W-catalyzed** | 5 | 11 | 5 | 8 | 5 | 5 |
| | 100 | 172 | 25 | 23 | 25 | 25 |
| | 225 | 198 | 100 | 101 | 100 | 20 |
| | 325 | 321 | 225 | 207 | | |
| **Blind Test** | 150 | 286 | 150 | 110 | 75 | 11 |
| **Pt-catalyzed** | 3 | 0 | 5 | 5 | 5 | 3 |
| | 100 | 208 | 25 | 25 | 25 | 21 |
| | 225 | 250 | 100 | 100 | 100 | 99 |
| | 325 | 372 | 225 | 166 | | |
| **Blind Test** | 150 | 324 | 150 | 102 | 75 | 42 |

For each of the larvae, larvae in cocoons, and adult IMMs, a "known" number of insects introduced into the experimental pail was compared with the calculated or "predicted" number of insects present. The resistance data measured by the device was processed in accordance with one embodiment of the present disclosure as described above. In particular, the predicted insect counts were derived from correlation curves created to show the resistance change when the sample fluid flow is changed from the reference pail to the experimental pail. To create the correlation curves, the signal (Net R) must be determined at each time insects are present. The signal is the difference between the resistance of the chip with insects absent (i.e. baseline conductance) and the resistance with insects present. Because the baseline resistance varies with time, the expected baseline resistance is computed using an equation derived by plotting selected baseline resistance values when insects are absent over time. For example, **FIGS. 16A-16C** illustrate the plots for the uncatalyzed chip for the three insect maturity stages. Then, correlation curves are created for each chip. For example, the curves for the uncatalyzed chip are illustrated with a quadratic fit in **FIGS. 17A-17C.**

As seen above, the agreement between the known and predicted numbers is good, with some variation when analyte (i.e. VOCs) concentration is expected to be very low. It is believed that the sensor device responds to the female pheromone for the adults, to the larva semiochemical 2-palmitoyl-1,3-cyclohexanedione for the larvae, to 2-oleoyl-1,3,-cyclohexanedione and 2-palmitoyl-1,3-cyclohexanedione for the cocoons. The larvae build their cocoons using their mandibular secretions (i.e. saliva) that have a high concentration of 2-oleoyl-1,3-cyclohexanedione and the frass that

they produce contains a high concentration of 2-palmitoyl-1,3,-cyclohexanedione. There is some over-estimation for live larvae and some under estimation for adult moths. However, it should be noted that pheromone and semiochemicals production varies with the time of day and is, therefore, not always as consistent as analyte flow in a simulated environment.

## EXAMPLE 4

In a fourth test, an embodiment of the present disclosure was used to detect navel orangeworm (NOW) adult females, larvae, and larvae in cocoons within a stored food product. A number of 946 ml (one-quart) glass jars were each filled with a small amount of white wheat flour in accordance with the following table:

| Jar | Food Product | Insects Present |
|---|---|---|
| Control | White wheat flour | None |
| Ex. 1 | White wheat flour | 50 NOW adult females |
| Ex. 2 | White wheat flour | 50 NOW larvae, approximately 5th instar |
| Ex. 3 | White wheat flour | 50 NOW pupated larvae in cocoons |
| Ex. 4 | White wheat flour | 100 NOW adult females |
| Ex. 5 | White wheat flour | 100 NOW larvae, approximately 5th instar |
| Ex. 6 | White wheat flour | 100 NOW pupated larvae in cocoons |
| Ex. 7 | White wheat flour | 1X of NOW eggs (approx. 100 eggs) |
| Ex. 8 | White wheat flour | 2X of NOW eggs (approx. 200 eggs) |

One jar containing no insects, no larvae, no pheromones, and no semiochemicals was used a reference or control jar, while the other jars would contain the insects. First, a baseline conductance was determined by sampling the headspace of the reference jar. Then, a fluid flow samples from the headspace of one of the experimental jars (e.g. Ex. 1 - Ex. 8) would be tested. The data acquired using a Pd-catalyzed chip operating at 300°C is illustrated in **FIG. 18.** In particular, the vertical arrows indicate when the flow of headspace air from the jar containing the insects began. As seen, the immediate decrease in resistance shows the instantaneous response of the sensor chip to the analyte VOC. From this data, it is clear that the headspace air over the 100 adults, 100 larvae, 100 cocoons, and 2X egg count causes a greater resistance change than does the headspace air over the 50 adults, 50 larvae, 50 cocoons, and 1X egg count. That is, the signal scales with the population or number of adults, larvae, cocoons, and eggs.

The present specification has been set forth with reference to preferred embodiments. Obviously, modifications and alterations will occur to others upon reading and understanding the present specification. It is intended that the invention be construed as including all such modifications and alterations insofar as they come within the scope of the appended claims.

## Claims

**1.** A device for identifying an insect infestation of a stored product by detecting one or more target volatile organic compounds (VOCs) within a target fluid flow, the device comprising:

a sensor array (304) having a plurality of VOC sensors (306), wherein each VOC sensor includes:

a substrate (318);
a resistive heater circuit (324) formed on a first side (320) of the substrate
a sensing circuit (326) formed on a second side (322) of the substrate; and
a chemically-sensitive film (328) formed over the sensing circuit on the second side of the substrate;

**characterized in that** at least one VOC sensor of the plurality of VOC sensors is configured to detect the presence of 11,13-hexadecadienal as an egg-specific VOC.

**2.** The device of claim 1, wherein the sensor array comprises from two to ten VOC sensors.

**3.** The device of claim 1 or 2, wherein the resistive heater circuit of at least one of the plurality of VOC sensors is a serpentine pattern having a longitudinal trace width from about 0.288 mm to about 0.352 mm and a longitudinal trace spacing width from about 0.333 mm to about 0.407 mm.

**4.** The device of any one of claims 1 to 3, wherein the sensing circuit of at least one of the plurality of VOC sensors includes a first sensing element (346) and a second sensing element (348) forming a pair of extended inter-digitated contacts;

wherein the first sensing element comprises a plurality of extended contacts, each contact having a latitudinal trace width of from about 0.162 mm to about 0.198 mm and a latitudinal trace spacing of from about 0.738 mm to about 0.902 mm; and
wherein the second sensing element comprising a plurality of extended contacts, each contact having a latitudinal trace width of from about 0.162 mm to about 0.198 mm and a latitudinal trace spacing of from about 0.738 mm to about 0.902 mm.

**5.** The device of claim 4, wherein each of the first and second sensing elements comprise at least three extended contacts, and wherein the sensing circuit has a latitudinal trace spacing between each extended contact of the first and second sensing elements of from about 0.288 mm to about 0.352 mm.

**6.** The device of any one of claims 1 to 5, wherein at least one of the resistive heater circuit and the sensing circuit is formed from a composition comprising platinum, and the chemically sensitive film is a nano-crystalline tin oxide film formed from an aqueous tin oxide gel, and wherein the chemically sensitive film preferably comprises a doping agent selected from a group consisting of: platinum; palladium; molybdenum; tungsten; nickel; ruthenium; and osmium.

**7.** The device of any one of claims 1 to 6, wherein at least one of the VOC sensors of the plurality of VOC sensors is configured to detect the presence of at least one of the one or more target VOCs selected from a group consisting of: 4,8-dimethyldecanal; (Z,Z)-3,6-(11R)-Dodecadien-11-olide; (Z,Z)-3,6-Dodecadienolide; (Z,Z)-5,8-(11R)-Tetradecadien-13-olide; (Z)-5-Tetradecen-13-olide; (R)-(Z)-14-Methyl-8-hexadecenal; (R)-(E)-14-Methyl-8-hexadecenal; γ-ethyl-γ-butyrolactone; (Z,E)-9,12-Tetradecadienyl acetate; (Z,E)-9,12-Tetra-decadien-1-ol; (Z,E)-9,12-Tetradecadienal; (Z)-9-Tetradecenyl acetate; (Z)-11-Hexa-decenyl acetate; (2S,3R,1'S)-2,3-Dihydro-3,5-dimethyl-2-ethyl-6(1-methyl-2-oxobutyl)-4H-pyran-4-one; (2S,3R,1'R)-2,3-Dihydro-3,5-dimethyl-2-ethyl-6(1-methyl-2-oxobutyl)-4H-pyran-4-one; (4S,6S,7S)-7-Hydroxy-4,6-dimethylnonan-3-one; (2S,3S)-2,6-Diethyl-3,5-dimethyl-3,4-dihydro-2H-pyran; 2-Palmitoyl-cyclohexane-1,3-dione; and 2-Oleoyl-cyclo-hexane-1,3-dione.

**8.** The device of any one of claims 1 to 7, further comprising a controller (374), wherein the sensor array is operatively connected to the controller, the controller being configured to:

measure a conductance for one or more of the plurality of VOC sensors;
determine a set of conductance change values corresponding to each of the one or more VOC sensors contacted with the target fluid flow; and
determine a gas component concentration for one or more of the target VOCs within the target fluid flow based on the set of conductance change values.

**9.** A system for identifying an insect infestation of a stored product, the system comprising:

a device for identifying an insect infestation of a stored product according to claim 8,
a testing chamber (308) enclosing the sensor array, and
an air transfer unit configured to retrieve a fluid flow and deliver the fluid flow to the testing chamber; wherein the controller is operatively connected to the air transfer unit (376) and the sensor array, and wherein the controller is configured to:

operate the air transfer unit to retrieve the fluid flow from and deliver the fluid flow to the testing chamber, wherein one or more of the plurality of VOC sensors are in fluid contact with the fluid flow;
operate the sensor array to measure a conductance for one or more of the plurality of VOC sensors;
determine a set of conductance change values corresponding to each of the one or more VOC sensors; and
determine a gas component concentration for one or more target VOCs within the fluid flow based on the set of conductance change values.

**10.** A method of identifying an insect infestation of a stored product by detecting one or more target volatile organic compounds (VOCs) within a target fluid flow, wherein the target fluid flow is preferably an air sample taken from within a proximity to the stored product being evaluated, the method comprising:

heating, via a device comprising a plurality of VOC sensors, at least one of the plurality of VOC sensors to at least a first operating temperature;
contacting the one or more VOC sensors with the target fluid flow;
determining a set of conductance change values ($\Delta K_i$) corresponding to each of the one or more VOC sensors contacted with the target fluid flow; and
determining a gas component concentration ($[X]_n$) for one or more of the target VOCs within the target fluid flow based on the set of conductance change values, **characterized in that** at least one of the plurality of VOC sensors is configured to detect the presence of 11,13-hexadecadienal as an egg-specific VOC.

**11.** The method of claim 10, wherein the method further comprises:

measuring a signal conductance for the one or more VOC sensors after contacting the one or more VOC sensors with the target fluid flow;
wherein the set of conductance change values ($\Delta K_i$) is determined based on the difference between the signal conductance for each of the one or more VOC sensors contacted with the target fluid flow and a baseline conductance of each of the corresponding VOC sensors.

**12.** The method of claim 11, wherein the baseline conductance for the one or more VOC sensors is measured while the one or more VOC sensors are in an atmosphere absent of any target VOCs, wherein the method preferably further comprises:
adjusting the baseline conductance of one or more of the VOC sensors after being contacted with at least one target VOC to match the baseline conductance of the corresponding VOC sensor prior to contact with the at least one target VOC, wherein the baseline conductance is adjusted by heating one or more of the VOC sensors to at least a second operating temperature.

**13.** The method of any one of claims 10 to 12, wherein the method further comprises:
determining one or more specific net conductance values for one or more of the VOC sensors, wherein each specific net conductance value corresponds to one of the target VOCs, wherein each specific net conductance value corresponding to a target VOC is determined by:

contacting the one or more VOC sensors with a control fluid flow having a known concentration of the target VOC;
measuring a test conductance for each of the one or more VOC sensors; and
for each of the one or more VOC sensors, calculating a specific net conductance value based on the measured test conductance of the VOC sensor and the known concentration of the target VOC within the control fluid flow, wherein the method preferably further comprises:
determining a plurality of specific net conductance values for one or more of the VOC sensors, wherein each of the specific net conductance values for each of the VOC sensors corresponds to a different target VOC.

**14.** The method of any one of claims 10 to 13, wherein the gas component concentration ($[X]_n$) for the one or more target VOCs within the target fluid flow is determined based on the set of conductance change values and the one or more specific net conductance values for each of the one or more of VOC sensors.

**15.** The method of any one of claims 10 to 14, wherein the first operating temperature is between about 180°C and about 400°C.

## Patentansprüche

**1.** Vorrichtung zum Identifizieren eines Insektenbefalls eines gelagerten Produkts durch Detektion von einer oder mehreren flüchtigen organischen Verbindungen (VOCs) als Ziel innerhalb eines Zielfluidstroms, welche Vorrichtung aufweist:

ein Sensorfeld (304), das eine Mehrzahl von VOC-Sensoren (306) aufweist,
bei dem jeder VOC-Sensor aufweist:

ein Substrat (318);
eine Widerstandsheizschaltung (324), die auf einer ersten Seite (320) des Substrates ausgebildet ist;
eine Erfassungsschaltung (326), die auf einer zweiten Seite (322) des Substrates ausgebildet ist; und

einen chemisch-sensitiven Film (328), der über der Erfassungsschaltung auf der zweiten Seite des Substrates ausgebildet ist;

**dadurch gekennzeichnet, dass** mindestens ein VOC-Sensor aus der Mehrzahl von VOC-Sensoren konfiguriert ist zum Detektieren der Anwesenheit von 11,13-Hexadekadienal als einer Eierspezifischen VOC.

**2.** Vorrichtung nach Anspruch 1, bei der das Sensorfeld von zwei bis zehn VOC-Sensoren aufweist.

**3.** Vorrichtung nach Anspruch 1 oder 2, bei der die Widerstandsheizschaltung mindestens eines der Mehrzahl der VOC-Sensoren ein Serpentinenmuster mit einer longitudinalen Spurbreite von ungefähr 0,288 mm bis ungefähr 0,352 mm und einer longitudinalen Spurabstandsbreite von ungefähr 0,333 mm bis ungefähr 0,407 mm ist.

**4.** Vorrichtung nach einem der Ansprüche 1 bis 3, bei der die Erfassungsschaltung mindestens eines der Mehrzahl von VOC-Sensoren ein erstes Erfassungselement (346) und ein zweites Erfassungselement (348) aufweist, die ein Paar von länglichen verzahnten Kontakten bilden, bei der das erste Erfassungselement eine Mehrzahl von länglichen Kontakten aufweist, von denen jeder Kontakt eine latitudinale Spurbreite von ungefähr 0,162 mm bis ungefähr 0,198 mm und eine latitudinale Spurabstandsbreite von ungefähr 0,738 bis ungefähr 0,902 mm aufweist, und
bei der das zweite Erfassungselement eine Mehrzahl von länglichen Kontakten aufweist, von denen jeder Kontakt eine latitudinale Spurbreite von ungefähr 0,162 mm bis ungefähr 0,198 mm und eine latitudinale Spurabstandsbreite von ungefähr 0,738 bis ungefähr 0,902 mm aufweist.

**5.** Vorrichtung nach Anspruch 4, bei der jedes der ersten und zweiten Erfassungselemente mindestens drei längliche Kontakte aufweist, und bei der die Erfassungsschaltung eine latitudinale Spurabstandsbreite zwischen jedem länglichen Kontakt der ersten und zweiten Erfassungselemente von ungefähr 0,288 mm bis ungefähr 0,352 mm aufweist.

**6.** Vorrichtung nach einem der Ansprüche 1 bis 5, bei der mindestens eine Schaltung aus der Widerstandsheizschaltung und der Erfassungsschaltung aus einer Zusammensetzung ausgebildet ist, die Platin enthält, und der chemisch-sensitive Film ein nanokristalliner Zinnoxidfilm ist, der aus einem wässrigen Zinnoxidgel gebildet ist, und bei der der chemisch-sensitive Film bevorzugter Weise ein Dotierungsmittel aufweist, das ausgewählt ist aus einer Gruppe, die besteht aus Platin, Palladium, Molybdän, Wolfram, Nickel, Ruthenium und Osmium.

**7.** Vorrichtung nach einem der Ansprüche 1 bis 6, bei der mindestens einer der VOC-Sensoren aus der Mehrzahl der VOC-Sensoren konfiguriert ist zum Detektieren der Anwesenheit von mindestens einer aus der einen oder mehreren Ziel-VOCs, die ausgewählt ist/sind aus einer Gruppe, die besteht aus: 4,8-Dimethyldecanal; (Z,Z)-3,6-(11R)-Dodekadien-11-olid; (Z,Z)-3,6-Dodekadienolid; (Z,Z)-5,8-(11R)-Tetradekadien-13-olid; (Z)-5-Tetradecen-13-olid; (R)-(Z)-14-Methyl-8-Hexadecenal; (R)-(E)-14-Methyl-8-Hexadecen-al; γ-Ethyl-γ-Butyrolacton; (Z,E)-9,12-Tetradekadienylacetat; (Z,E)-9,12-Tetradekadien-1-ol; (Z,E)-9,12-Tetradekadienal; (Z)-9-Tetradecenylacetat; (Z)-11-Hexadecenylacetat; (2S,3R,1'S)-2,3-Dihydro-3,5-dimethyl-2-ethyl-6(1-methyl-2-oxobutyl)-4H-pyran-4-on; (2S,3R,1'R)-2,3-Dihydro-3,5-dimethyl-2-ethyl-6(1-methyl-2-oxobutyl)-4H-pyran-4-on; (4S,6S,7S)-7-Hydroxy-4,6-dimethylnonan-3-on; (2S,3S)-2,6-Diethyl-3,5-dimethyl-3,4-dihydro-2H-pyran; 2-Palmitoyl-cyclohexan-1,3-dion, und 2-Oleoyl-cyclo-hexane-1,3-dion.

**8.** Vorrichtung nach einem der Ansprüche 1 bis 7, die weiter einen Controller (374) aufweist, bei der das Sensorfeld operativ mit dem Controller verbunden ist, welcher Controller konfiguriert ist zum:

Messen einer Leitfähigkeit für einen oder mehrere der Mehrzahl von VOC-Sensoren,
Bestimmen eines Satzes von Leitfähigkeitsänderungswerten entsprechend zu jedem der einen oder mehreren VOC-Sensoren, die mit dem Zielfluidstrom kontaktiert wurden, und
Bestimmen einer Gaskomponentenkonzentration für eine oder mehrere der Ziel-VOCs innerhalb des Zielfluidstroms basierend auf dem Satz von Leitfähigkeitsänderungswerten.

**9.** System zum Identifizieren eines Insektenbefalls eines gelagerten Produkts, welches System aufweist:

eine Vorrichtung zum Identifizieren eines Insektenbefalls eines gelagerten Produkts nach Anspruch 8,
eine Testkammer (308), die das Sensorfeld einschließt, und
eine Lufttransfereinheit, die konfiguriert ist zum Holen eines Fluidstroms und zum Liefern des Fluidstroms zu der Testkammer, bei dem
der Controller operativ mit der Lufttransfereinheit (376) und dem Sensorfeld verbunden ist, und bei dem der Controller konfiguriert ist zum:

Betreiben der Lufttransfereinheit zum Erhalten des Fluidstroms von und zum Liefern des Fluidstroms zu der Testkammer, wobei einer oder mehrere der Mehrzahl von VOC-Sensoren in Fluidkontakt mit dem Fluidstrom sind,
Betreiben des Sensorfeldes zum Messen einer Leitfähigkeit für einen oder mehrere der Mehrzahl von VOC-Sensoren,
Bestimmen eines Satzes von Leitfähigkeitsänderungswerten entsprechend zu jedem der einen oder mehreren VOC-Sensoren; und
Bestimmen einer Gaskomponentenkonzentration für eine oder mehrere Ziel-VOCs innerhalb des Fluidstroms basierend auf dem Satz von Leitfähigkeitsänderungswerten.

**10.** Verfahren zum Identifizieren eines Insektenbefalls eines gelagerten Produkts durch Detektieren einer oder mehrerer flüchtiger organischer Verbindungen (VOCs) als Ziel innerhalb eines Zielfluidstroms, bei dem der Zielfluidstrom bevorzugter Weise eine Luftprobe ist, die aus der Umgebung des gelagerten Produkts, das zu bewerten ist, entnommen ist, welches Verfahren aufweist:

Erwärmen, über eine Vorrichtung, die eine Mehrzahl von VOC-Sensoren aufweist, mindestens eines der Mehrzahl von VOC-Sensoren auf mindestens eine erste Betriebstemperatur;
Kontaktieren des einen oder der mehreren VOC-Sensoren mit dem Zielfluidstrom;
Bestimmen eines Satzes von Leitfähigkeitsänderungswerten ($\Delta K_i$) entsprechend zu jedem aus dem einen oder mehreren VOC-Sensoren, die mit dem Zielfluidstrom kontaktiert wurden, und Bestimmen einer Gaskomponentenkonzentration ($[X]_n$) für eine oder mehrere der Ziel-VOCs innerhalb des Zielfluidstroms basierend auf dem Satz von Leitfähigkeitsänderungswerten;
**dadurch gekennzeichnet, dass** mindestens einer aus der Mehrzahl von VOC-Sensoren konfiguriert ist zum Detektieren der Anwesenheit von 11,13-Hexadekadienal als einer Eier-spezifischen VOC.

**11.** Verfahren nach Anspruch 10, bei dem das Verfahren weiter aufweist:

Messen einer Signalleitfähigkeit für einen oder mehrere VOC-Sensoren nach dem Kontaktieren des einen oder der mehreren VOC-Sensoren mit dem Zielfluidstrom;
bei dem der Satz von Leitfähigkeitsänderungswerten ($\Delta K_i$) bestimmt wird basierend auf dem Unterschied zwischen der Signalleitfähigkeit für jeden der einen oder mehreren VOC-Sensoren,
die mit dem Zielfluidstrom kontaktiert wurden, und einer Basislinienleitfähigkeit jedes der entsprechenden VOC-Sensoren.

**12.** Verfahren nach Anspruch 11, bei dem die Basislinienleitfähigkeit für den einen oder die mehreren VOC-Sensoren gemessen wird, während der eine oder die mehreren VOC-Sensoren in einer Atmosphäre sind, die keines der Ziel-VOCs enthält, bei dem das Verfahren bevorzugter Weise weiter aufweist:
Einstellen der Basislinienleitfähigkeit des einen oder der mehreren VOC-Sensoren, nachdem sie mit mindestens einem Ziel-VOC kontaktiert wurden, um die Basislinienleitfähigkeit des entsprechenden VOC-Sensors vor einem Kontakt mit dem mindestens einen Ziel-VOC zur Übereinstimmung zu bringen, wobei die Basislinienleitfähigkeit eingestellt wird durch Erwärmen eines oder mehrerer der VOC-Sensoren auf mindestens eine zweite Betriebstemperatur.

**13.** Verfahren nach einem der Ansprüche 10 bis 12, bei dem das Verfahren weiter aufweist:

Bestimmen eines oder mehrerer spezifischer Nettoleitfähigkeitswerte für einen oder mehrere der VOC-Sensoren, wobei jeder spezifische Nettoleitfähigkeitswert einem der Ziel-VOCs entspricht, bei dem jeder spezifische Nettoleitfähigkeitswert, der einem Ziel-VOC entspricht, bestimmt wird durch;
Kontaktieren des einen oder der mehreren VOC-Sensoren mit einem Überprüfungsfluidstrom, der eine bekannte Konzentration des Ziel-VOCs enthält,
Messen einer Testleitfähigkeit für jeden der einen oder mehreren VOC-Sensoren, und
für jeden der ein oder mehreren VOC-Sensoren, Berechnen eines spezifischen Nettoleitfähigkeitswerts basierend auf der gemessenen Testleitfähigkeit des VOC-Sensors und der bekannten Konzentration des Ziel-VOCs innerhalb des Überprüfungsfluidstroms, bei dem das Verfahren bevorzugter Weise weiter aufweist:
Bestimmen einer Mehrzahl von spezifischen Nettoleitfähigkeitswerten für einen oder mehrere der VOC-Sensoren, bei dem jeder der spezifischen Nettoleitfähigkeitswerte für jeden der VOC-Sensoren einem unterschiedlichen Ziel-VOC entspricht.

14. Verfahren nach einem der Ansprüche 10 bis 13, bei dem die Gaskomponentenkonzentration ($[X]_n$) für die eine oder mehreren der Ziel-VOCs innerhalb des Zielfluidstroms bestimmt wird basierend auf dem Satz von Leitfähigkeitsänderungswerten und dem einen oder den mehreren spezifischen Nettoleitfähigkeitswerten für jeden der einen oder mehreren VOC-Sensoren.

15. Verfahren nach einem der Ansprüche 10 bis 14, bei dem die erste Betriebstemperatur zwischen ungefähr 180°C und ungefähr 400°C liegt.

## Revendications

1. Dispositif permettant d'identifier une infestation d'insectes sur un produit stocké en détectant un ou plusieurs composés organiques volatils (COV) cibles dans un écoulement de fluide cible, le dispositif comprenant :
un réseau de capteurs (304) ayant une pluralité de capteurs COV (306), dans lequel chaque capteur COV comprend :

    un substrat (318) ;
    un circuit de chauffage résistif (324) formé sur une première face (320) du substrat ;
    un circuit de détection (326) formé sur une deuxième face (322) du substrat ; et
    un film chimiquement sensible (328) formé sur le circuit de détection sur la deuxième face du substrat ;
    **caractérisé en ce qu'**au moins un capteur COV de la pluralité de capteurs COV est configuré pour détecter la présence de 11,13-hexadécadiénal en tant que COV spécifique à l'œuf.

2. Dispositif de la revendication 1, dans lequel le réseau de capteurs comprend de deux à dix capteurs COV.

3. Dispositif de la revendication 1 ou 2, dans lequel le circuit de chauffage résistif d'au moins un des capteurs COV est un motif en serpentin ayant une largeur de trace longitudinale d'environ 0,288 mm à environ 0,352 mm et une largeur d'espacement de trace longitudinale d'environ 0,333 mm à environ 0,407 mm.

4. Dispositif de l'une quelconque des revendications 1 à 3, dans lequel le circuit de détection d'au moins un de la pluralité des capteurs COV comprend un premier élément de détection (346) et un deuxième élément de détection (348) formant une paire de contacts interdigités étendus :

    dans lequel le premier élément de détection comprend une pluralité de contacts étendus, chaque contact ayant une largeur de trace latitudinale d'environ 0,162 mm à environ 0,198 mm et un espacement de trace latitudinale d'environ 0,738 mm à environ 0,902 mm ; et
    dans lequel le deuxième élément de détection comprend une pluralité de contacts étendus, chaque contact ayant une largeur de trace latitudinale d'environ 0,162 mm à environ 0,198 mm et un espacement de trace latitudinale d'environ 0,738 mm à environ 0,902 mm.

5. Dispositif de la revendication 4, dans lequel chacun des premier et deuxième éléments de détection comprend au moins trois contacts étendus, et dans lequel le circuit de détection a un espacement de trace latitudinale entre chaque contact étendu des premier et deuxième éléments de détection d'environ 0,288 mm à environ 0,352 mm.

6. Dispositif de l'une quelconque des revendications 1 à 5, dans lequel au moins l'un du circuit de chauffage résistif et le circuit de détection sont formés à partir d'une composition comprenant du platine, et le film chimiquement sensible est un film d'oxyde d'étain nanocristallin formé à partir d'un gel aqueux d'oxyde d'étain, et dans lequel le film chimiquement sensible comprend de préférence un agent dopant choisi dans un groupe comprenant : le platine ; le palladium ; le molybdène ; le tungstène ; le nickel ; le ruthénium ; et l'osmium.

7. Dispositif de l'une quelconque des revendications 1 à 6, dans lequel au moins un des capteurs COV de la pluralité de capteurs COV est configuré pour détecter la présence d'au moins un parmi le ou les COV cibles choisis dans un groupe constitué de : le 4,8-diméthyldécanal ; le (Z,Z)-3,6-(11R)-Dodécadien-11-olide ; le (Z,Z)-3,6-Dodécadienolide ; le (Z,Z)-5,8-(11R)-Tétradécadien-13-olide ; le (Z)-5-Tétradécène-13-olide ; le (R)-(Z)-14-Méthyl-8-hexadécénal ; le (R)-(E)-14-Méthyl-8-hexadécén-al ; le γ-éthyl-γ-butyrolactone ; l'acétate de (Z,E)-9,12-Tétradécadiényle ; le (Z,E)-9,12-Tétra-décadién-1-ol ; le (Z,E)-9,12-Tétradécadiénal ; l'acétate de (Z)-9-Tétradécényle ; l'acétate de (Z)-11-Hexa-décényle ; le (2S,3R,1'S)-2,3-Dihydro-3,5-dimethyl-2-ethyl-6(1-méthyl-2-oxobutyl)-4H-pyran-4-one; le (2S,3R,1'R)-2,3-Dihydro-3,5-diméthyl-2-éthyl-6(1-méthyl-2-oxobutyl)-4H-pyran-4-one ; le (4S,6S,7S)-7-Hydroxy-4,6-diméthylnonan-3-one ; le (2S,3S)-2,6-Diéthyl-3,5-diméthyl-3,4-dihydro-2H-pyran ; le 2-Palmitoyl-cyclohexane-1,3-dione ; et le 2-Oleoyl-cyclo-hexane-1,3-dione.

**8.** Dispositif de l'une quelconque des revendications 1 à 7 comprend en outre un dispositif de commande (374), dans lequel
le réseau de capteurs est connecté de manière fonctionnelle au dispositif de commande, le dispositif de commande étant configuré pour :

mesurer une conductance d'un ou de plusieurs de la pluralité de capteurs COV ;
déterminer un ensemble de valeurs de changement de conductance correspondant à chacun du ou des capteurs COV en contact avec l'écoulement de fluide cible ; et
déterminer une concentration de composants gazeux pour un ou plusieurs des COV cibles dans l'écoulement de fluide cible sur la base de l'ensemble des valeurs de changement de conductance.

**9.** Système d'identification d'une infestation d'insectes sur un produit stocké, comprenant :

un dispositif permettant d'identifier une infestation d'insectes sur un produit stocké selon la revendication 8,
une chambre d'essai (308) renfermant le réseau de capteurs, et
une unité de transfert d'air configurée pour récupérer un écoulement de fluide et acheminer l'écoulement de fluide vers la chambre d'essai ; dans lequel le dispositif de commande est connecté de manière fonctionnellement à l'unité de transfert d'air (376) et au réseau de capteurs, et dans lequel le dispositif de commande est configuré pour :

faire fonctionner l'unité de transfert d'air pour récupérer l'écoulement de fluide et acheminer l'écoulement de fluide vers la chambre d'essai, dans lequel un ou plusieurs de la pluralité de capteurs COV sont en contact avec l'écoulement de fluide ;
faire fonctionner le réseau de capteurs pour mesurer une conductance d'un ou de plusieurs capteurs COV ;
déterminer un ensemble de valeurs de changement de conductance correspondant à chacun du ou des capteurs COV ; et
déterminer une concentration de composants gazeux pour un ou plusieurs COV cibles dans l'écoulement de fluide sur la base de l'ensemble des valeurs de changement de conductance.

**10.** Procédé d'identification d'une infestation d'insectes sur un produit stocké par la détection d'un ou plusieurs composés organiques volatils (COV) cibles dans un écoulement de fluide cible, dans lequel l'écoulement de fluide cible est de préférence un échantillon d'air prélevé à proximité du produit stocké à évaluer, le procédé comprenant les étapes consistant à :

chauffer, par l'intermédiaire d'un dispositif comprenant une pluralité de capteurs COV, au moins un de la pluralité de capteurs COV à au moins une première température de fonctionnement ;
mettre en contact le ou les capteurs COV avec l'écoulement de fluide cible ;
déterminer un ensemble de valeurs de changement de conductance ($\Delta K_i$) correspondant à chacun du ou des capteurs COV en contact avec l'écoulement de fluide cible ; et
déterminer une concentration de composants gazeux ($[X]_n$) pour un ou plusieurs des COV cibles dans l'écoulement de fluide cible sur la base de l'ensemble des valeurs de changement de conductance, **caractérisé en ce qu'**au moins un de la pluralité de capteurs COV de la pluralité est configuré pour détecter la présence de 11,13-hexadécadiénal en tant que COV spécifique à l'œuf.

**11.** Procédé de la revendication 10, dans lequel le procédé comprend en outre l'étape consistant à :

mesurer une conductance de signal pour le ou les capteurs COV après avoir mis en contact le ou les capteurs COV avec l'écoulement de fluide cible ;
dans lequel l'ensemble des valeurs de changement de conductance ($\Delta K_i$) est déterminé sur la base de la différence entre la conductance de signal pour chacun du ou des capteurs COV en contact avec l'écoulement de fluide cible et une conductance de ligne de base de chacun des capteurs COV correspondants.

**12.** Procédé de la revendication 11, dans lequel la conductance de ligne de base pour le ou les capteurs COV est mesurée alors que le ou les capteurs COV sont dans une atmosphère dépourvue de tout COV cible, dans lequel le procédé comprend de préférence en outre l'étape consistant à :
ajuster la conductance de ligne base d'un ou de plusieurs capteurs COV après contact avec au moins un COV cible

pour qu'elle corresponde à la conductance de base du capteur COV correspondant avant contact avec au moins un COV cible, dans lequel la conductance de ligne de base est ajustée en chauffant un ou plusieurs des capteurs COV à au moins une deuxième température de fonctionnement.

**13.** Procédé de l'une quelconque des revendications 10 à 12, dans lequel le procédé comprend en outre les étapes consistant à :
déterminer une ou plusieurs valeurs de conductance nette spécifique pour un ou plusieurs des capteurs COV, dans lequel chaque valeur de conductance nette spécifique correspond à l'un des COV cibles, dans lequel chaque valeur de conductance nette spécifique correspondant à un COV cible est déterminée par :

mettre en contact le ou les capteurs COV avec un écoulement de fluide de contrôle ayant une concentration connue du COV cible ;
mesurer une conductance d'essai pour chacun du ou des capteurs COV ; et pour chacun du ou des capteurs COV, calculer une valeur de conductance nette spécifique basée sur la conductance d'essai mesurée du capteur COV et la concentration connue du COV cible dans l'écoulement de fluide de contrôle, dans lequel le procédé comprend de préférence en outre :
déterminer une pluralité de valeurs de conductance nette spécifiques pour un ou plusieurs des capteurs COV, dans lequel chacune des valeurs de conductance nette spécifiques pour chacun des capteurs COV correspondant à un COV cible différent.

**14.** Procédé de l'une quelconque des revendications 10 à 13, dans lequel la concentration de composants gazeux ($[X]_n$) pour le ou les COV cibles dans l'écoulement de fluide cible est déterminée sur la base de l'ensemble des valeurs de changement de conductance et de la ou des valeurs de conductance nette spécifique pour chacun du ou des capteurs COV ou pour plusieurs d'entre eux.

**15.** Procédé de l'une quelconque des revendications 10 à 14, dans lequel la première température de fonctionnement est comprise entre environ 180°C et environ 400°C.

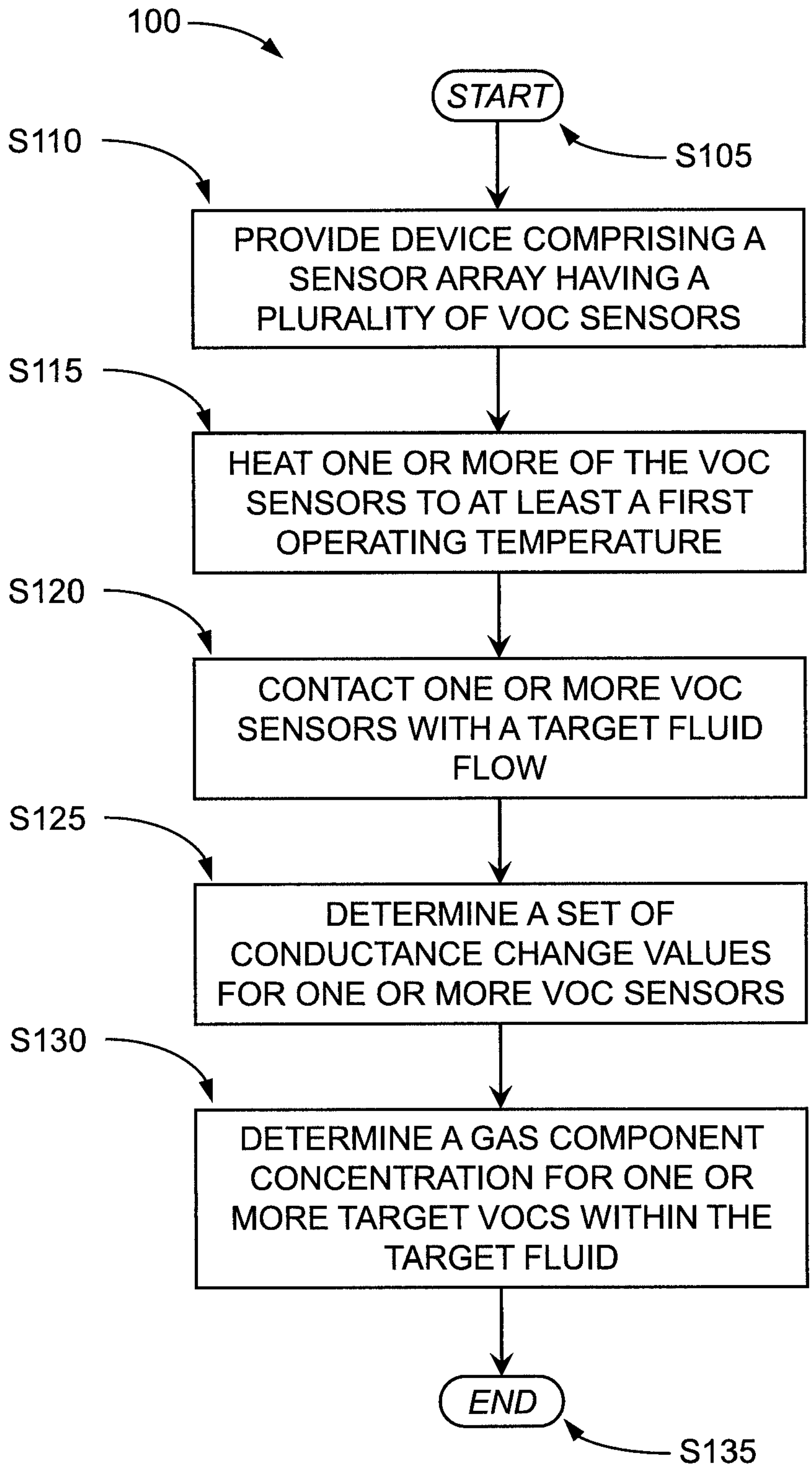
100
START
S105
S110
PROVIDE DEVICE COMPRISING A SENSOR ARRAY HAVING A PLURALITY OF VOC SENSORS
S115
HEAT ONE OR MORE OF THE VOC SENSORS TO AT LEAST A FIRST OPERATING TEMPERATURE
S120
CONTACT ONE OR MORE VOC SENSORS WITH A TARGET FLUID FLOW
S125
DETERMINE A SET OF CONDUCTANCE CHANGE VALUES FOR ONE OR MORE VOC SENSORS
S130
DETERMINE A GAS COMPONENT CONCENTRATION FOR ONE OR MORE TARGET VOCS WITHIN THE TARGET FLUID
END
S135

FIG. 1

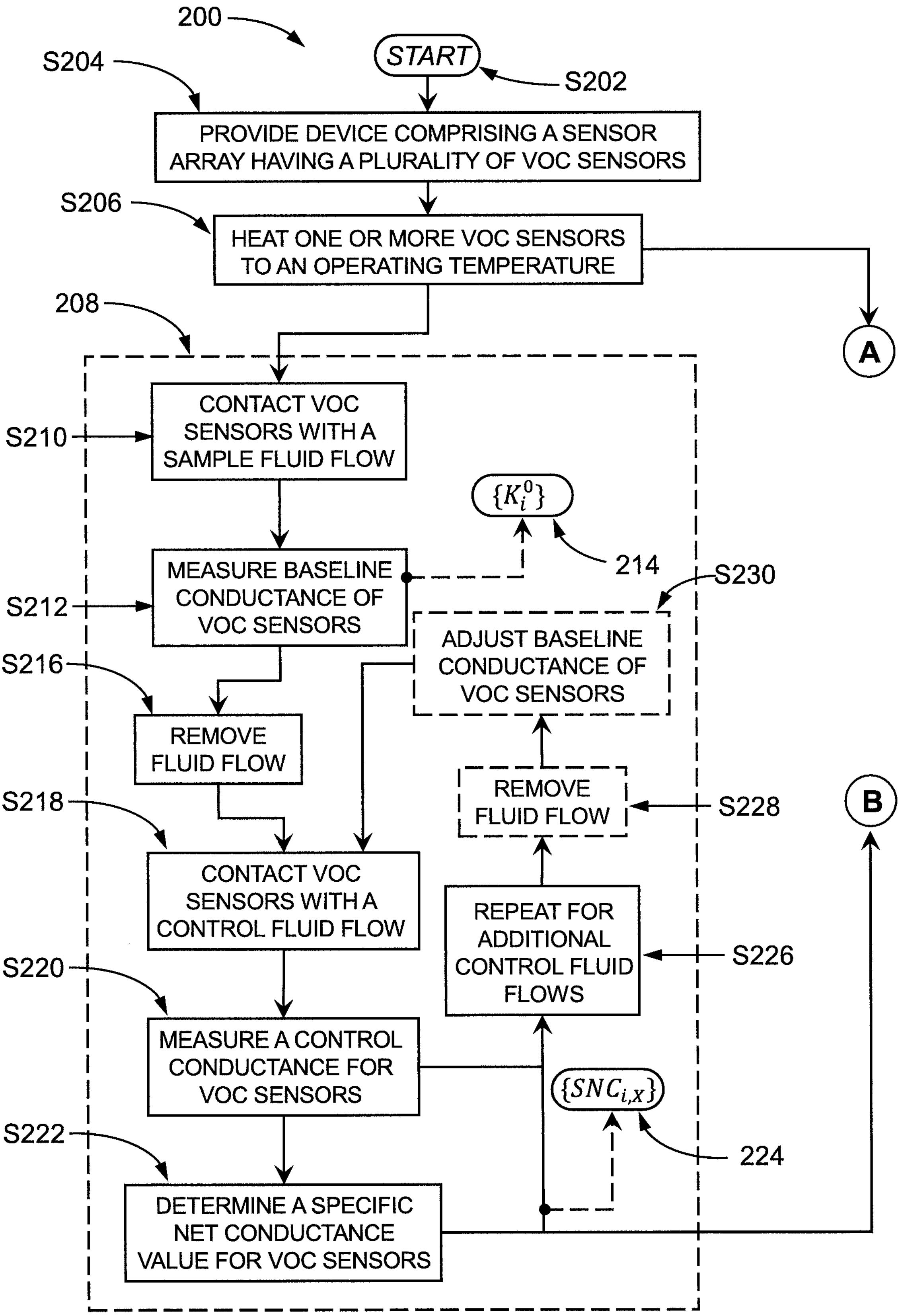
200
START
S202
S204
PROVIDE DEVICE COMPRISING A SENSOR ARRAY HAVING A PLURALITY OF VOC SENSORS
S206
HEAT ONE OR MORE VOC SENSORS TO AN OPERATING TEMPERATURE
A
208
S210
CONTACT VOC SENSORS WITH A SAMPLE FLUID FLOW
$\{K_i^0\}$
S212
MEASURE BASELINE CONDUCTANCE OF VOC SENSORS
214
S230
ADJUST BASELINE CONDUCTANCE OF VOC SENSORS
S216
REMOVE FLUID FLOW
REMOVE FLUID FLOW
S228
B
S218
CONTACT VOC SENSORS WITH A CONTROL FLUID FLOW
REPEAT FOR ADDITIONAL CONTROL FLUID FLOWS
S226
S220
MEASURE A CONTROL CONDUCTANCE FOR VOC SENSORS
$\{SNC_{i,x}\}$
S222
224
DETERMINE A SPECIFIC NET CONDUCTANCE VALUE FOR VOC SENSORS

FIG. 2A

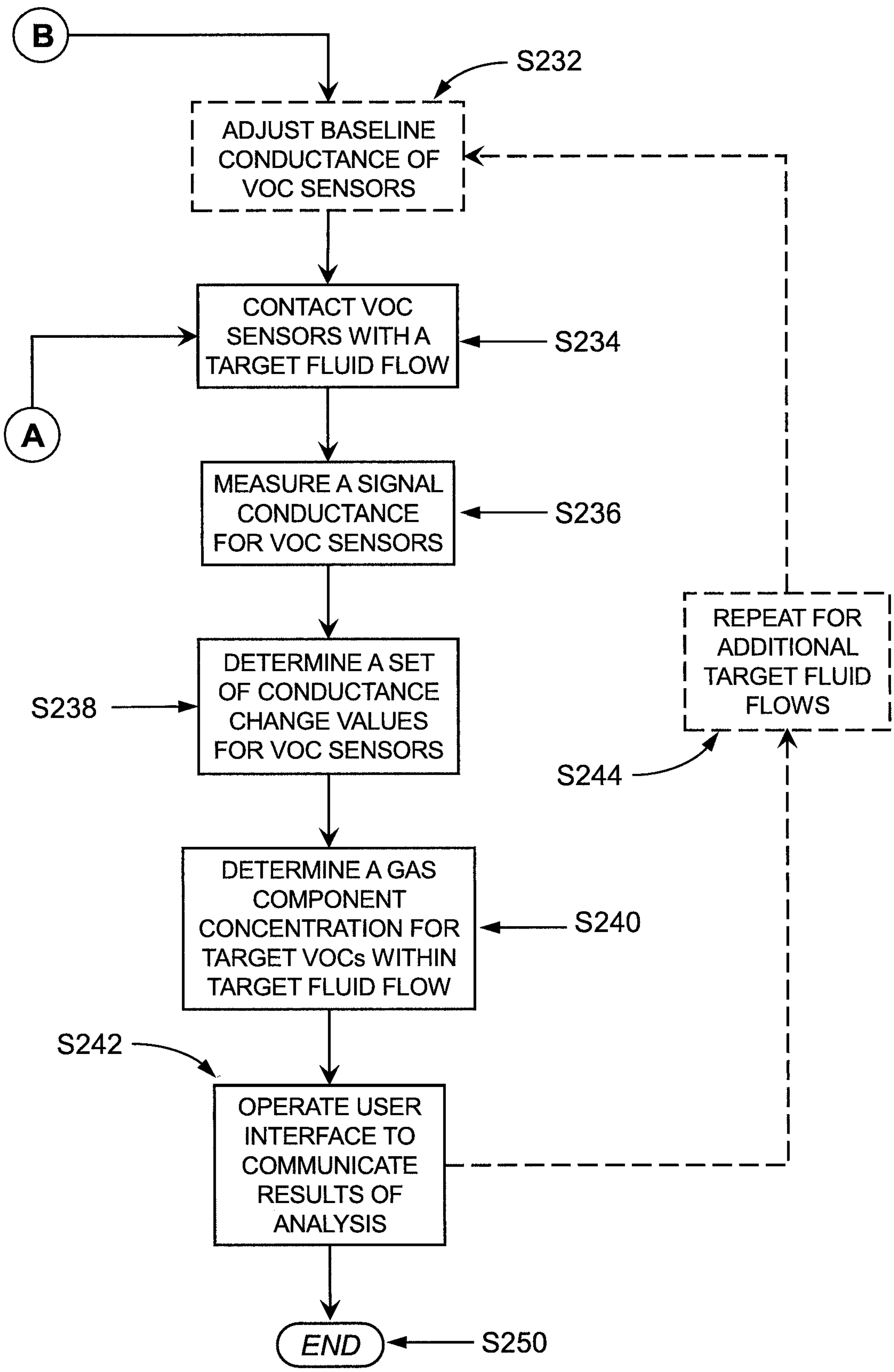
B
S232
ADJUST BASELINE CONDUCTANCE OF VOC SENSORS
CONTACT VOC SENSORS WITH A TARGET FLUID FLOW
S234
A
MEASURE A SIGNAL CONDUCTANCE FOR VOC SENSORS
S236
S238
DETERMINE A SET OF CONDUCTANCE CHANGE VALUES FOR VOC SENSORS
REPEAT FOR ADDITIONAL TARGET FLUID FLOWS
S244
DETERMINE A GAS COMPONENT CONCENTRATION FOR TARGET VOCs WITHIN TARGET FLUID FLOW
S240
S242
OPERATE USER INTERFACE TO COMMUNICATE RESULTS OF ANALYSIS
END
S250

FIG. 2B

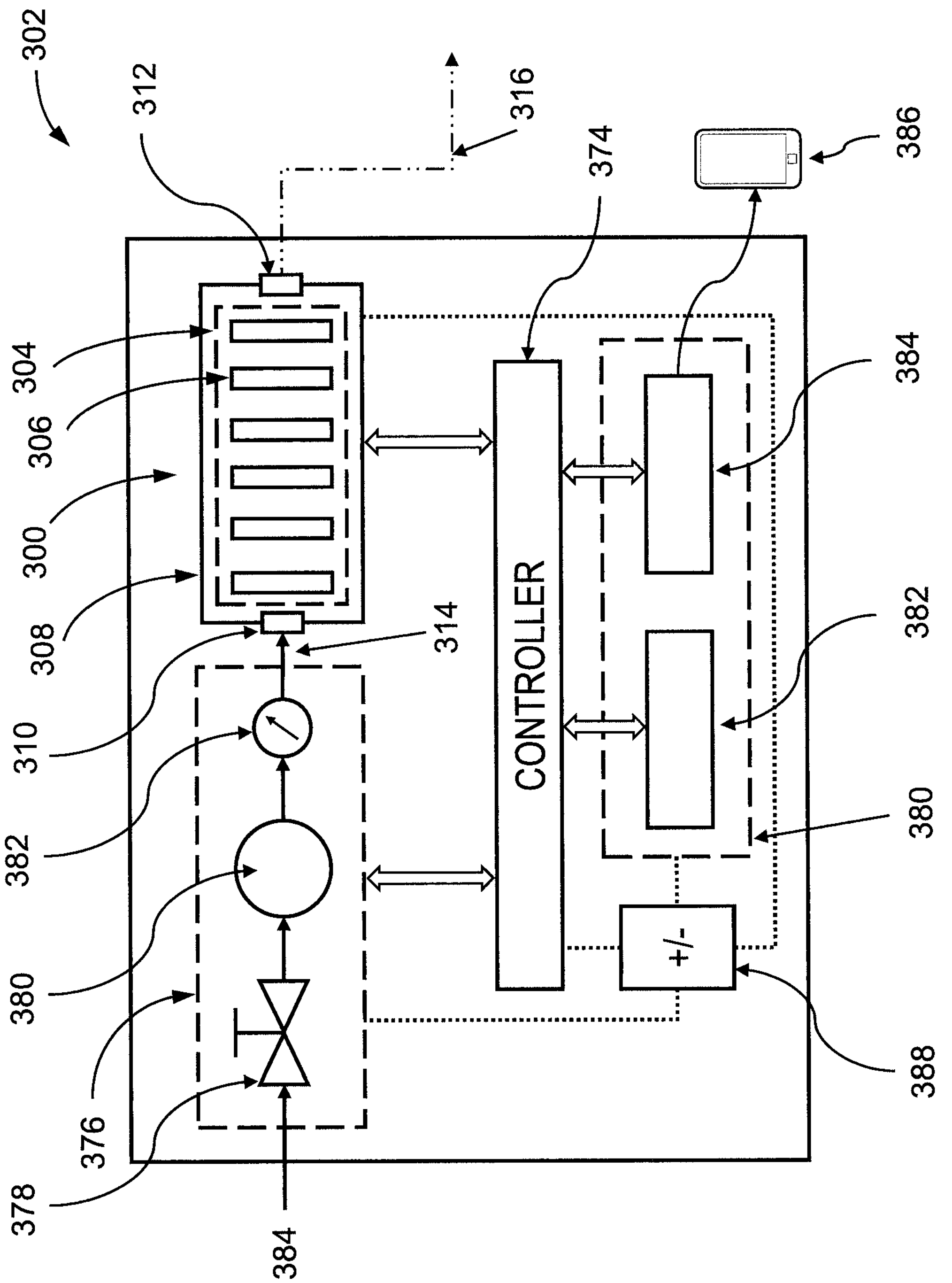
302
312
316
374
386
304
306
300
308
314
310
382
380
376
378
384
CONTROLLER
+/-
384
382
380
388

FIG. 3

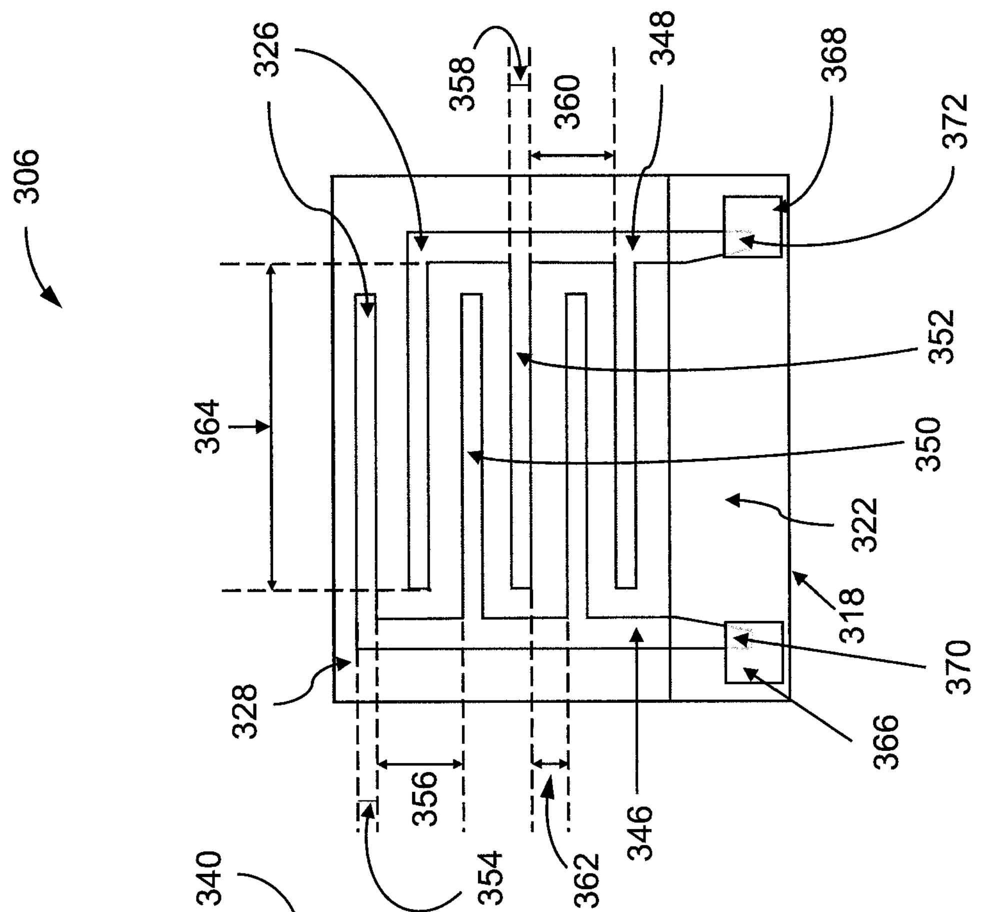
306
326
358
360
348
368
372
364
352
350
322
318
328
370
366
356
346
354
362

FIG. 4B

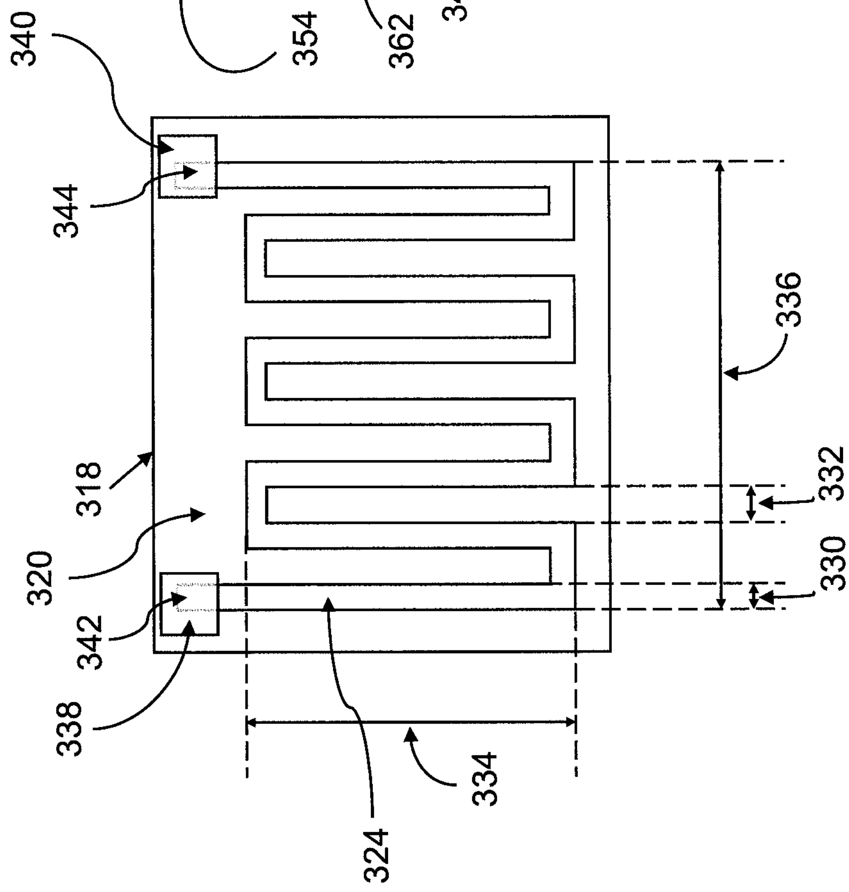
340
344
336
318
332
320
330
342
338
334
324

FIG. 4A

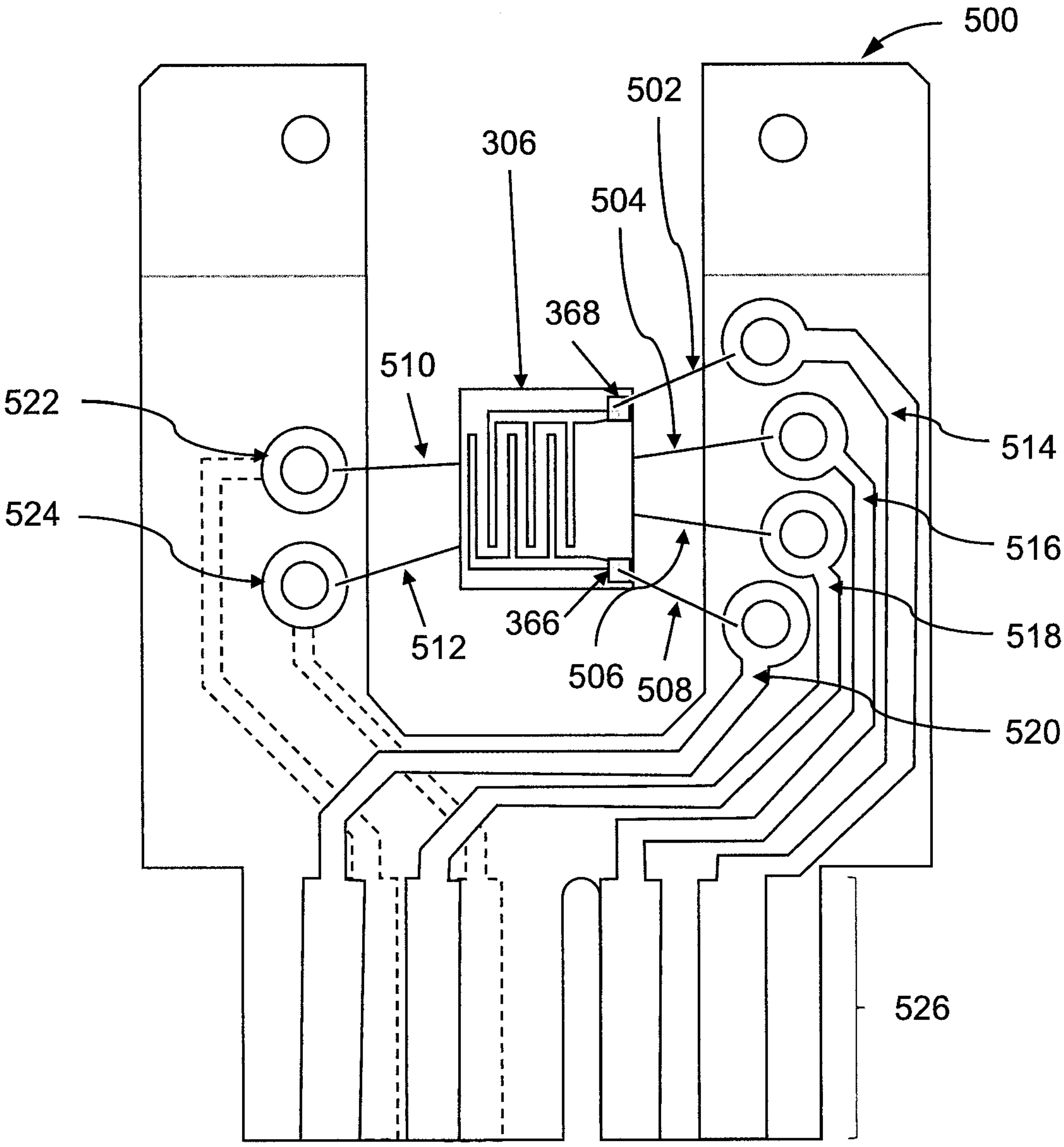
500
502
306
504
368
510
522
514
524
516
518
512
366
506
508
520
526

FIG. 5

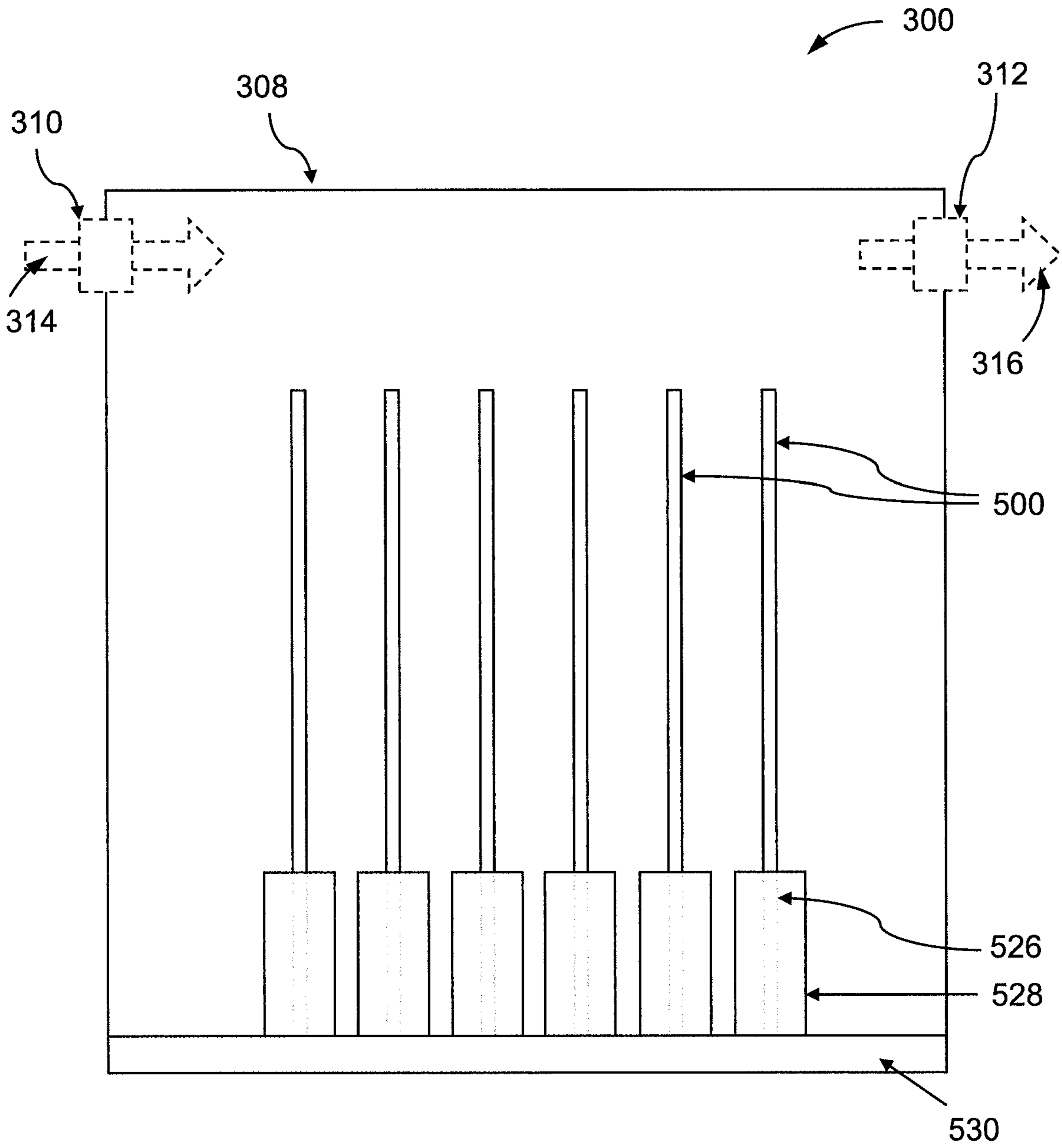


FIG. 6

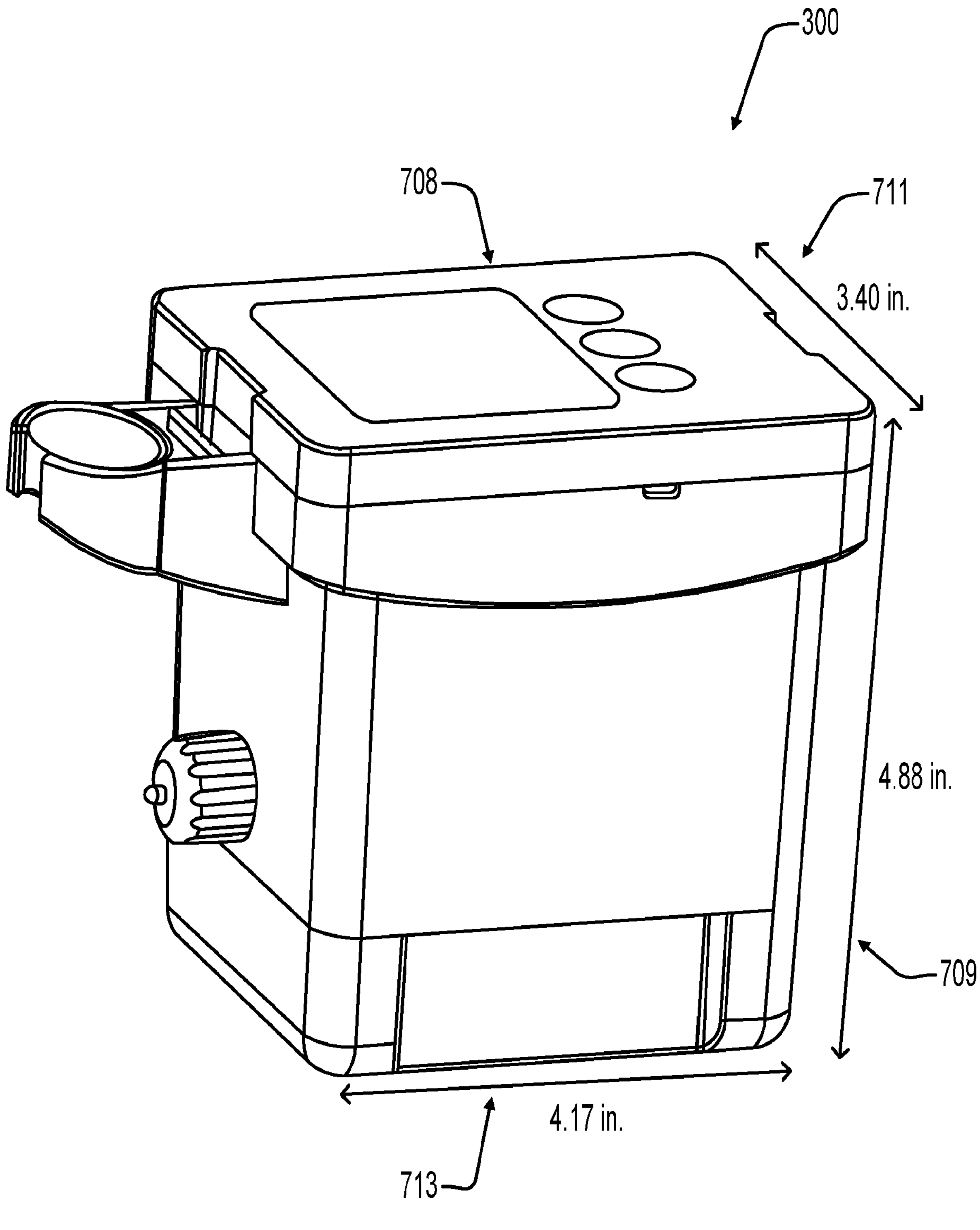


FIG. 7

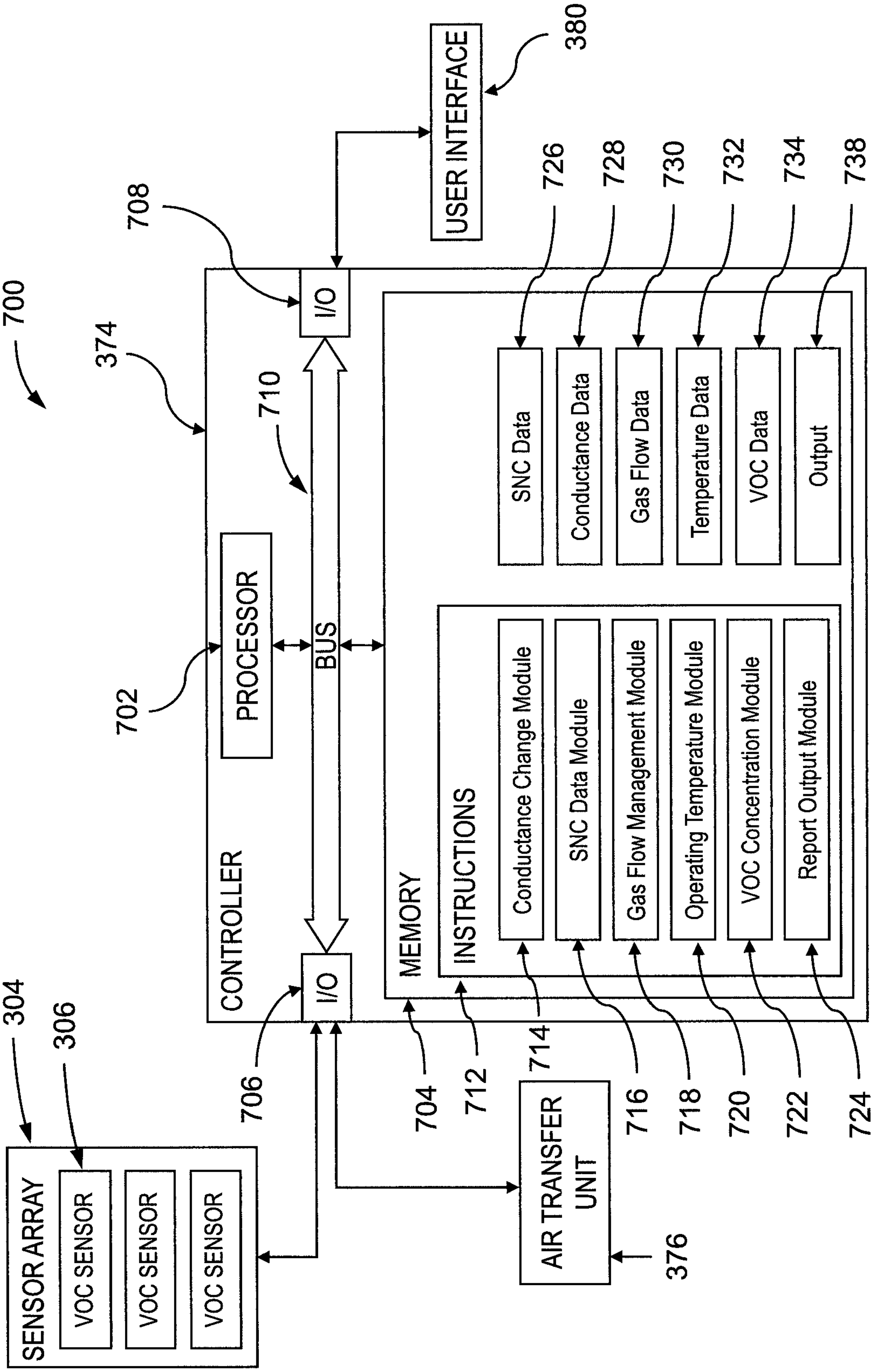
700
374
708
380
USER INTERFACE
702
PROCESSOR
710
BUS
I/O
I/O
CONTROLLER
MEMORY
INSTRUCTIONS
Conductance Change Module
SNC Data Module
Gas Flow Management Module
Operating Temperature Module
VOC Concentration Module
Report Output Module
SNC Data
Conductance Data
Gas Flow Data
Temperature Data
VOC Data
Output
726
728
730
732
734
738
304
306
SENSOR ARRAY
VOC SENSOR
VOC SENSOR
VOC SENSOR
706
704
712
714
716
718
720
722
724
AIR TRANSFER UNIT
376

FIG. 8

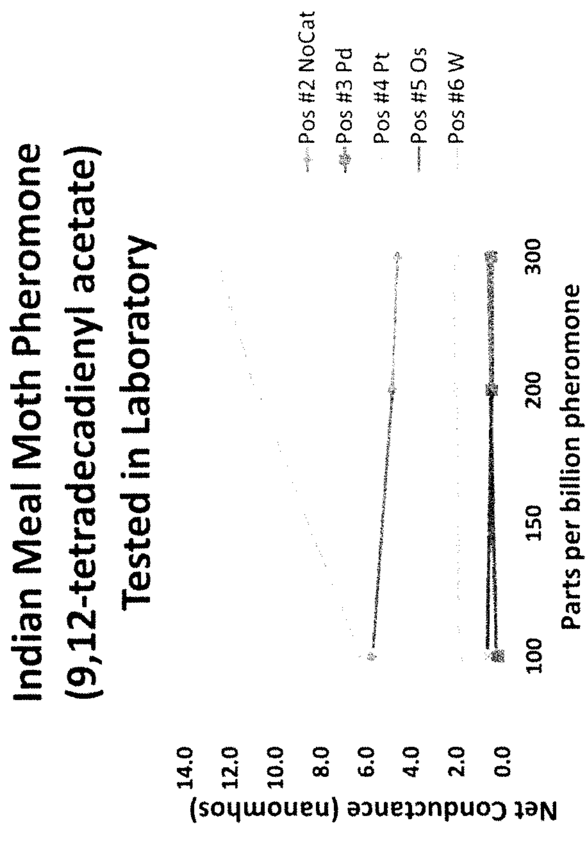
Indian Meal Moth Pheromone
(9,12-tetradecadienyl acetate)
Tested in Laboratory
Net Conductance (nanomhos)
14.0
12.0
10.0
8.0
6.0
4.0
2.0
0.0
100
150
200
300
Parts per billion pheromone
Pos #2 NoCat
Pos #3 Pd
Pos #4 Pt
Pos #5 Os
Pos #6 W

FIG. 9A

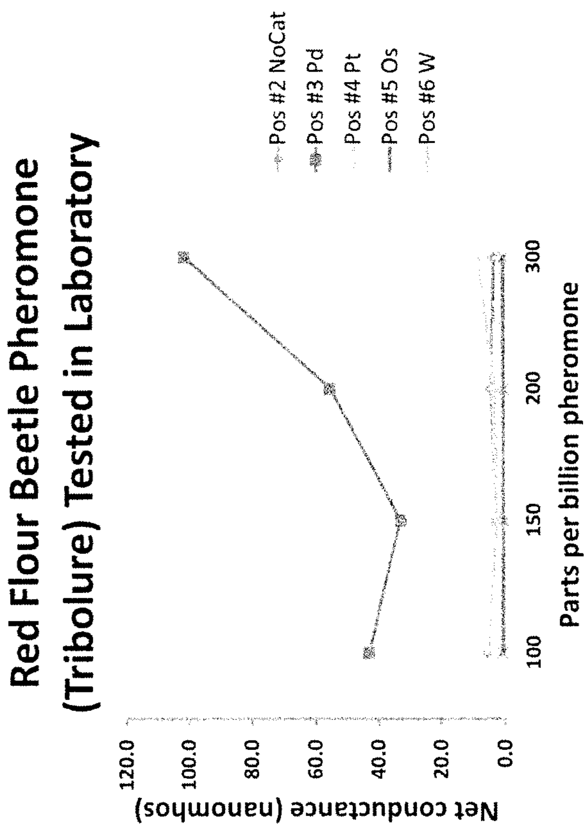
Red Flour Beetle Pheromone
(Tribolure) Tested in Laboratory
Net conductance (nanomhos)
120.0
100.0
80.0
60.0
40.0
20.0
0.0
100
150
200
300
Parts per billion pheromone
Pos #2 NoCat
Pos #3 Pd
Pos #4 Pt
Pos #5 Os
Pos #6 W

FIG. 9B

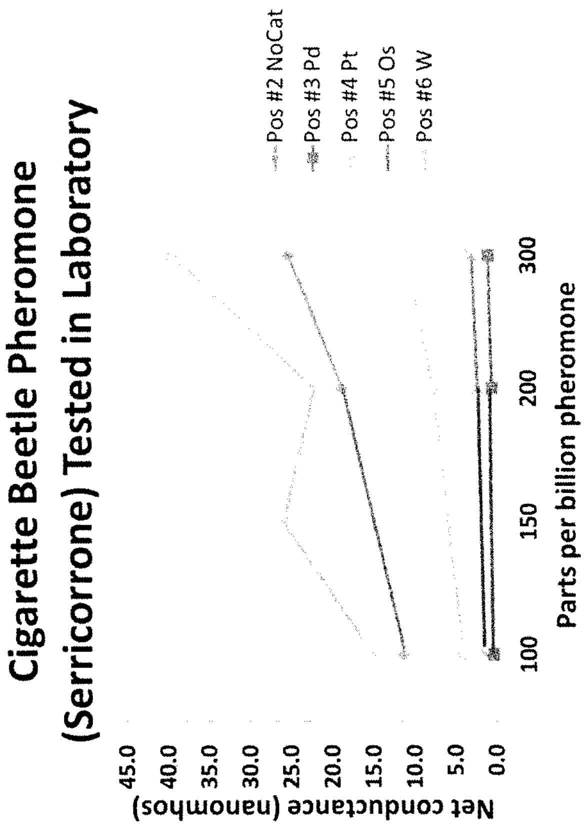
Cigarette Beetle Pheromone
(Serricorrone) Tested in Laboratory
Net conductance (nanomhos)
45.0
40.0
35.0
30.0
25.0
20.0
15.0
10.0
5.0
0.0
100
150
200
300
Parts per billion pheromone
Pos #2 NoCat
Pos #3 Pd
Pos #4 Pt
Pos #5 Os
Pos #6 W

FIG. 9C

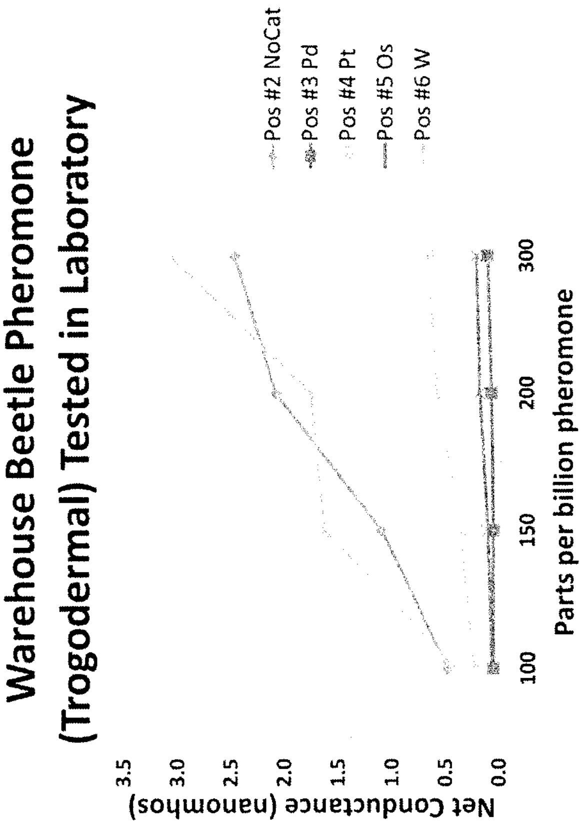
Warehouse Beetle Pheromone
(Trogodermal) Tested in Laboratory
Net Conductance (nanomhos)
3.5
3.0
2.5
2.0
1.5
1.0
0.5
0.0
100
150
200
300
Parts per billion pheromone
Pos #2 NoCat
Pos #3 Pd
Pos #4 Pt
Pos #5 Os
Pos #6 W

FIG. 9D

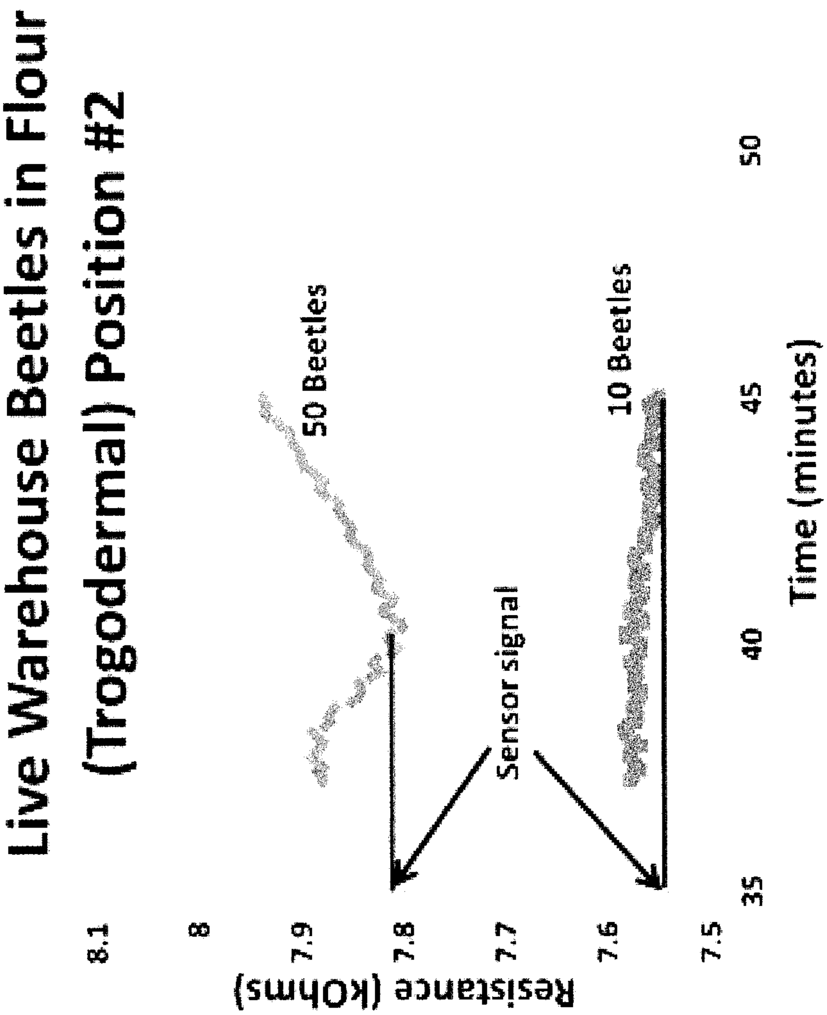
Live Warehouse Beetles in Flour (Trogodermal) Position #2
Resistance (kOhms)
8.1
8
7.9
7.8
7.7
7.6
7.5
35
40
45
50
Time (minutes)
50 Beetles
10 Beetles
Sensor signal

FIG. 10A

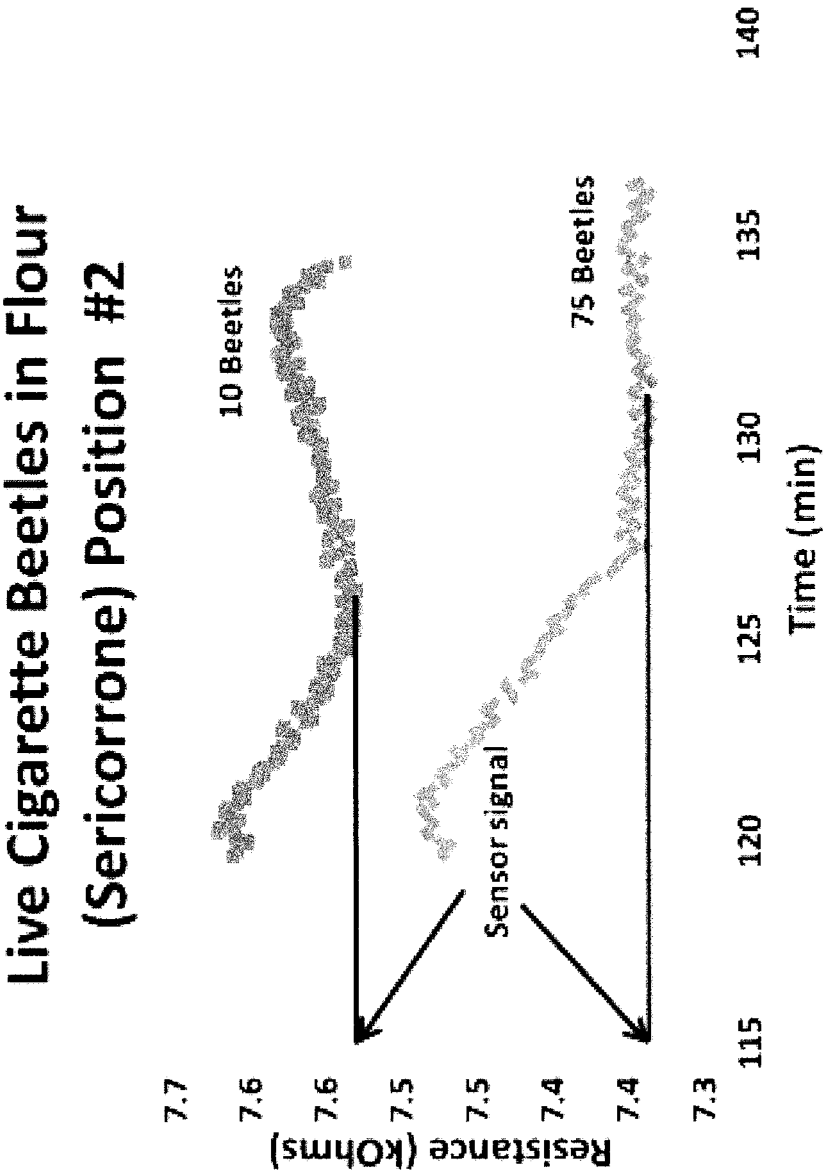
Live Cigarette Beetles in Flour
(Sericorrone) Position #2
10 Beetles
75 Beetles
Sensor signal
Resistance (kOhms)
7.7
7.6
7.6
7.5
7.5
7.4
7.4
7.3
115
120
125
130
135
140
Time (min)

FIG. 10B

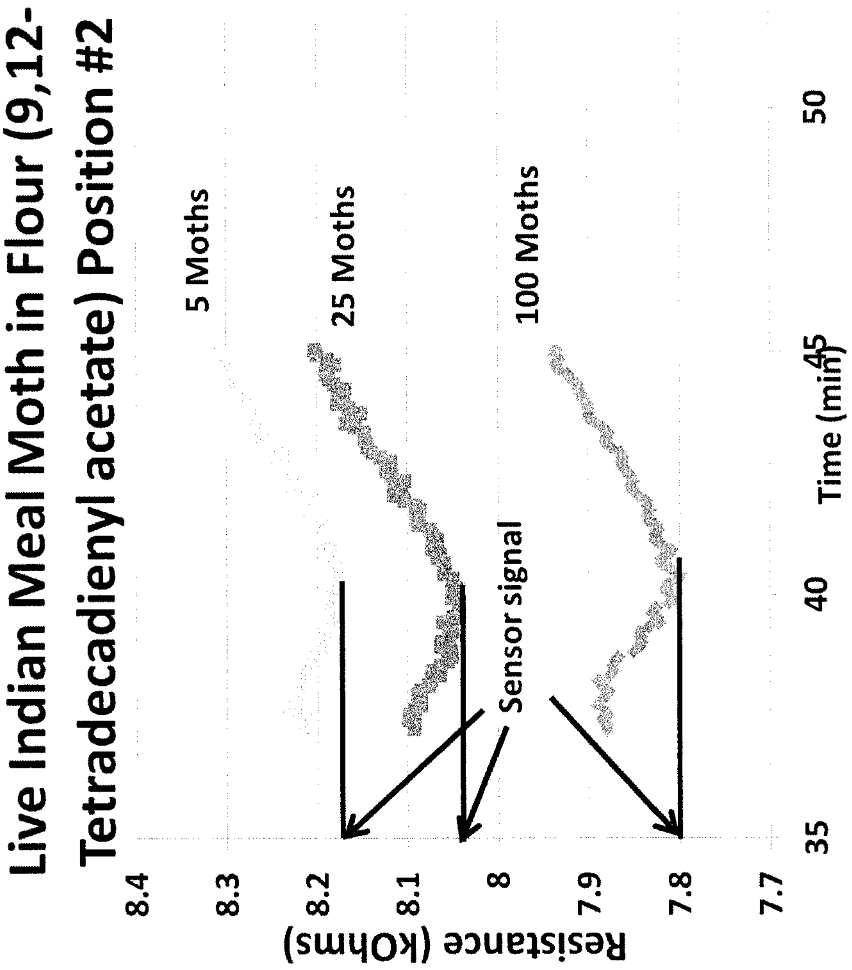
Live Indian Meal Moth in Flour (9,12-Tetradecadienyl acetate) Position #2
Resistance (kOhms)
8.4
8.3
8.2
8.1
8
7.9
7.8
7.7
35
40
45
50
Time (min)
5 Moths
25 Moths
100 Moths
Sensor signal

FIG. 10C

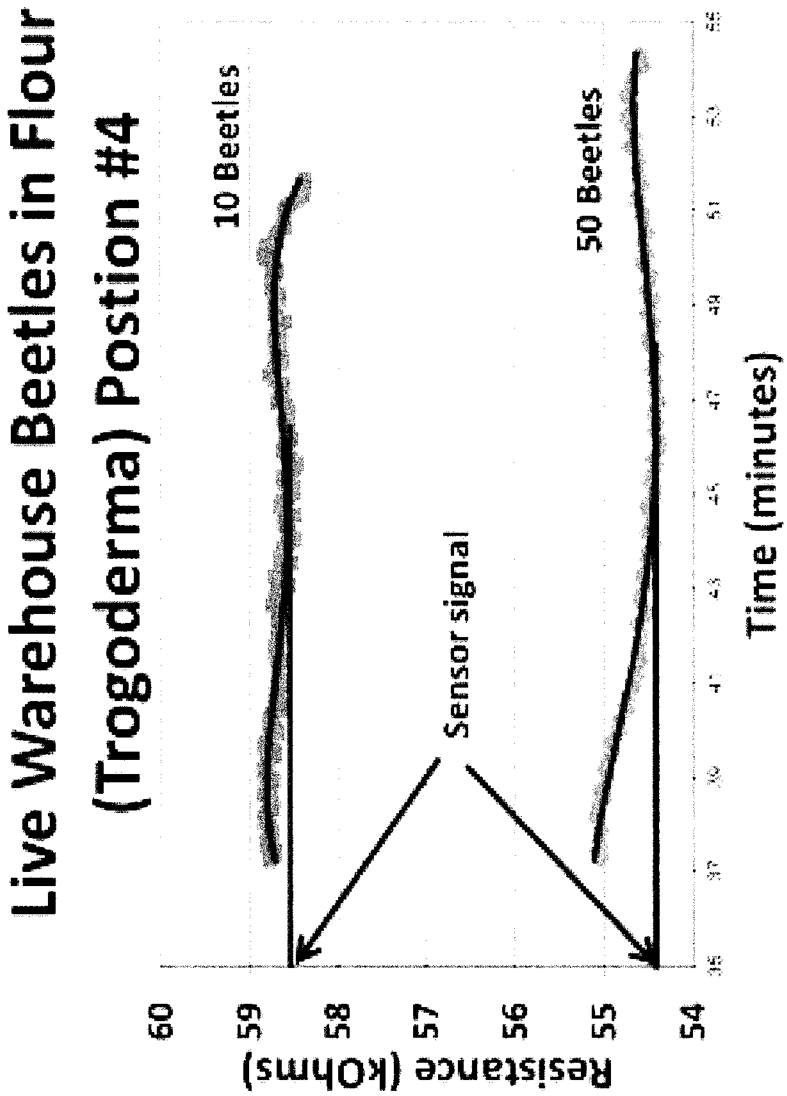
Live Warehouse Beetles in Flour
(Trogoderma) Postion #4
Resistance (kOhms)
60
59
58
57
56
55
54
10 Beetles
50 Beetles
Sensor signal
Time (minutes)

FIG. 11A

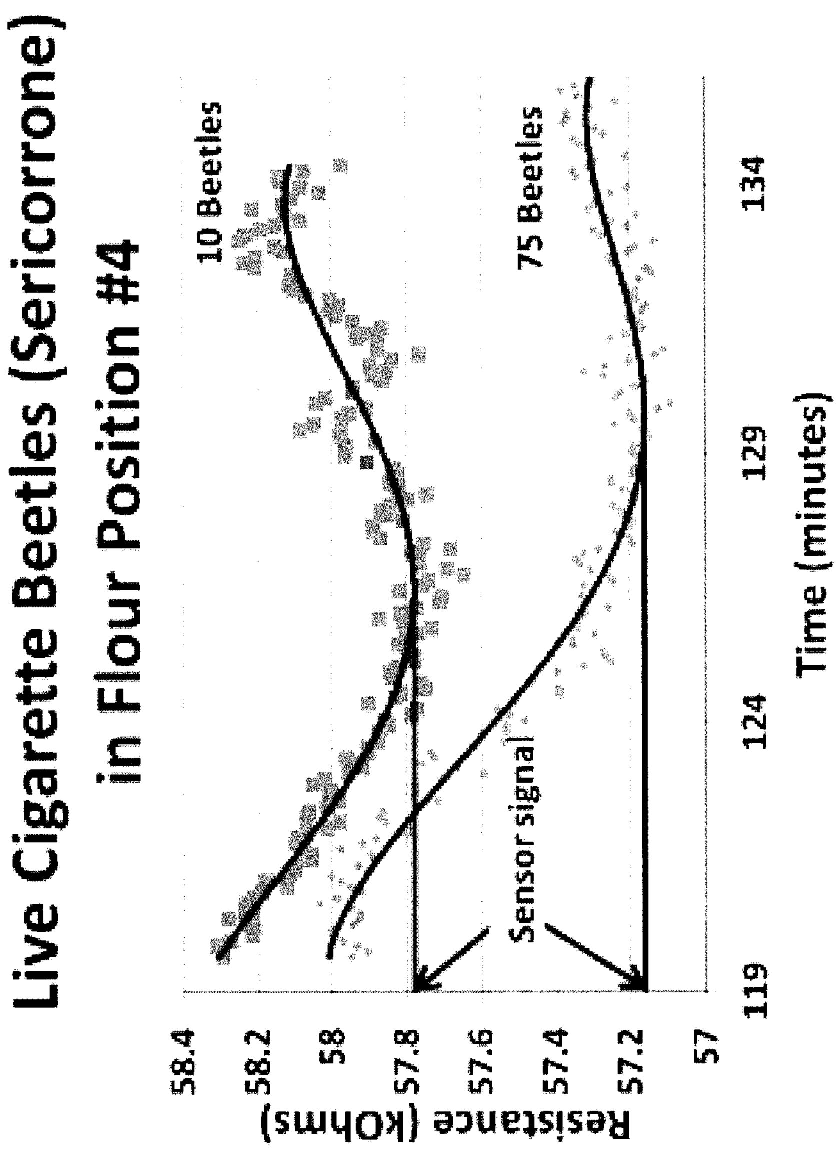
Live Cigarette Beetles (Sericorrone)
in Flour Position #4
10 Beetles
75 Beetles
Sensor signal
Resistance (kOhms)
58.4
58.2
58
57.8
57.6
57.4
57.2
57
119
124
129
134
Time (minutes)

FIG. 11B

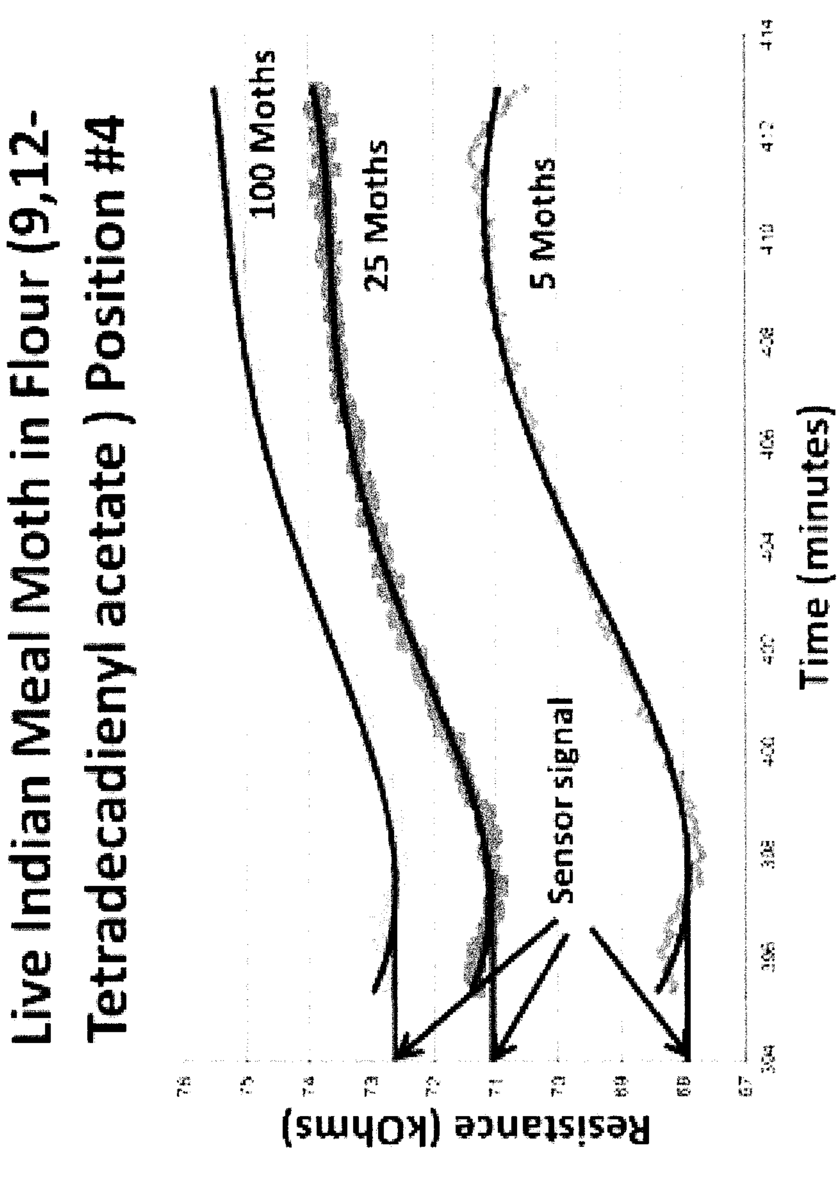
Live Indian Meal Moth in Flour (9,12-Tetradecadienyl acetate ) Position #4
100 Moths
25 Moths
5 Moths
Sensor signal
Resistance (kOhms)
Time (minutes)

FIG. 11C

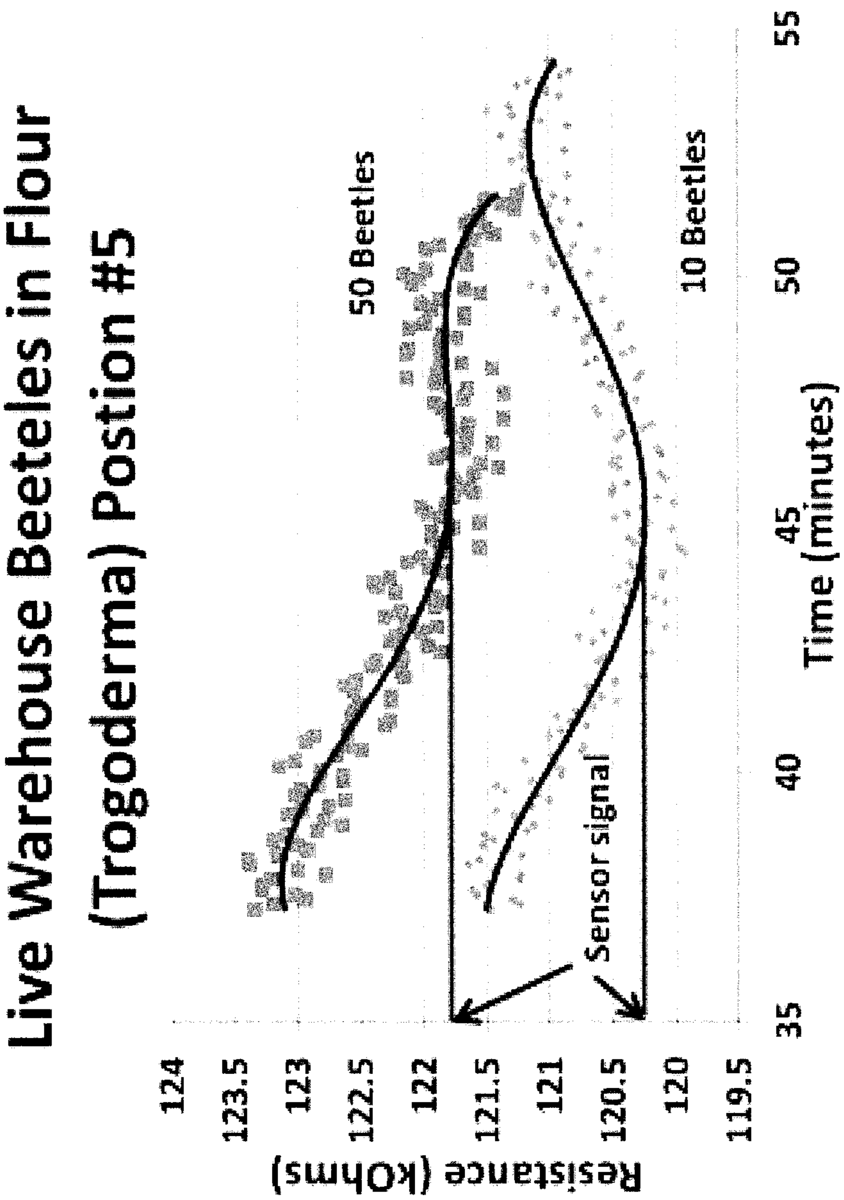
Live Warehouse Beeteles in Flour
(Trogoderma) Postion #5
Resistance (kOhms)
124
123.5
123
122.5
122
121.5
121
120.5
120
119.5
35
40
45
50
55
Time (minutes)
50 Beetles
10 Beetles
Sensor signal

FIG. 12A

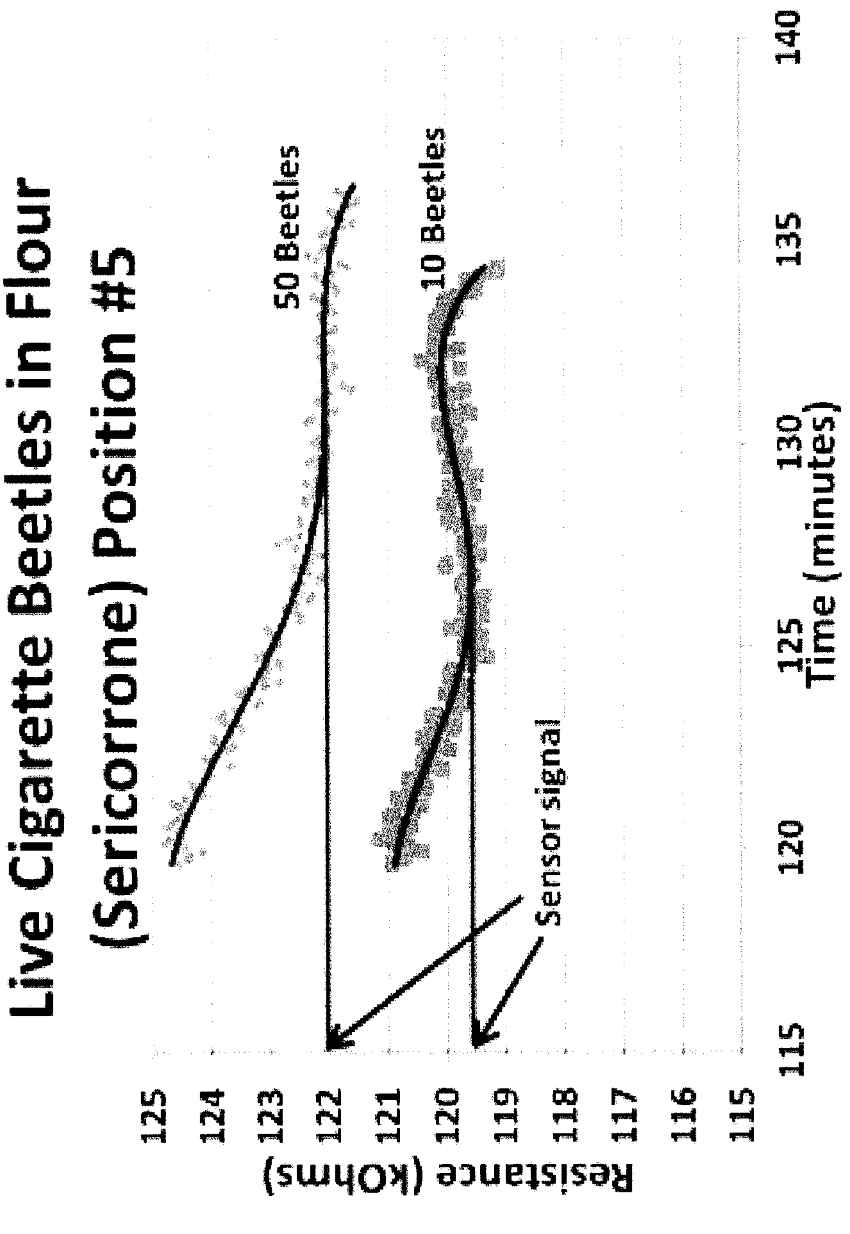

Live Cigarette Beetles in Flour (Sericorrone) Position #5
50 Beetles
10 Beetles
Sensor signal
Resistance (kOhms)
125
124
123
122
121
120
119
118
117
116
115
Time (minutes)
115
120
125
130
135
140


FIG. 12B

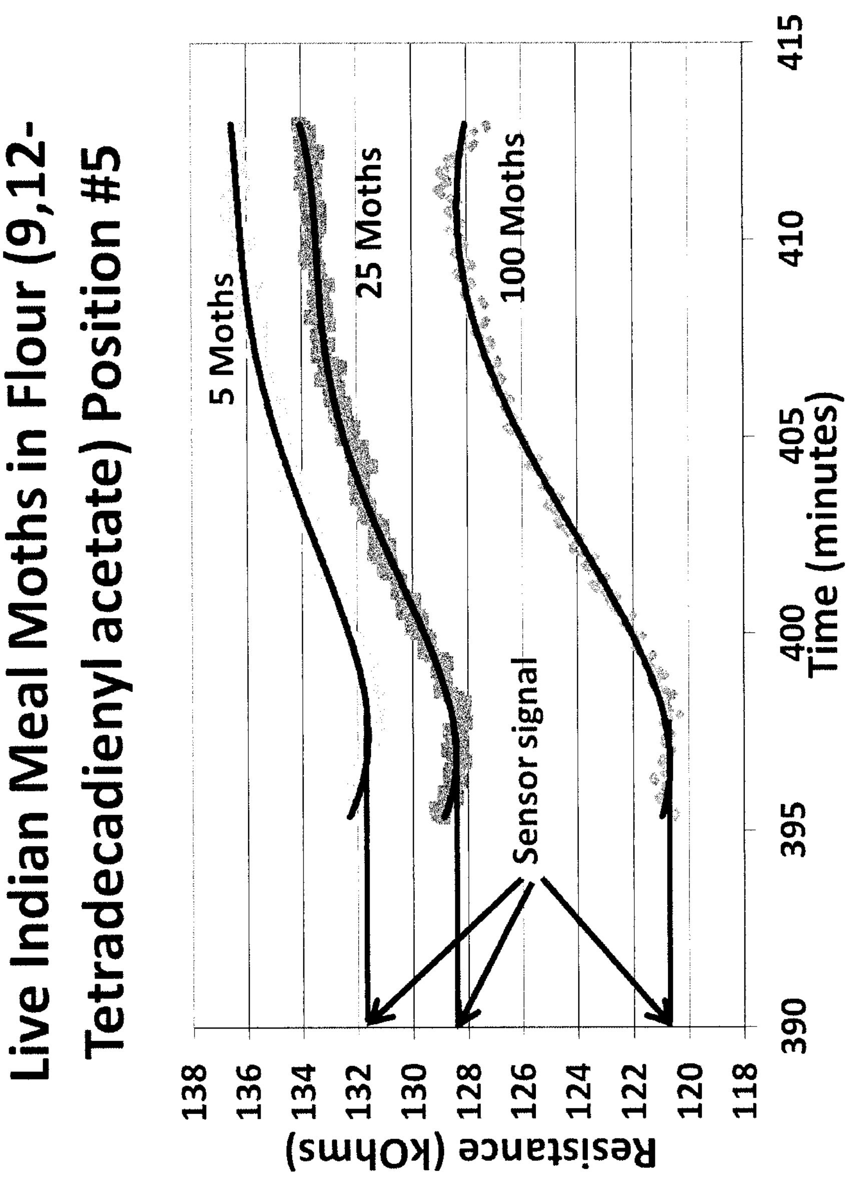
Live Indian Meal Moths in Flour (9,12-Tetradecadienyl acetate) Position #5
Resistance (kOhms)
138
136
134
132
130
128
126
124
122
120
118
Time (minutes)
390
395
400
405
410
415
5 Moths
25 Moths
100 Moths
Sensor signal

FIG. 12C

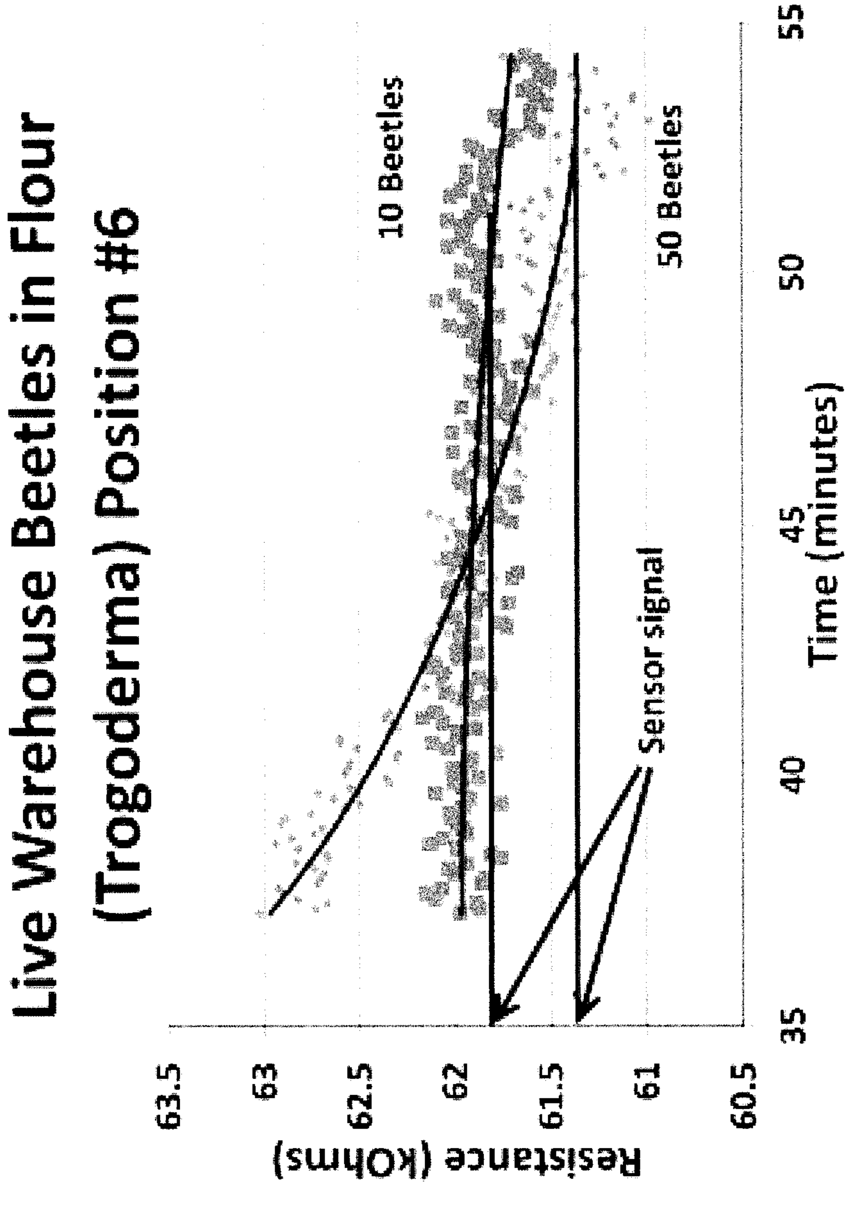
Live Warehouse Beetles in Flour
(Trogoderma) Position #6
10 Beetles
50 Beetles
Sensor signal
Resistance (kOhms)
Time (minutes)
63.5
63
62.5
62
61.5
61
60.5
35
40
45
50
55

FIG. 13A

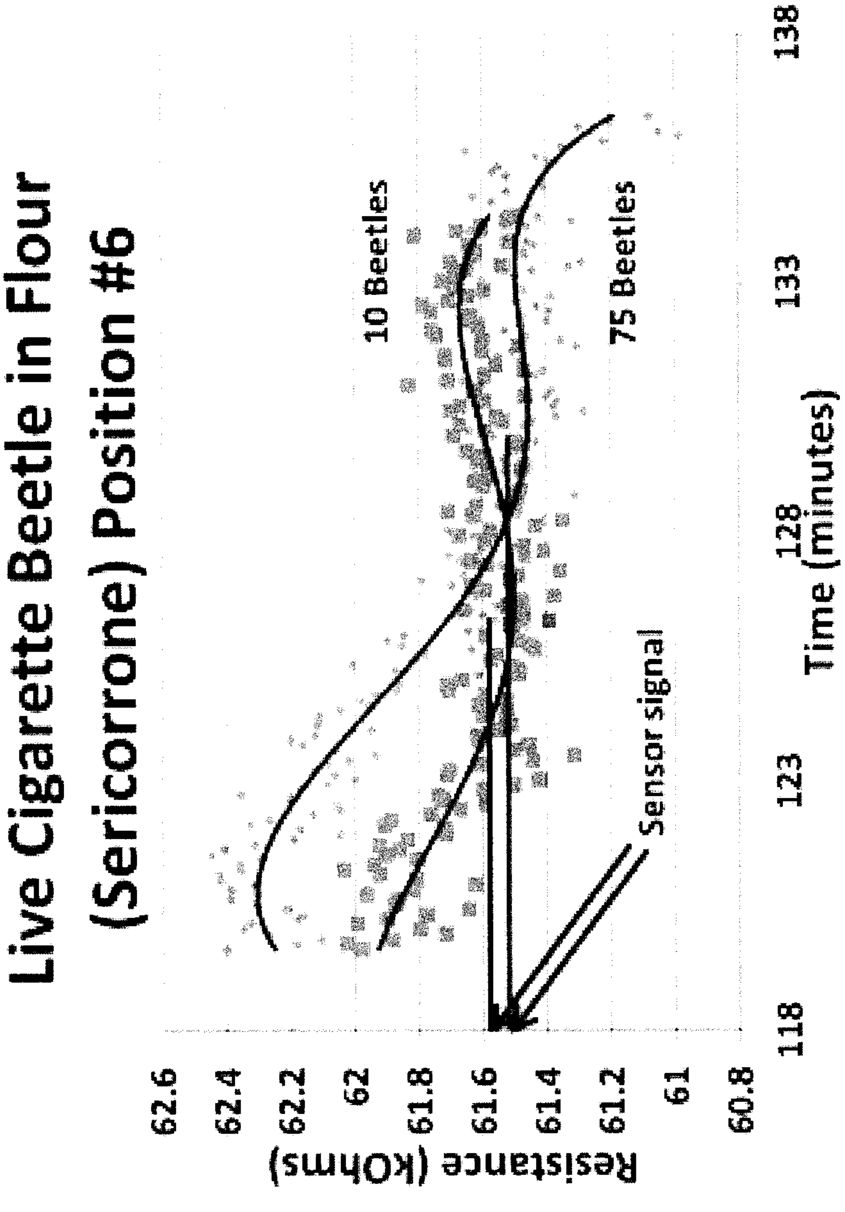
Live Cigarette Beetle in Flour
(Sericorrone) Position #6
Resistance (kOhms)
62.6
62.4
62.2
62
61.8
61.6
61.4
61.2
61
60.8
118
123
128
133
138
Time (minutes)
10 Beetles
75 Beetles
Sensor signal

FIG. 13B

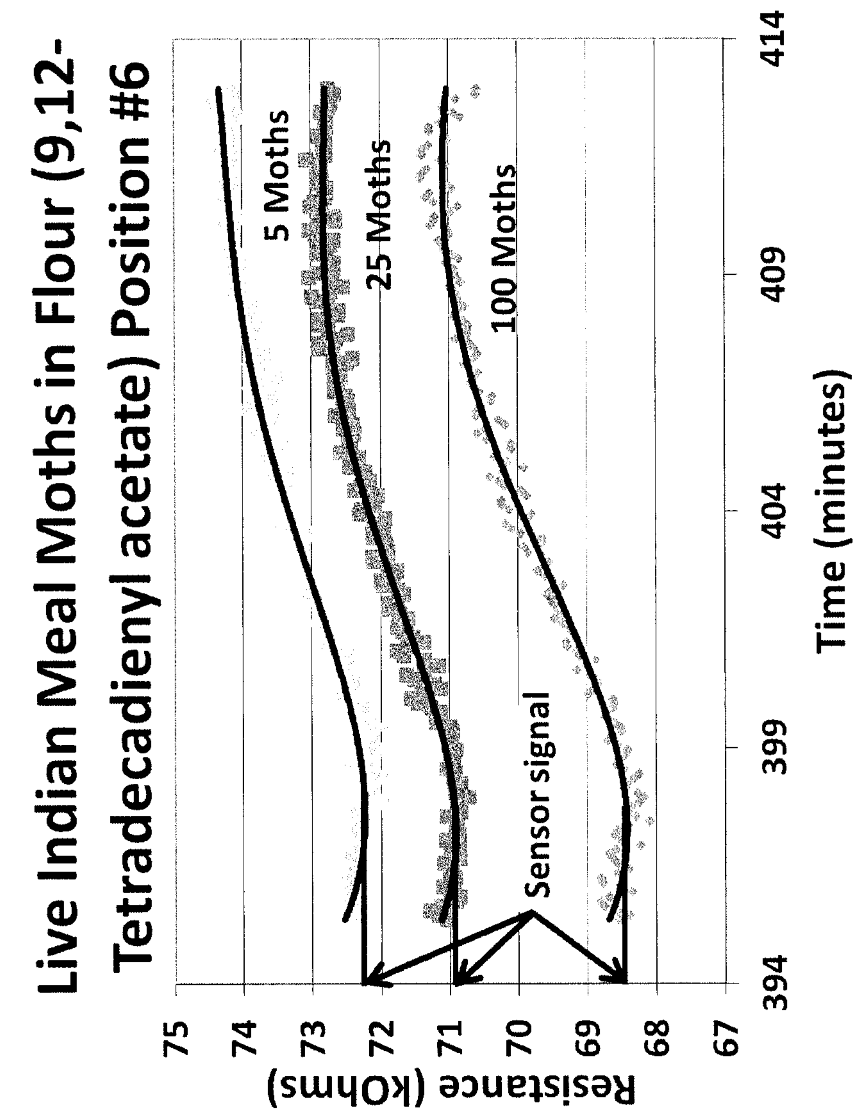
Live Indian Meal Moths in Flour (9,12-Tetradecadienyl acetate) Position #6
Resistance (kOhms)
75
74
73
72
71
70
69
68
67
394
399
404
409
414
Time (minutes)
5 Moths
25 Moths
100 Moths
Sensor signal

FIG. 13C

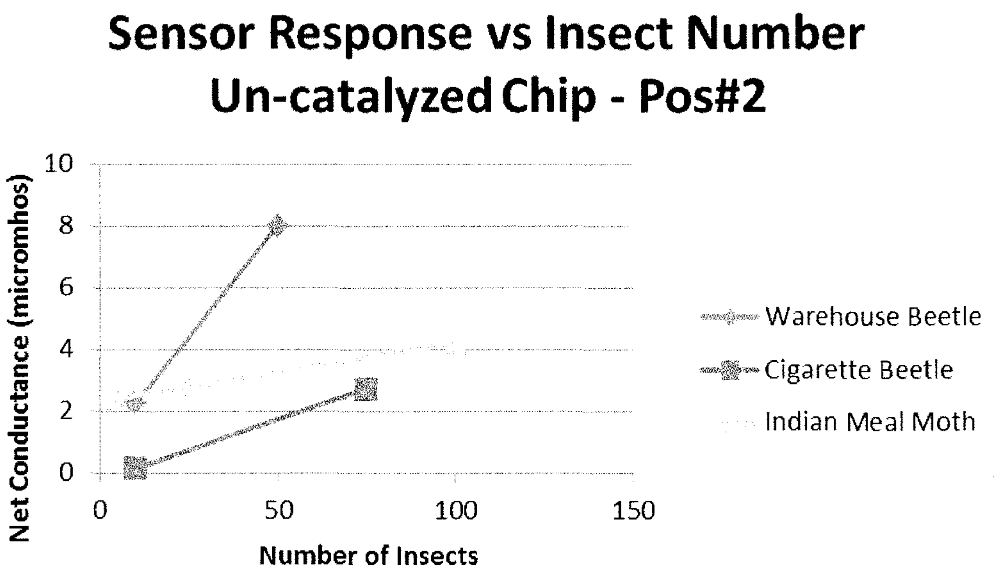


FIG. 14A

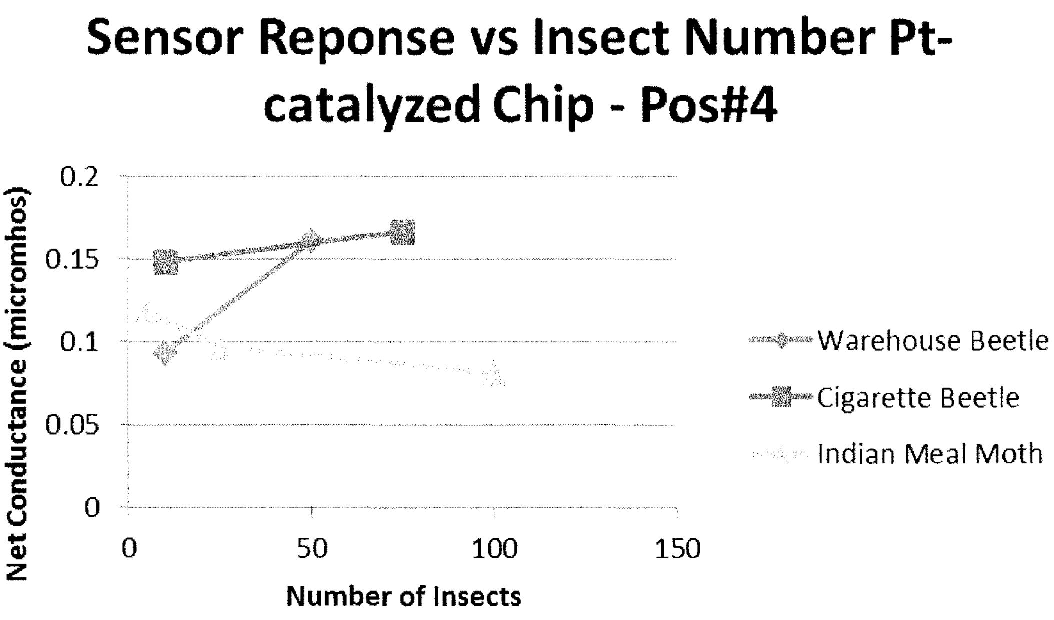


FIG. 14B

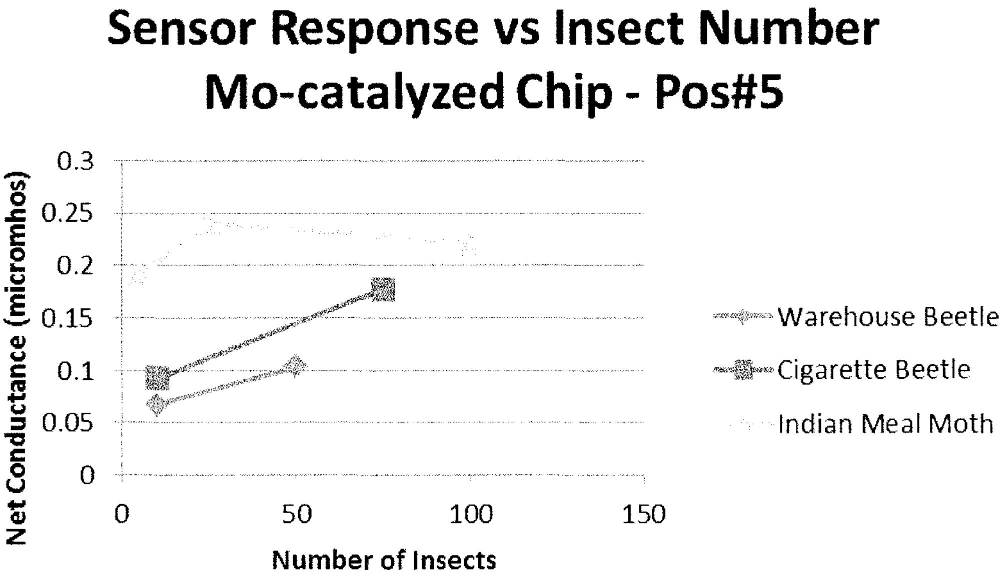


FIG. 14C

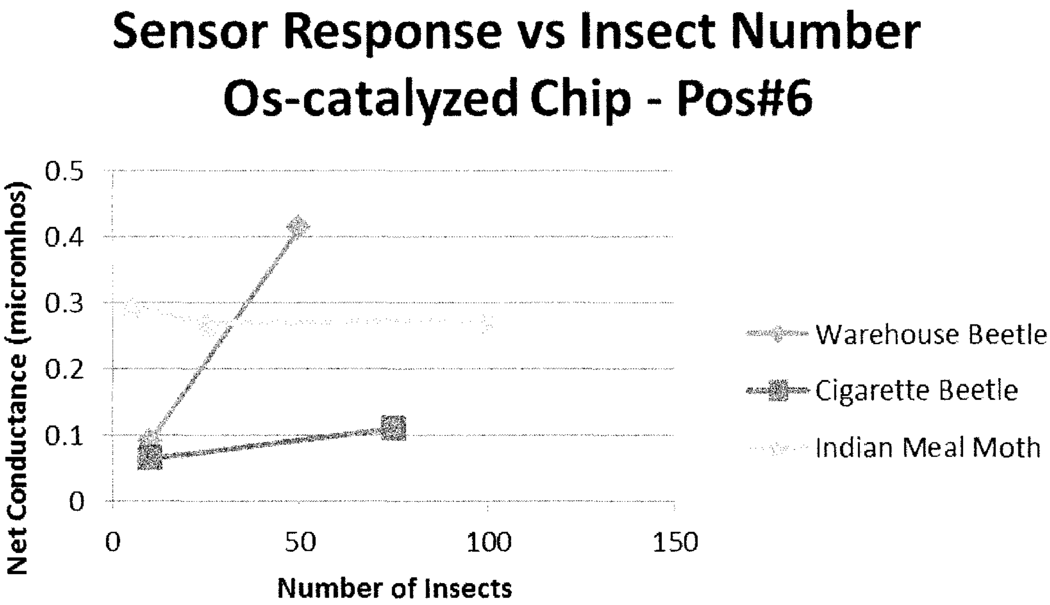


FIG. 14D

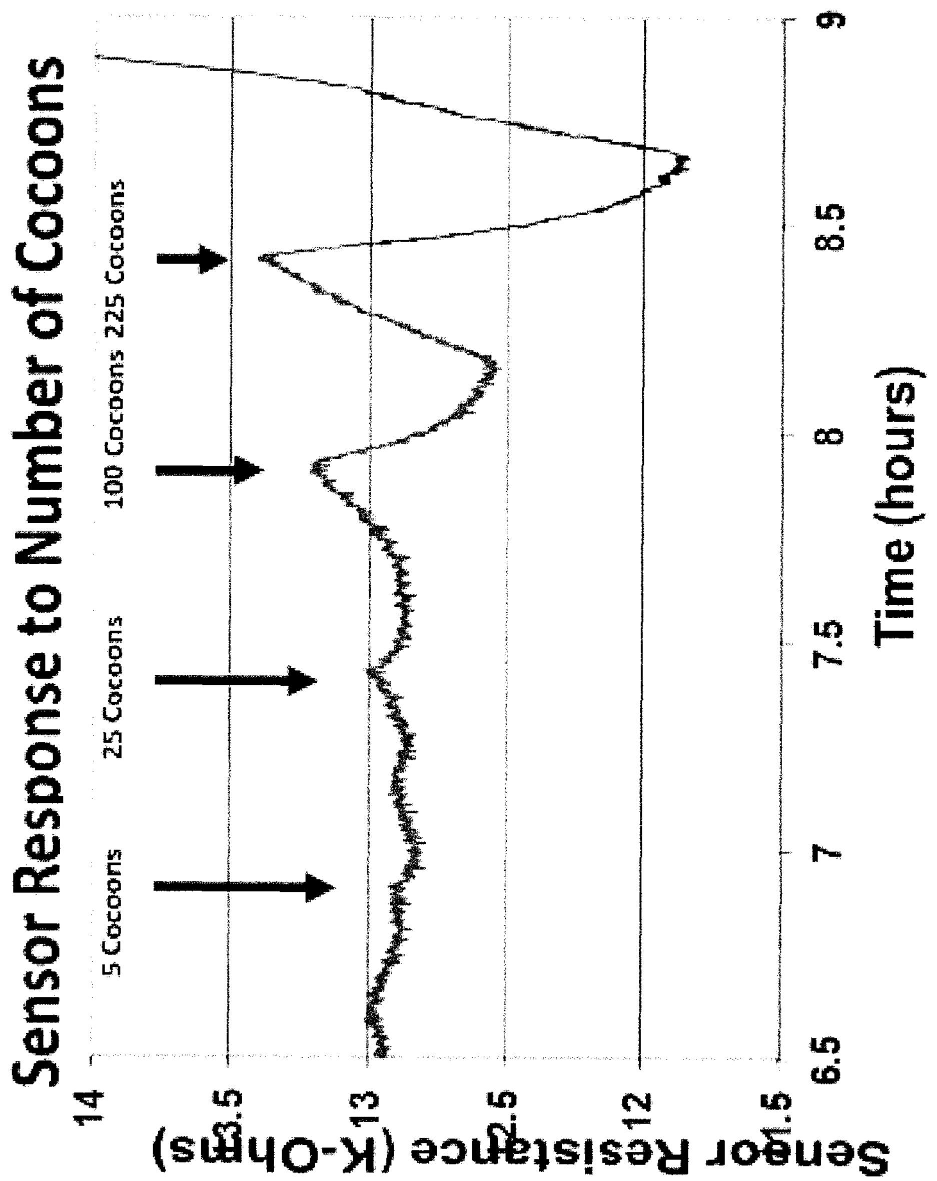
Sensor Response to Number of Cocoons
5 Cocoons
25 Cocoons
100 Cocoons
225 Cocoons
Sensor Resistance (K-Ohms)
14
13
12
Time (hours)
6.5
7
7.5
8
8.5
9

FIG. 15

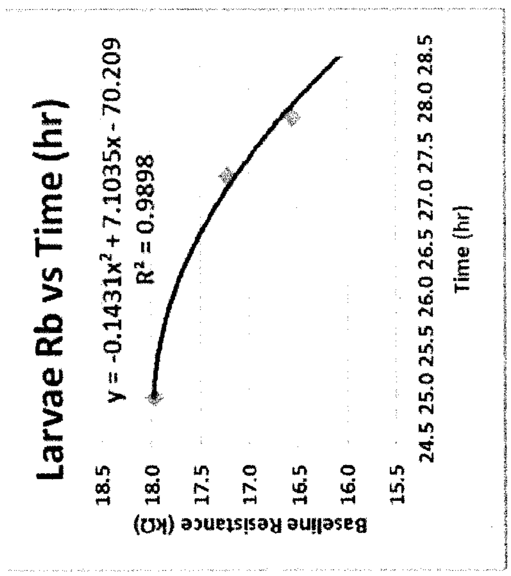


FIG. 16A

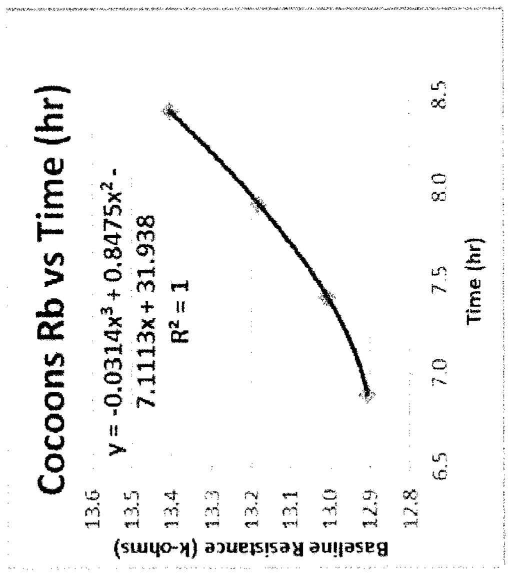


FIG. 16B

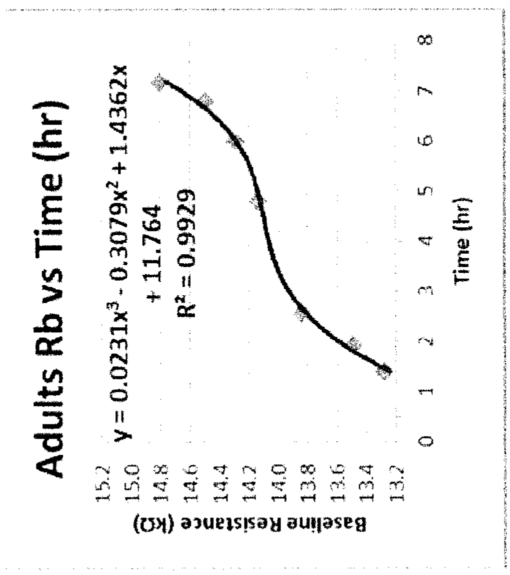


FIG. 16C

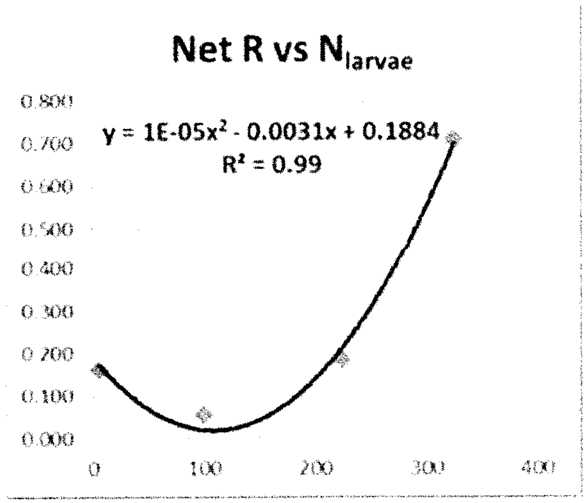


FIG. 17A

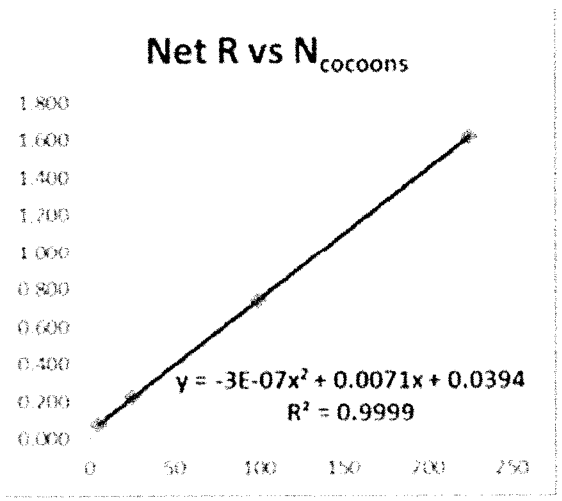


FIG. 17B

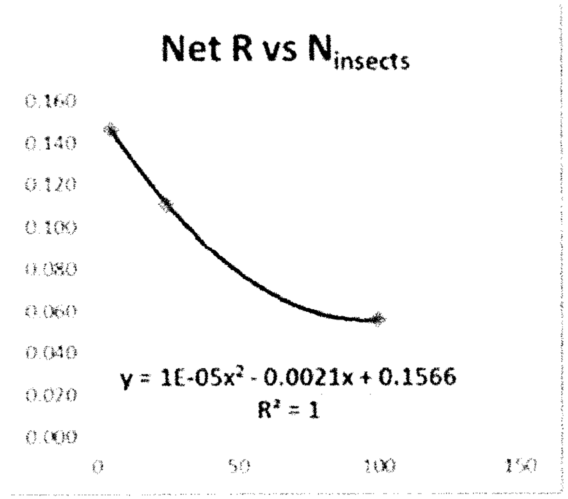


FIG. 17C

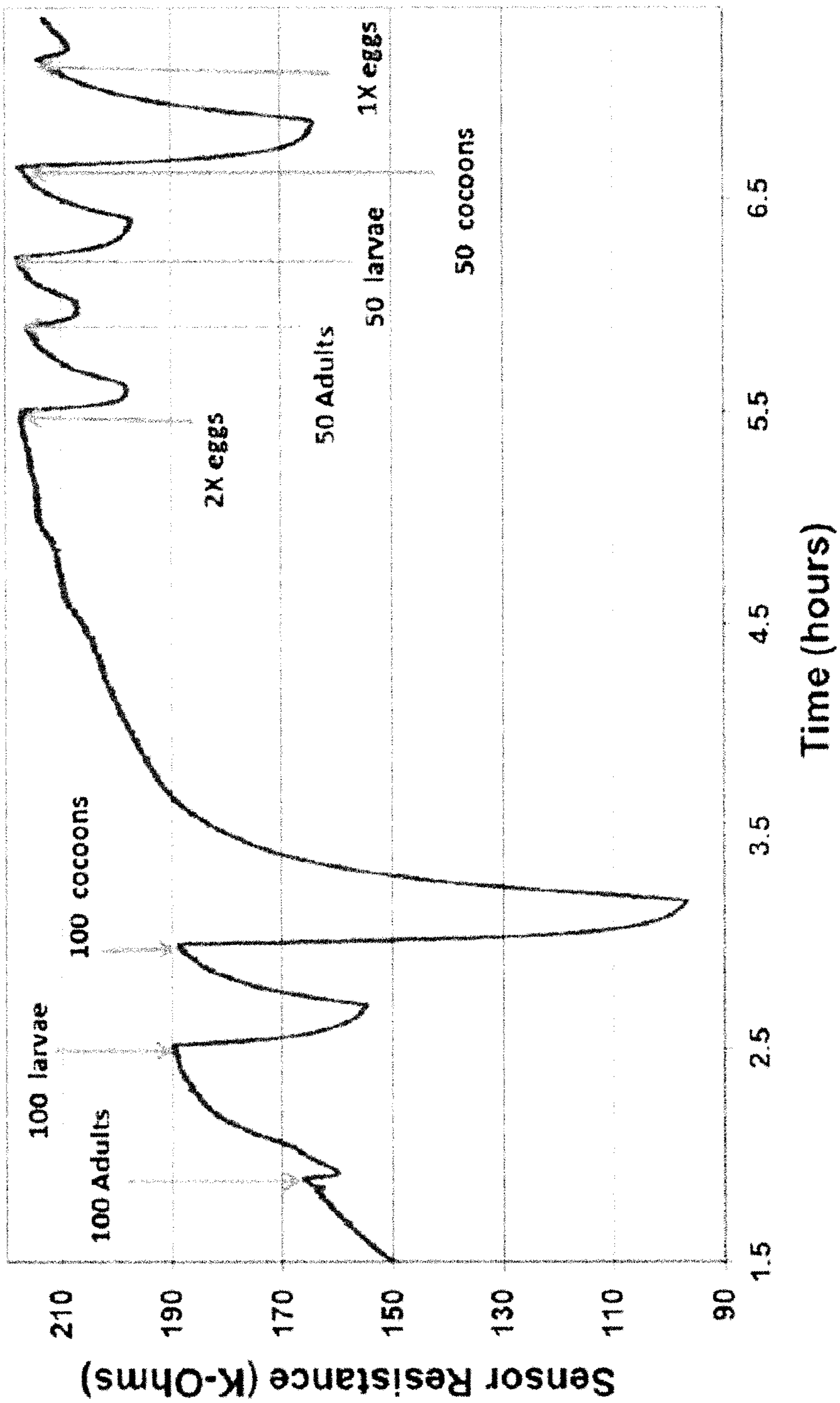
Sensor Resistance (K-Ohms)
210
190
170
150
130
110
90
1.5
2.5
3.5
4.5
5.5
6.5
Time (hours)
100 Adults
100 larvae
100 cocoons
2X eggs
50 Adults
50 larvae
50 cocoons
1X eggs

FIG. 18

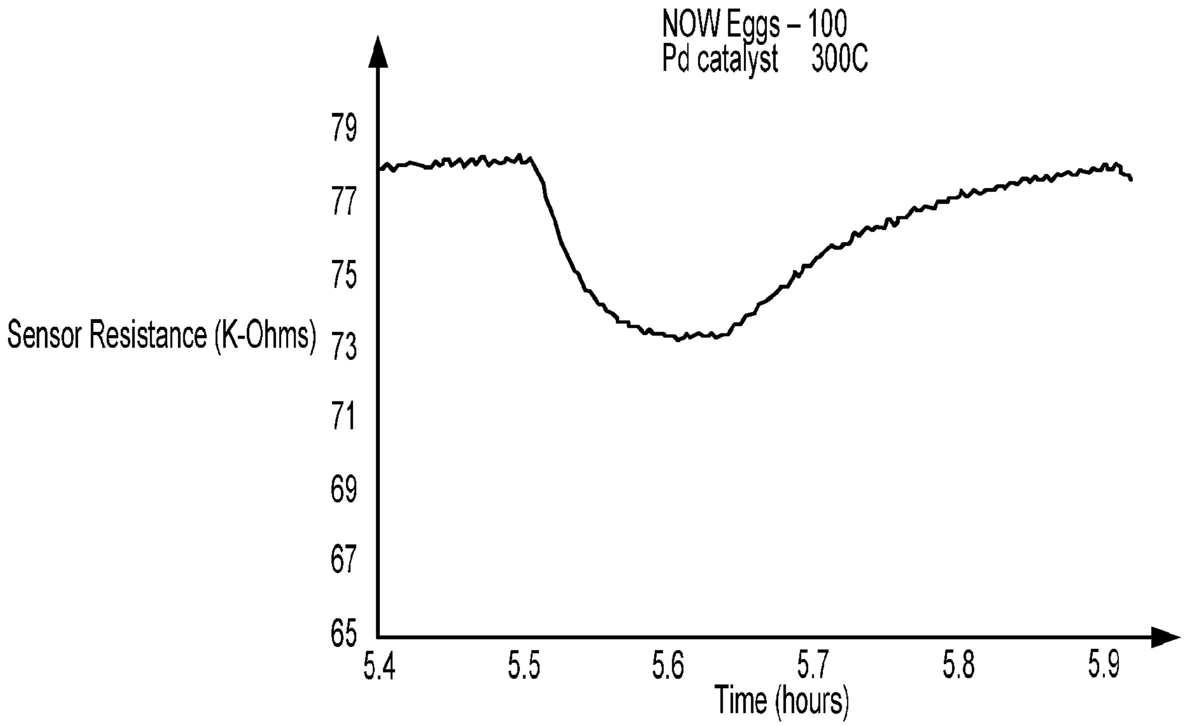


FIG. 19A

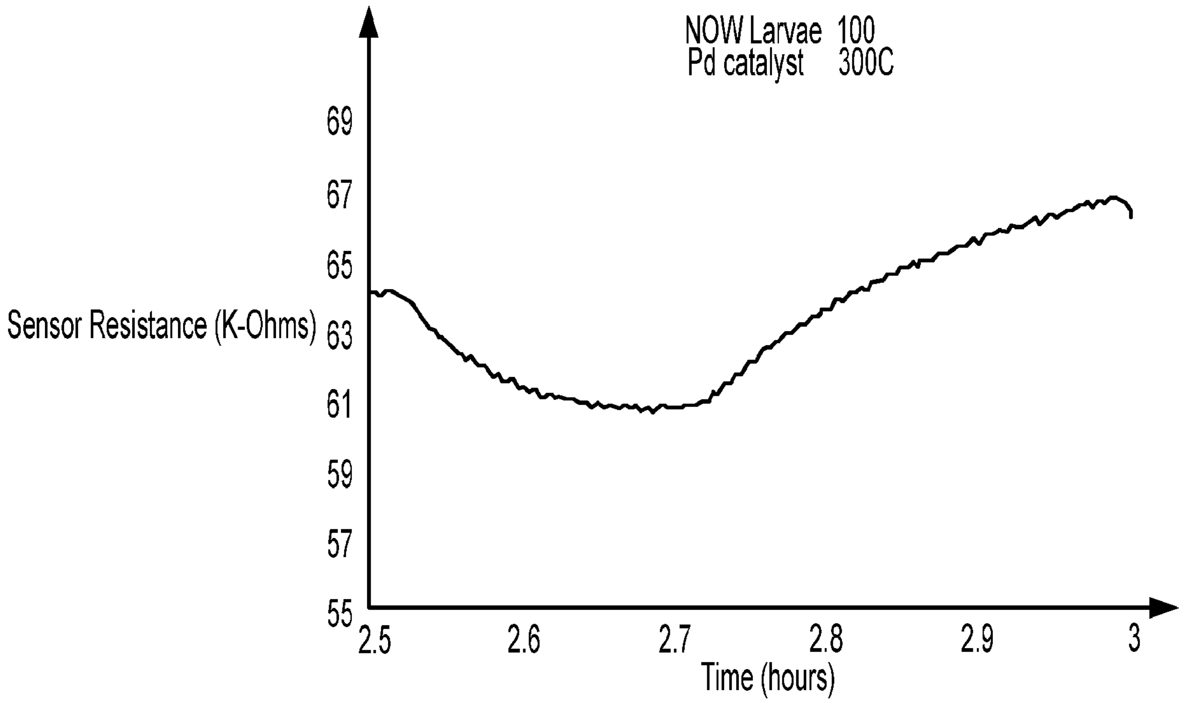


FIG. 19B

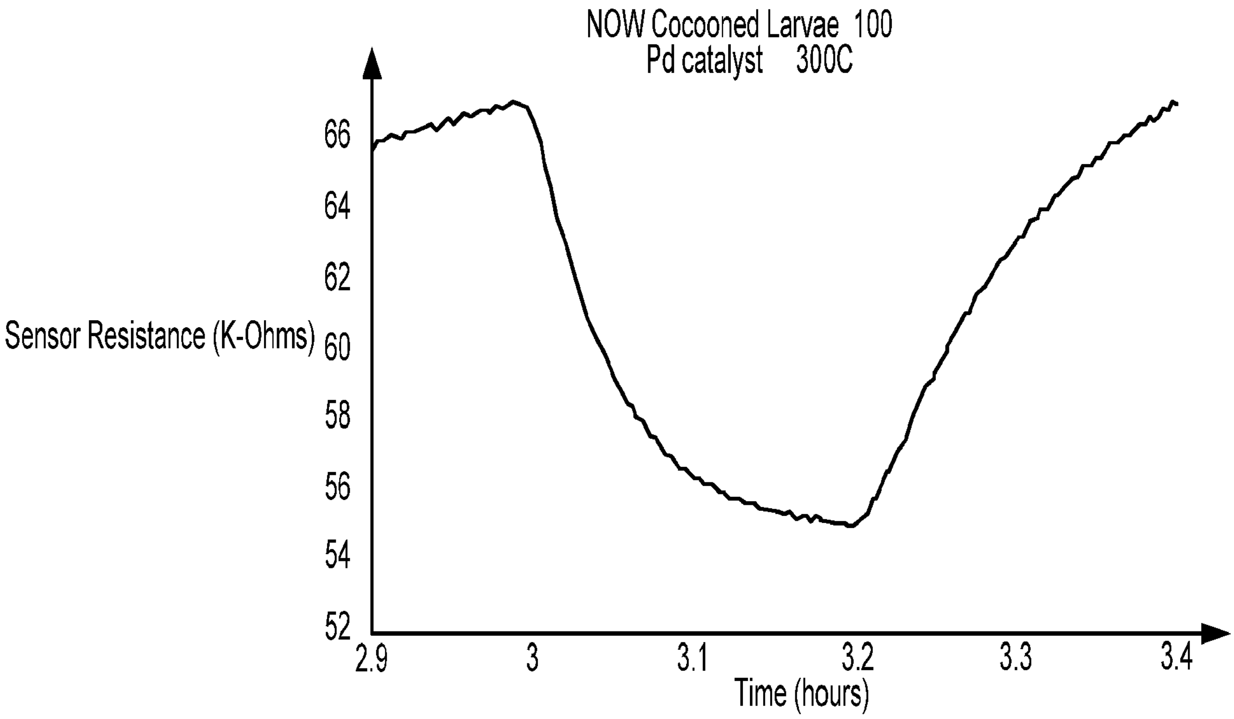


FIG. 19C

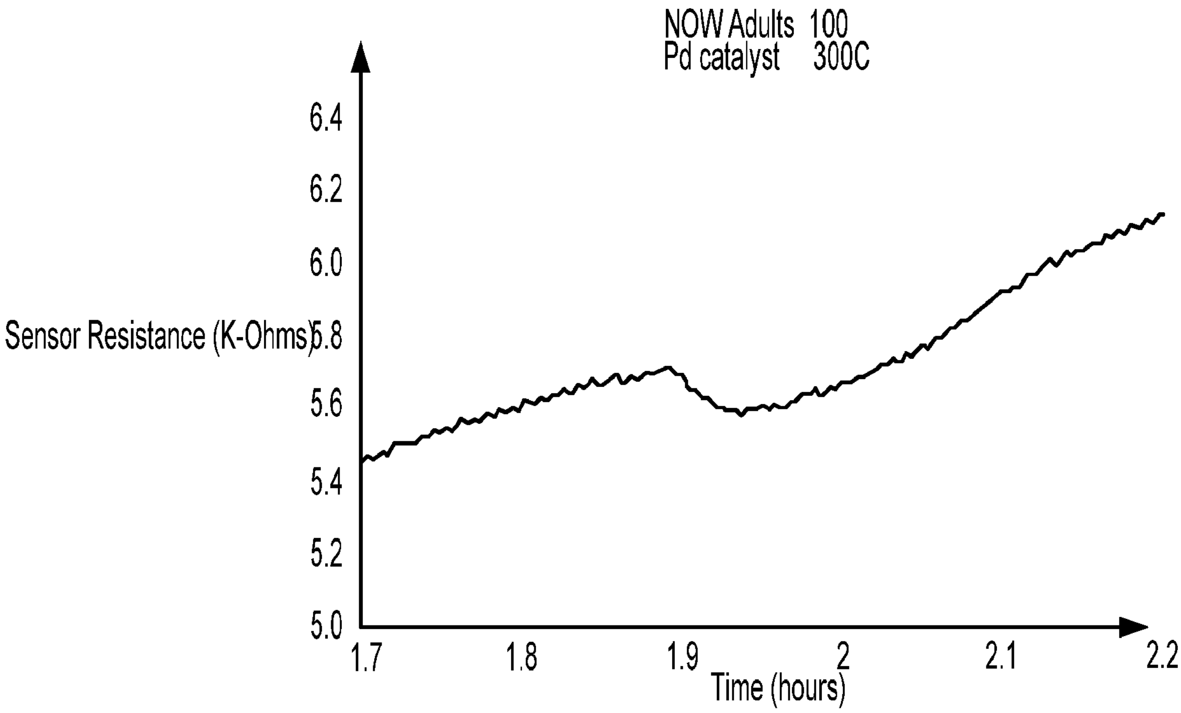


FIG. 19D

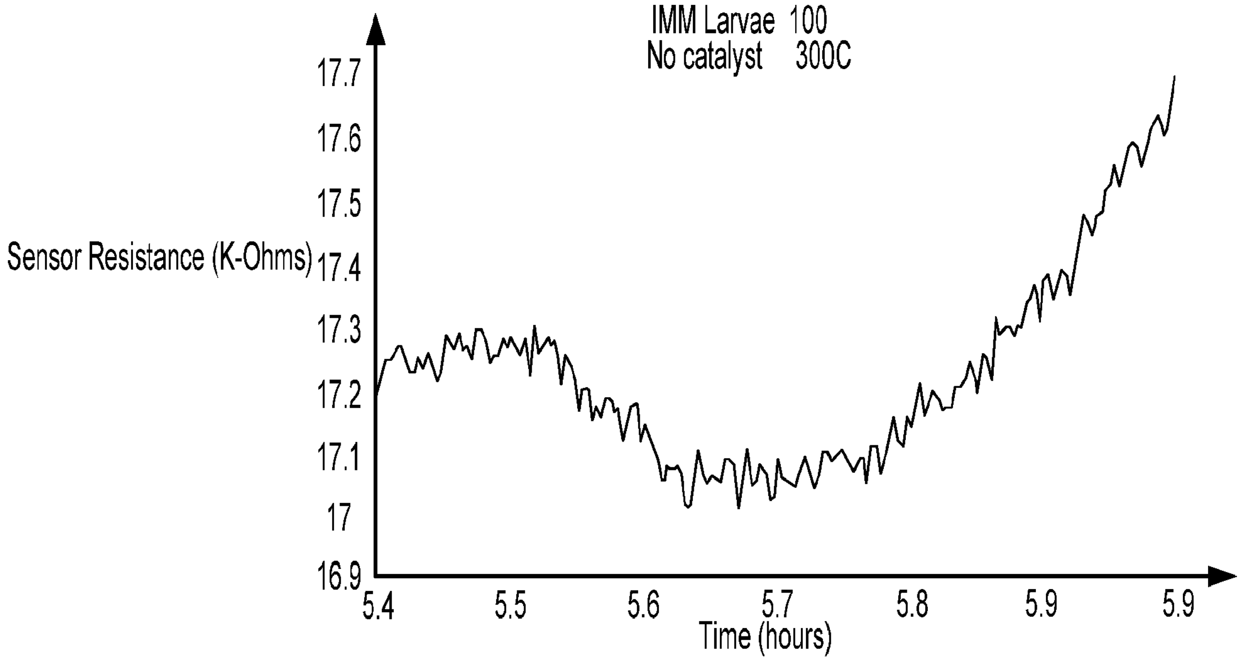


FIG. 19E

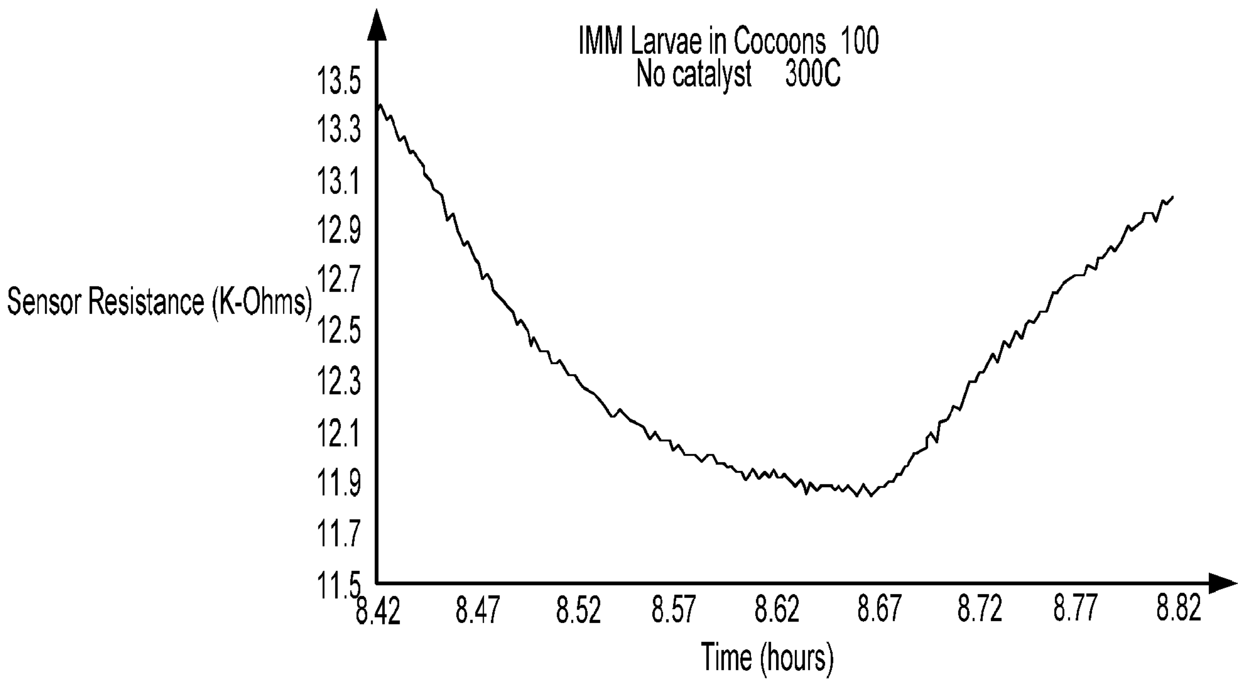


FIG. 19F

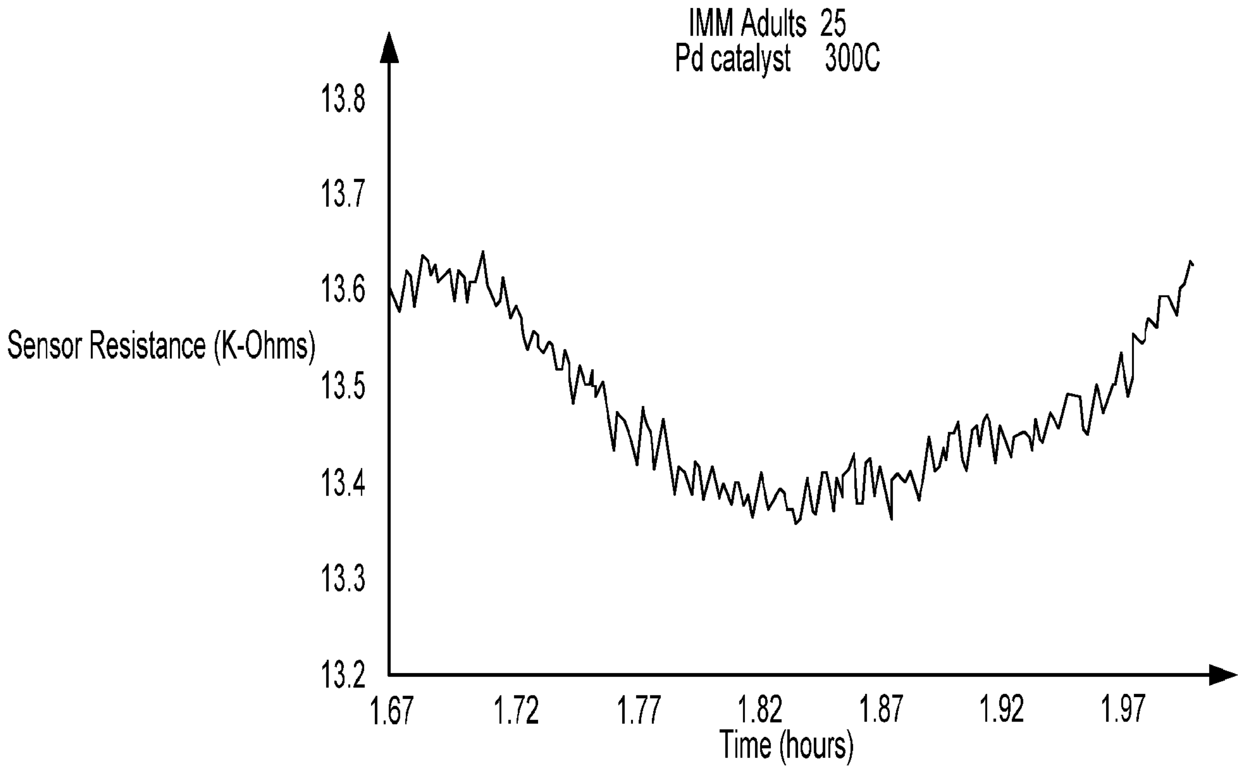
IMM Adults 25
Pd catalyst 300C
Sensor Resistance (K-Ohms)
13.8
13.7
13.6
13.5
13.4
13.3
13.2
1.67
1.72
1.77
1.82
1.87
1.92
1.97
Time (hours)

FIG. 19G

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2019234895 A


### Non-patent literature cited in the description


- **ARTHUR F. H. ; JOHNSON J. A. ; NEVEN L. G. ; HALLMAN G. J. ; FOLLETT P. A.** Insect Pest Management in Postharvest Ecosystems in the United States of America. *Outlooks on Pest Management,* 2009, vol. 20, 279-284
- **HAGSTRUM D.W. ; SUBRAMANYAM B.** Fundamentals of Stored-Product Entomology. *St. Paul: AACC Int,* 2006
- **MUELLER ; DAVID K.** Stored Product Protection: A Period of Transition. *Insects Limited, Indianapolis, Ind,* 1998
- **OKUMURA, G.T.** A Report of Canthariasis and Allergy Caused by Trogoderma (Coleoptera: Dermestidae). *California Vector Views,* 1967, vol. 14 (3), 19-22
- **PHILLIPS, T.W ; COGAN, P.M ; FADAMIRO, H.Y.** Alternatives to Pesticides in Stored-Product IPM. Kluwer Academic Publishers, 2000, 273-302
- **RESENER, A.M.** National Survey of Stored Product Insects. *Fumigants and Pheromones,* 1997, (46), 3-4